# EUROPÄISCHE PATENTSCHRIFT

(11) **EP 1 456 337 B1**
(45) Veröffentlichungstag und Bekanntmachung des Hinweises auf die Patenterteilung: **24.01.2007**
(21) Anmeldenummer: 02795195.3
(22) Anmeldetag: 16.12.2002
(51) Int. Cl.: C11D 3/39, C11D 3/37, C08F 8/42, C08F 8/32, A61K 8/22, A61K 8/49, A61Q 5/00

(54) **VERWENDUNG VON TRÄGER FIXIERTEN BLEICHKATALYSATORKOMPLEXVERBINDUNGEN ALS KATALYSATOREN FÜR PERSAUERSTOFFVERBINDUNGEN**
USE OF SUPPORT-FIXED BLEACHING CATALYST COMPLEX COMPOUNDS AS CATALYSTS FOR PEROXIDE COMPOUNDS
UTILISATION DES COMPOSES DE COMPLEXES CATALYSEURS DE BLANCHIMENT FIXES SUR SUPPORT COMME CATALYSEURS POUR COMPOSES PEROXYGENES

(30) Priorität: 21.12.2001 DE 10163331
(43) Veröffentlichungstag der Anmeldung: 15.09.2004
(73) Patentinhaber: Henkel Kommanditgesellschaft auf Aktien, 40589 Düsseldorf (DE)
(72) Erfinder: GENTSCHEV, Pavel, 40479 Düsseldorf (DE); DÖRING, Steve, 40591 Düsseldorf (DE); BREYER, Jacques, B-9070 Heusden-Destelbergen (BE); MACHIN, Antonio, E-08013 Barcelona (ES)
(86) Internationale Anmeldenummer: PCT/EP2002/014290
(87) Internationale Veröffentlichungsnummer: WO 2003/054128

(56) Entgegenhaltungen:
- WO-A-01/18166
- WO-A-98/44162
- DE-A- 2 645 079
- US-A- 3 873 668
- US-A- 4 104 466
- US-A- 4 230 828
- E. TSUCHIDA, H. NISHIDE: "Polymer-Metal Complexes" ADVANCES IN POLYMER SCIENCE, Bd. 24, 1977, Seiten 1-87, XP001146742

## Beschreibung

Die vorliegende Erfindung betrifft die Verwendung von trägerfixierten Komplexverbindungen als Bleichkatalysatoren. Insbesondere betrifft es die Verwendung trägerfixierte Bleichkatalysatorkomplexverbindungen von Übergangsmetallen mit stickstoffhaltigen polydentaten Liganden als Katalysatoren für Persauerstoffverbindungen, geeignet zum Bleichen von Farbanschmutzungen beim Waschen von Textilien und Reinigen harter Oberflächen, beispielsweise von Geschirr, sowie Wasch-, Reinigungs- und Desinfektionsmittel, die diese Katalysatoren enthalten.

Anorganische Persauerstoffverbindungen, insbesondere Wasserstoffperoxid und feste Persauerstoffverbindungen, die sich in Wasser unter Freisetzung von Wasserstoffperoxid lösen, wie Natriumperborat und Natriumcarbonat-Perhydrat, werden seit langem als Oxidationsmittel zu Desinfektions- und Bleichzwecken verwendet. Die Oxidationswirkung dieser Substanzen hängt in verdünnten Lösungen stark von der Temperatur ab; so erzielt man beispielsweise mit H₂O₂ oder Perborat in alkalischen Bleichflotten erst bei Temperaturen oberhalb von etwa 80 °C eine ausreichend schnelle Bleiche verschmutzter Textilien. Bei niedrigeren Temperaturen kann die Oxidationswirkung der anorganischen Persauerstoffverbindungen durch Zusatz sogenannter Bleichaktivatoren verbessert werden, für die zahlreiche Vorschläge, vor allem aus den Stoffklassen der N- oder O-Acylverbindungen, beispielsweise mehrfach acylierte Alkylendiamine, insbesondere Tetraacetylethylendiamin, acylierte Glykolurile, insbesondere Tetraacetylglykoluril, N-acylierte Hydantoine, Hydrazide, Triazole, Hydrotriazine, Urazole, Diketopiperazine, Sulfurylamide und Cyanurate, außerdem Carbonsäureanhydride, insbesondere Phthalsäureanhydrid, Carbonsäureester; insbesondere Natriumnonanoyloxy-benzolsulfonat, Natrium-isononanoyloxy-benzolsulfonat und acylierte Zuckerderivate, wie Pentaacetylglukose, in der Literatur bekannt geworden sind. Durch Zusatz dieser Substanzen kann die Bleichwirkung wässriger Peroxidflotten so weit gesteigert werden, dass bereits bei Temperaturen um 60 °C im wesentlichen die gleichen Wirkungen wie mit der Peroxidflotte allein bei 95 °C eintreten.

Im Bemühen um energiesparende Wasch- und Bleichverfahren gewinnen in den letzten Jahren Anwendungstemperaturen deutlich unterhalb 60 °C, insbesondere unterhalb 45 °C bis herunter zur Kaltwassertemperatur unterhalb 20 °C an Bedeutung.

Im Stand der Technik ist die Verwendung von Übergangsmetallsalzen und - komplexen, wie zum Beispiel in den europäischen Patentanmeldungen EP 392 592, EP 443 651, EP 458 397, EP 544 490, EP 549 271 und WO 01/48138 vorgeschlagen, sogenannten Bleichkatalysatoren, bekannt.

Es wurde nun beobachtet, dass Textilien, insbesondere Buntwäsche nach mehreren Waschungen ausbleichen. Ohne auf eine bestimmte Theorie festgelegt zu sein, wird vermutet, dass die im Stand der Technik verwendeten Katalysatoren nicht nur die Persauerstoffverbindungen katalysieren, sondern auch direkt die zu bleichenden Oberflächen, wie Textiloberflächen, kontaktieren und zumindest teilweise an deren Oberflächen noch nach Beendigung des Reinigungsvorganges verbleiben. Die in den Komplexverbindungen enthaltenden Übergangsmetallsalze werden in nachweisbaren Mengen während des Waschvorganges ausgewaschen, wobei diese Übergangsmetallsalze dann oxidiert werden können und so eine Farbschädigung verursachen, da sie den zu bleichenden Gegenstand, beispielsweise Textilien, direkt kontaktieren. Beispielsweise wird Mn(II) nachweislich zu Braunstein MnO₂ oxidiert. Braunstein ist ein nicht ungefährliches, sehr kräftiges Oxidationsmittel, insbesondere gegenüber leicht oxidierbaren Stoffen, wie organischen Farbstoffverbindungen. Bei den ausgewaschenen Übergangsmetallsalzen besteht, vermutlich wegen der hohen Reaktivität der aus ihnen und den Persauerstoffverbindungen entstehenden oxidierenden Intermediaten, die Gefahr der Faserschädigung und/oder Farbveränderung gefärbter Textilien und im Extremfall die Gefahr einer oxidativen Textilschädigung.

Sämtliche im Stand der Technik bekannten Bleichkatalysatoren haben den Nachteil, dass diese im erhöhten Umfang die Oberflächen der zu bleichenden Artikel kontaktieren, daran anhaften und sogar in die Oberflächen der zu bleichenden Artikel, beispielsweise in die Tiefe des Textils, eindringen können, so dass eine erhöhte Gefahr von ungewünschten Farbveränderungen besteht und bei Textilien in seltenen Fällen sogar Löcher, infolge von Faserschädigungen, auftreten können.

Aufgabe der vorliegende Erfindung ist es Bleichkatalysatoren zur Verfügung zu stellen, die die vorstehenden Nachteile im Stand der Technik überwindet. Insbesondere ist es Aufgabe der vorliegenden Erfindung Übergangsmetallsalz freie Komplexverbindungen und/oder Übergangsmetallsalz enthaltende Komplexverbindungen, geeignet als Bleichkatalysatoren, zur Verfügung zu stellen die die Gefahr von ungewünschten Farbveränderungen und/oder Faserschädigungen deutlich gegenüber dem Stand der Technik verringert oder sogar verhindert. Noch eine Aufgabe der vorliegenden Erfindung ist es Übergangsmetallsalz freie Komplexverbindungen und/oder Übergangsmetallsalz enthaltende Komplexverbindungen, geeignet als Bleichkatalysatoren, zur Verfügung zu stellen die die Oxidations- und Bleichwirkung anorganischer Persauerstoffverbindungen bei niedrigen Temperaturen unterhalb von 80 °C, insbesondere im Temperaturbereich von ca. 15 °C bis 45 °C, katalysieren.

Aufgabe der vorliegenden Erfindung ist es, die Nachteile im Stand der Technik zu überwinden, sowie die Bleichkatalysatoreigenschaften zu verbessern als auch die benötigte Menge an Peroxid, unter Erhalt der gleichen Bleichleistung, zu senken.

Es wurde nun überraschend gefunden, dass die gemäß Anspruch 1 verwendeten an wenigstens einen Träger über wenigstens einen organischen Liganden kovalent gebundene bzw. fixiertere Bleichkatalysatorkomplexe die vorgenannten Nachteile des Standes der Technik überwinden. Insbesondere hat sich gezeigt, dass die Menge an eingesetztem Peroxid reduziert werden kann, um die gleiche Bleichwirkung hervorzurufen, wie im Vergleich zu den gleichen, nicht an einen Träger gebundenen Bleichkatalysatorkomplexen. Es hat sich überraschend gezeigt, dass erfindungsgemäß verwendeten an wenigstens einen Träger über wenigstens einen organischen Liganden kovalent gebundene bzw. fixierte, Übergangsmetall aufweisende, Bleichkatalysatorkomplexe, insbesondere mit stickstoffhaltigen polydentaten Liganden, auch eine verbesserte bleichkatalysierende Wirkung besitzen.

Der polymere Träger führt vorteilhafterweise dazu, eine Akkumulation des Katalysators im Gewebe zu verhindern. Insbesondere sind die Träger so ausgewählt, dass der Katalysator in einer festen Form vorliegt bzw. eine heterogene Katalyse stattfinden kann. Bekannte Nachteile der heterogenen Katalyse, wie der Phasenwechsel von Substrat und Katalysator, treffen hier nicht zu, da die Katalyse von aktivierten bleichaktiven Persauerstoffverbindungen nicht direkt am Ort der Anschmutzung stattfinden muß. Die Halbwertszeit von üblichen Persauerstoffverbindungen (z. B. Peressigsäure) ist in der Regel so hoch, daß die Persauerstoffverbindungen über Diffusion bis zum Ort der Anschmutzung (dem Wirkort) gelangen können. Ein weiterer Vorteil der trägerfixierten Katalysatoren ist die Wiederverwendbarkeit der Katalysatoren bzw. eine leichte Abtrennbarkeit des Katalysatormaterials. Bevorzugt sind insbesondere solche Träger (bevorzugt aus flexiblen, polymeren Materialien), die für den Einsatz z. B. in der Waschmaschine geeignet sind.

Die Aufgabe der Erfindung wird durch einen trägerfixierten Bleichkatalysator(en), geeignet zur Katalyse von Peroxidverbindungen gelöst, wobei der oder die trägerfixierten Bleichkatalysatoren über wenigstens einen organischen Liganden des Bleichkatalysators kovalent an einen Träger gebunden ist und der oder die Bleichkatalysatoren einen Komplex mit wenigstens einem Übergangsmetall ausbilden. Mit kovalenter Bindung sind hier bevorzugt solche chemischen Bindungen gemeint, die in wäßrigen Lösungen, und insbesondere in säure- oder basenhaltigen Lösungen nicht gespalten werden. Insbesondere wird die kovalente Bindung zwischen Bleichkatalysator und Träger unter typischen Waschbedingungen (basisch, pH > 9, > 60 min, wäßrige Tensidlösung, Temperatur >30°C) oder auch sauren Bedingungen (z.B. pH 3) nicht geöffnet. Besonders geeignete säure- als auch basenstabile Verbindung sind z. B. sekundäre und tertiäre Amine, C-C-Verknüpfungen oder Etherbindungen. (R-H₂C-N_{(tert.)}-R₂, (R-H₂C-NH_{(sek.)}-R, R-H₂C-CH₂-R-Bindungen oder R-H₂C-O-CH₂-R-(Etherbindungen).

Es hat sich gezeigt, dass auch freie Liganden, die an einen Träger kovalent sind, verwendbar sind, die erst am Einsatzort mit einem Übergangsmetall, das von einer anderen Quelle, beispielsweise aus der Bleichmittelzusammensetzung und/oder dem verwendeten Wasser, abstammt, den Übergangsmetall aufweisenden Komplex ausbildet.

Die Aufgabe der Erfindung wird somit auch durch einen trägerfixierten Bleichkatalysator(en) zur Katalyse von Peroxidverbindungen gelöst, bei dem ein an einen Träger kovalent gebundener Ligand ein Übergangsmetall freier Liganden ist, der mit einem Übergangsmetall chelatisiert, das von einer anderen Quelle, vorzugsweise aus der Bleichmittelzusammensetzung und/oder zugesetztem Wasser, abstammt, und damit den Übergangsmetall aufweisenden Komplex ausbildet.

Es wurde außerdem festgestellt, dass der trägerfixierte Bleichkatalysator geeignet ist Persauerstoffverbindungen und/oder Sauerstoff zu aktivieren. Somit können auch Persauerstoff freie Mittel verwendet werden, wenn ausreichende Mengen Sauerstoff am Einsatzort zur Verfügung stehen.

Im Sinne dieser Erfindung können ein oder mehrere Übergangsmetall aufweisende Komplex über einen oder mehrere Liganden an wenigstens einen Träger gebunden sein. Es versteht sich von selbst, dass sowohl die erfindungsgemäß verwendbaren Liganden als auch die Komplexe gleich oder unterschiedlich sein können.

Der Begriff "optional", wie in der Beschreibung gebraucht, umfaßt sämtliche denkbaren Variationen. Verbindungen bzw. Gruppen die "optional" substituiert sind, umfassen somit im Sinne dieser Erfindung unsubstituierte und substituierte Verbindungen bzw. Gruppen.

Im Sinne dieser Erfindung stehen die Begriffe "Alkyl, Alkoxy, Aryl, Alkenyl, Alkylen, Arylen, Amine, Halogen, Carboxylatderivate, Cycloalkyl, Carbonylderivate, C1-C6-Heterocycloalkyl, Heterocycloalkyl, Heteroaryl, Heteroarylen, Sulphonat, Sulphat, Phosphonat, Phosphat, Phosphin, Phosphinoxid ", wenn nicht anders angegeben, für:
Alkyl = lineares oder verzweigtes C1-C8-Alkyl.
Alkenyl = C2-C6-Alkenyl.
Cycloalkyl = C3-C8-Cycloalkyl.
Alkoxy = C1-C6-Alkoxy.
Aryl = Homoaromaten mit einem Molekulargewicht von ≤300.

Alkylen = Methylen; 1,1-Ethylen; 1,2-Ethylen; 1,1-Propyliden; 1,2-Propylen; 1,3-Propylen; 2,2-Propyliden; Butan-2-ol-1,4-diyl; Propan-2-ol-1,3-diyl; 1,4-Butylen; Cyclohexan-1,1-diyl; Cyclohexan-1,2-diyl; Cyclohexan-1,3-diyl; Cyclohexan-1,4-diyl; Cyclopentan-1,1-diyl; Cyclopentan-1,2-diyl; und/oder Cyclopentan-1,3-diyl.

Arylen = 1,2-Phenylen; 1,3-Phenylen; 1,4-Phenylen; 1,2-Naphtalenylen; 1,3-Naphtalenylen; 1,4-Naphtalenylen; 2,3-Naphtalenylen; 1-Hydroxy-2,3-phenylen; 1-Hydroxy-2,4-phenylen; 1-Hydroxy-2,5-phenylen; und/oder/oder 1-Hydroxy-2,6-phenylen.

Heteroaryl = Pyridinyl; Pyrimidinyl; Pyrazinyl; Triazolyl; Pyridazinyl; 1,3,5-Triazinyl; Quinolinyl. Isoquinolinyl; Quinoxalinyl; Imidazolyl; Pyrazolyl; Benzimidazolyl; Thiazolyl; Oxazolidinyl; Pyrrolyl; Carbazolyl; Indolyl; und/oder Isoindolyl, worin das Heteroaryl mit der Verbindung über ein Ring-Atom des jeweiligen Heteroaryl-Restes verbunden ist.

Heteroarylen = Pyridindiyl; Quinolindiyl; Pyrazodiyl; Pyrazoldiyl; Triazolediyl; Pyrazindiyl; und/oder Imidazolediyl, worin das Heteroarylen die Verbindung über ein Atom des gewählten Heteroarylen verbrückt; besonders bevorzugt sind Pyridin-2,3-diyl; Pyridin-2,4-diyl; Pyridin-2,5-diyl; Pyridin-2,6-diyl; Pyridin-3,4-diyl; Pyridin-3,5-diyl; Quinolin-2,3-diyl; Quinolin-2,4-diyl; Quinolin-2,8-diyl; Isoquinolin-1,3-diyl; Isoquinolin-1,4-diyl; Pyrazol-1,3-diyl; Pyrazol-3,5-diyl; Triazole-3,5-diyl; Triazole-1,3-diyl; Pyrazin-2,5-diyl; und/oder Imidazole-2,4-diyl.

C1-C6-Heterocycloalkyl = Piperidinyl; Piperidine; 1,4-Piperazine, Tetrahydrothiophene; Tetrahydrofuran; 1,4,7-Triazacyclononan; 1,4,8,11-Tetraazacyclotetradecan; 1,4,7,10,13-Pentaazacyclopentadecan; 1,4-Diaza-7-thiacyclononan; 1,4-Diaza-7-oxa-cyclononan; 1,4,7,10-Tetraazacyclododecan; 1,4-Dioxane; 1,4,7-Trithiacyclononan; Pyrrolidin; und/oder Tetrahydropyran, worin das Heterocycloalkyl mit dem -C1-C6-Alkyl über ein Ringatom des gewählten Heterocycloalkyl verbunden sein kann.

Heterocycloalkylen = Piperidin-1,2-ylen; Piperidin-2,6-ylen; Piperidin-4,4-yliden; 1,4-Piperazin-1,4-ylen; 1,4-Piperazin-2,3-ylen; 1,4-Piperazin-2,5-ylen; 1,4-Piperazin-2,6-ylen; 1,4-Piperazin-1,2-ylen; 1,4-Piperazin-1,3-ylen; 1,4-Piperazin-1,4-ylen; Tetrahydrothiophen-2,5-ylen; Tetrahydrothiophen-3,4-ylen; Tetrahydrothiophen-2,3-ylen; Tetrahydrofuran-2,5-ylen; Tetrahydrofuran-3,4-ylen; Tetrahydrofuran-2,3-ylen; Pyrrolidin-2,5-ylen; Pyrrolidin-3,4-ylen; Pyrrolidin-2,3-ylen; Pyrrolidin-1,2-ylen; Pyrrolidin-1,3-ylen; Pyrrolidin-2,2-yliden; 1,4,7-Triazacyclonon-1,4-ylen; 1,4,7-Triazacyclonon-2,3-ylen; 1,4,7-Triazacyclonon-2,9-ylen; 1,4,7-Triazacyclonon-3,8-ylen; 1,4,7-Triazacyclonon-2,2-yliden; 1,4,8,11-Tetraazacyclotetradec-1,4-ylen; 1,4,8,11-Tetraazacyclotetradec-1,8-ylen; 1,4,8,11-Tetraazacyclotetradec-2,3-ylen; 1,4,8,11-Tetraazacyclotetradec-2,5-ylen; 1,4,8,11-Tetraazacyclotetradec-1,2-ylen; 1,4,8,11 -Tetraazacyclotetradec-2,2-yliden; 1,4,7,10-Tetraazacyclododec-1,4-ylen; 1,4,7,10-Tetraazacyclododec-1,7-ylen; 1,4,7,10-Tetraazacyclododec-1,2-ylen; 1,4,7,10-Tetraazacyclododec-2,3-ylen; 1,4,7,10-Tetraazacyclododec-2,2-yliden; 1,4,7,10,13-Pentaazacyclopentadec-1,4-ylen; 1,4,7,10,13-Pentaazacyclopentadec-1,7-ylen; 1,4,7,10,13-Pentaazacyclopentadec-2,3-ylen; 1,4,7,10,13-Pentaazacyclopentadec-1,2-ylen; 1,4,7,10,13-Pentaazacyclopentadec-2,2-yliden; 1,4-Diaza-7-thia-cyclonon-1,4-ylen; 1,4-Diaza-7-thia-cyclonon-1,2-ylen; 1,4-Diaza-7-thia-cyclonon-2,3-ylen; 1,4-Diaza-7-thia-cyclonon-6,8-ylen; 1,4-Diaza-7-thia-cyclonon-2,2-yliden; 1,4-Diaza-7-oxa-cyclonon-1,4-ylen; 1,4-Diaza-7-oxa-cyclonon-1,2-ylen; 1,4-Diaza-7-oxa-cyclonon-2,3-ylen; 1,4-Diaza-7-oxa-cyclonon-6,8-ylen; 1,4-Diaza-7-oxa-cyclonon-2,2-yliden; 1,4-Dioxan-2,3-ylen; 1,4-Dioxan-2,6-ylen; 1,4-Dioxan-2,2-yliden; Tetrahydropyran-2,3-ylen; Tetrahydropyran-2,6-ylen; Tetrahydropyran-2,5-ylen; Tetrahydropyran-2,2-yliden; 1,4,7-Trithia-cyclonon-2,3-ylen; 1,4,7-Trithia-cyclonon-2,9-ylen; und/oder 1,4,7-Trithia-cyclonon-2,2-yliden.

Heterocycloalkyl = Pyrrolinyl; Pyrrolidinyl; Morpholinyl; Piperidinyl; Piperazinyl; Hexamethylenimine; 1,4-Piperazinyl; Tetrahydrothiophenyl; Tetrahydrofuranyl; 1,4,7-Triazacyclononanyl; 1,4,8,11-Tetraazacyclotetradecanyl; 1,4,7,10,13-Pentaazacyclopentadecanyl; 1,4-Diaza-7-thiacyclononanyl; 1,4-Diaza-7-oxa-cyclononanyl; 1,4,7,10-Tetraazacyclododecanyl; 1,4-Dioxanyl; 1,4,7-Trithiacyclononanyl; Tetrahydropyranyl; und/oder Oxazolidinyl, wobei das Heterocycloalkyl mit der Verbindung über ein Ringatom of des jeweiligen Heterocycloalkyl verbunden ist.

Amine = -N(R)2 worin jedes R unabhängig voneinander ausgewählt ist aus der Gruppe umfassend: H; C1-C6-Alkyl; C1-C6-Alkyl-C6H5; und/oder Phenyl, wobei beide R einen -NC3 bis -NC5 heterocyclischen Ringschluß ausbilden können.

Halogen = F; Cl; Br und/oder I.
Sulphonat = -S(O)2OR, worin R = H; C1-C6-Alkyl; Phenyl; C1-C6-Alkyl-C6H5; Li; Na; K; Cs; Mg; und/oder Ca ist.

Sulphat = -OS(O)2OR, worin R = H; C1-C6-Alkyl; Phenyl; C1-C6-Alkyl-C6H5; Li; Na; K; Cs; Mg; und/oder Ca ist.

Sulphon: -S(O)2R, worin R = H; C1-C6-Alkyl; Phenyl; C1-C6-Alkyl-C6H5 und/oder Amine (zur Bildung von Sulphonamid) ausgewählt ist aus der Gruppe umfassend: -NR'2, worin jedes R' unabhängig voneinander ausgewählt ist aus der Gruppe umfassend: H; C1-C6-Alkyl; C1-C6-Alkyl-C6H5; und/oder Phenyl, worin wenn beide R' = C1-C6-Alkyl die R' gemeinsam einen -NC3 bis -NC5 heterocyclischen Ringschluss ausbilden können. Carboxylatderivate = -C(O)OR, worin R ausgewählt ist aus der Gruppe umfassend: H; C1-C6-Alkyl; Phenyl; C1-C6-Alkyl-C6H5; Li; Na; K; Cs; Mg; und/oder Ca.

Carbonylderivate = -C(O)R, worin R ausgewählt ist aus der Gruppe umfassend: H; C1-C6-Alkyl; Phenyl; C1-C6-Alkyl-C6H5 und/oder Amin (zur Ausbildung von Amid) ausgewählt ist aus der Gruppe umfassend: -NR'2, worin R' unabhängig voneinander ausgewählt ist aus der Gruppe umfassend: H; C1-C6-Alkyl; C1-C6-Alkyl-C6H5; und/oder Phenyl, worin wenn beide R' = C1-C6-Alkyl die R' gemeinsam einen -NC3 bis -NC5 heterocyclischen Ringschluss ausbilden können.

Phosphonat = -P(O)(OR)2, worin jedes R unabhängig voneinander ausgewählt ist aus der Gruppe umfassend: H; C1-C6-Alkyl; Phenyl; C1-C6-Alkyl-C6H5; Li; Na; K; Cs; Mg; und/oder Ca.

Phosphat = -OP(O)(OR)2, worin jedes R unabhängig voneinander ausgewählt ist aus der Gruppe umfassend: H; C1-C6-Alkyl; Phenyl; C1-C6-Alkyl-C6H5; Li; Na; K; Cs; Mg; und/oder Ca.

Phosphin = -P(R)2, worin jedes R unabhängig voneinander ausgewählt ist aus der Gruppe umfassend: H; C1-C6-Alkyl; Phenyl; C1-C6-Alkyl-C6H5.
Phosphinoxid = -P(O)R2, worin jedes R unabhängig voneinander ausgewählt ist aus der Gruppe umfassend: H; C1-C6-Alkyl; Phenyl; C1-C6-Alkyl-C6H5 und/oder Amin (zur Ausbildung von Phosphonamidat) ausgewählt ist aus der Gruppe umfassend: -NR'2, worin R' unabhängig voneinander ausgewählt ist aus der Gruppe umfassend: H; C1-C6-Alkyl; C1-C6-Alkyl-C6H5; und/oder Phenyl, worin wenn beide R' = C1-C6-Alkyl die R' gemeinsam einen -NC3 bis -NC5 heterocyclischen Ringschluss ausbilden können; steht.

### Besonders bevorzugt, wenn nicht anders angegeben ist:

Alkyl = lineares und/oder verzweigtes C1-C6-Alkyl;
Alkenyl = C3-C6-Alkenyl;
Cycloalkyl: C6-C8-Cycloalkyl;
Alkoxy = C1-C4-Alkoxy;

Alkylen = ausgewählt ist aus der Gruppe umfassend: Methylen; 1,2-Ethylen; 1,3-Propylen; Butan-2-ol-1,4-diyl; 1,4-Butylen; Cyclohexan-1,1-diyl; Cyclohexan-1,2-diyl; Cyclohexan-1,4-diyl; Cyclopentane-1,1-diyl; und/oder Cyclopentan-1,2-diyl;

Aryl = ausgewählt ist aus der Gruppe umfassend: Phenyl; Biphenyl; Naphthalenyl; Anthracenyl; und/oder Phenanthrenyl;

Arylene = ausgewählt ist aus der Gruppe umfassend:1,2-Phenylen; 1,3-Phenylen; 1,4-Phenylen; 1,2-Naphtalenylen; 1,4-Naphtalenylen; 2,3-Naphtalenylen und/oder 1-Hydroxy-2,6-phenylen;

Heteroaryl = ausgewählt ist aus der Gruppe umfassend: Pyridinyl; Pyrimidinyl; Quinolinyl; Pyrazolyl; Triazolyl; Isoquinolinyl; Imidazolyl; und/oder Oxazolidinyl,
worin das Heteroaryl mit der Verbindung über ein Ringatom des gewählten Heteroaryl verküpft ist;

Heteroarylen = ausgewählt ist aus der Gruppe umfassend: Pyridin-2,3-diyl; Pyridin-2,4-diyl; Pyridin-2,6-diyl; Pyridin-3,5-diyl; Quinolin-2,3-diyl; Quinolin-2,4-diyl; Isoquinolin-1,3-diyl; Isoquinolin-1,4-diyl; Pyrazol-3,5-diyl; und/oder Imidazole-2,4-diyl;

Heterocycloalkyl = ausgewählt ist aus der Gruppe umfassend: Pyrrolidinyl; Morpholinyl; Piperidinyl; Piperidinyl; 1,4-Piperazinyl; Tetrahydrofuranyl; 1,4,7-Triazacyclononanyl; 1,4,8,11-Tetraazacyclotetradecanyl; 1,4,7,10,13-Pentaazacyclopentadecanyl; 1,4,7,10-Tetraazacyclododecanyl; und/oder Piperazinyl; worin das Heterocycloalkyl mit der Verbindung über ein Ringatom des gewählten Heterocycloalkyl verküpft ist;

Heterocycloalkylen = ausgewählt ist aus der Gruppe umfassend: Piperidin-2,6-ylen; Piperidin-4,4-yliden; 1,4-Piperazin-1,4-ylen; 1,4-Piperazin-2,3-ylen; 1,4-Piperazin-2,6-ylen; Tetrahydrothiophen-2,5-ylen; Tetrahydrothiophen-3,4-ylen; Tetrahydrofuran-2,5-ylen; Tetrahydrofuran-3,4-ylen; Pyrrolidin-2,5-ylen; Pyrrolidin-2,2-yliden; 1,4,7-Triazacyclonon-1,4-ylen; 1,4,7-Triazacyclonon-2,3-ylen; 1,4,7-Triazacyclonon-2,2-yliden; 1,4,8,11-Tetraazacyclotetradec-1,4-ylen; 1,4,8,11-Tetraazacyclotetradec-1,8-ylen; 1,4,8,11-Tetraazacyclotetradec-2,3-ylen; 1,4,8,11-Tetraazacyclotetradec-2,2-yliden; 1,4,7,10-Tetraazacyclododec-1,4-ylen; 1,4,7,10-Tetraazacyclododec-1,7-ylen; 1,4,7,10-Tetraazacyclododec-2,3-ylen; 1,4,7,10-Tetraazacyclododec-2,2-yliden; 1,4,7,10,13-Pentaazacyclopentadec-1,4-ylen; 1,4,7,10,13-Pentaazacyclopentadec-1,7-ylen; 1,4-Diaza-7-thia-cyclonon-1,4-ylen; 1,4-Diaza-7-thia-cyclonon-2,3-ylen; 1,4-Diaza-7-thia-cyclonon-2,2-yliden; 1,4-Diaza-7-oxa-cyclonon-1,4-ylen; 1,4-Diaza-7-oxa-cyclonon-2,3-ylen; 1,4-Diaza-7-oxa-cyclonon-2,2-yliden; 1,4-Dioxan-2,6-ylen; 1,4-Dioxan-2,2-yliden; Tetrahydropyran-2,6-ylen; Tetrahydropyran-2,5-ylen; und/oder Tetrahydropyran-2,2-yliden;

C1-C6-Alkyl-heterocycloalkyl, worin das Heterocycloalkyl des -C1-C6-Heterocycloalkyl ausgewählt ist aus der Gruppe umfassend: Piperidinyl; 1,4-Piperazinyl; Tetrahydrofuranyl; 1,4,7-Triazacyclononanyl; 1,4,8,11-Tetraazacyclotetradecanyl; 1,4,7,10,13-Pentaazacyclopentadecanyl; 1,4,7,10-Tetraazacyclododecanyl; und/oder Pyrrolidinyl, wobei das Heterocycloalkyl mit dem -C1-C6-Alkyl über ein Ringatom des jeweiligen Heterocycloalkyl verknüpft ist;

Amin = -N(R)2, worin jedes R unabhängig voneinander ausgewählt ist aus der Gruppe umfassend: H; C1-C6-Alkyl; und/oder Benzyl;

Halogen = ausgewählt ist aus der Gruppe umfassend: F und/oder Cl;

Sulphonat = -S(O)2OR, worin R ausgewählt ist aus der Gruppe umfassend: H; C1-C6-Alkyl; Na; K; Mg; und/oder Ca;

Sulphat = -OS(O)2OR, worin R ausgewählt ist aus der Gruppe umfassend:H; C1-C6-Alkyl; Na; K; Mg; und/oder Ca;

Sulphon = -S(O)2R, worin R ausgewählt ist aus der Gruppe umfassend: H; C1-C6-Alkyl; Benzyl und/oder Amine ausgewählt ist aus der Gruppe umfassend: - NR'2, worin jedes R' unabhängig voneinander ausgewählt ist aus der Gruppe umfassend: Wasserstoff; C1-C6-Alkyl; und/oder Benzyl;

Carboxylatderivat = -C(O)OR; worin R ausgewählt ist aus der Gruppe umfassend: H; Na; K; Mg; Ca; C1-C6-Alkyl; und/oder Benzyl.

Carbonylderivat = -C(O)R; worin R ausgewählt ist aus der Gruppe umfassend: H; C1-C6-Alkyl; Benzyl und/oder Amine ausgewählt ist aus der Gruppe umfassend: - NR'2, worin jedes R' unabhängig voneinander ausgewählt ist aus der Gruppe umfassend: Wasserstoff; C1-C6-Alkyl; und/oder Benzyl;

Phosphonat = -P(O)(OR)2; worin jedes R unabhängig voneinander ausgewählt ist aus der Gruppe umfassend: H; C1-C6-Alkyl; Benzyl; Na; K; Mg; und/oder Ca; Phosphat = -OP(O)(OR)2; worin jedes R unabhängig voneinander ausgewählt ist aus der Gruppe umfassend: H; C1-C6-Alkyl; Benzyl; Na; K; Mg; und/oder Ca; Phosphine = -P(R)2; worin jedes R unabhängig voneinander ausgewählt ist aus der Gruppe umfassend: H; C1-C6-Alkyl; und/oder Benzyl;

Phosphinoxid = -P(O)R2; worin jedes R unabhängig voneinander ausgewählt ist aus der Gruppe umfassend: H; C1-C6-Alkyl; Benzyl und/oder Amine ausgewählt ist aus der Gruppe umfassend: -NR'2, worin jedes R' unabhängig voneinander ausgewählt ist aus der Gruppe umfassend: Wasserstoff; C1-C6-Alkyl; und/oder Benzyl.

Prinzipiell sind als Trägermaterial Formkörper, wie Pulver, Partikel, Folien, Gele, textile Fasern, geeignet.

Träger im Sinne dieser Erfindung, an die wenigstens ein Ligand wenigstens eines Bleichmittelkatalysators kovalent gebunden werden kann, umfassen Polymere, vorzugsweise ausgewählt aus der Gruppe umfassend Polyvinylchlorid, Polybutadien, Polychlorbutadien, Polyvinylidenchlorid, Polyacrylnitril, Polydichlormethyloxaisobutan, Polyurethan, Polystyrole, Polymethacrylate, Polyvinylalkohole, Polyethylenimine, Cellulose, Chitosan, Polysiloxane, Polyamide, Polyamine, Polyformaldehyde, Polyethylen, Polypropylen, Polytetrafluorethylen, Polyisobutylen, Polydimethylpheylenoxid, Chlormethyliertes Polystyrol und/oder Polyisocyanate, wobei Chlormethyliertes Polystyrol am meisten bevorzugt ist.

Prinzipiell sind als Trägermaterial Formkörper aus Thermoplasten wie Polypropylen (PP), Polyethylen (PE), Polyamiden (PA) und/oder Polyestern, geeignet.

Die Träger die erfindungsgemäß verwendbar sind, weisen ein Molekulargewicht von ≥ 100, ≥ 500, ≥ 1.000, vorzugsweise ≥10.000, weiter bevorzugt ≥ 50.000, noch weiter bevorzugt ≥ 100.000. Es können aber auch Träger mit Molekulargewichten von ≥ 1.000.000 und ≥10.000.000 verwendet werden.

An einem Träger können mehr als ein über wenigstens einen Liganden kovalent an den Träger gebundener Bleichkatalysator verknüpft sein. Vorzugsweise sind es mehr als 2, weiter bevorzugt mehr als 10 und noch weiter bevorzugt mehr als 20 Liganden, die an den Träger kovalent gebunden sind. Je nach Träger können es aber auch mehr als 50, insbesondere mehr als 100 oder auch mehr als 500 kovalent fixierte Liganden sein. Möglich sind auch mehr als 1000 oder sogar mehr als 10.000, wenn das Trägermaterial ein ausreichend hohes Molekulargewicht aufweist. Die an den Träger gebundenen Liganden können auch freie Liganden sein, d.h. diese Liganden weisen kein Übergangsmetall auf. In solchen Fällen stammt das Übergangsmetall aus anderen Quellen, wie beispielsweise der Waschflotte, Spülflotte oder dergleichen.

Unter Textilfasern sind sämtliche Fasern, die sich textil verarbeiten lassen, zu verstehen.

Textilfasern lassen sich nach Herkunft oder stofflicher Beschaffenheit in folgende Gruppen einteilen:
1. Naturfasern: Bei diesen wird zwischen Fasern pflanzlicher, tierischer und mineralischer Herkunft unterschieden. Die den Faserpflanzen entstammenden Pflanzenfasern (= pflanzliche Fasern) werden weiter unterteilt in a) Samenfasern, z.B. Baumwolle, Kapok, b) Bastfasern, z.B. Flachs, Hanf, Jute, Kenaf, Ramie, Rosella, Sunn, Urena, c) Hartfasern, z.B. Alfa- od. Espartogras, Fique, Henequen, Kokos, Manila, Phormium, Sisal. Tierfasern ( = tierische Fasern) gliedern sich in die Untergruppen a) Wolle, b) feine Tierhaare, z.B. Angora, Alpaka, Guanako, Kamel, Kanin, Kaschmir, Lama, Mohair, Vikunja, Yak, c) grobe Tierhaare, z.B. Rinder- u. Rosshaar, Ziegenhaar und d) Seiden, z.B. Maulbeer- und Tussahseide.
2. Chemiefasern: Diese früher Kunstfasern genannten Fasern lassen sich in solche aus natürlichen und synthetischen Polymeren sowie aus anorganischen Stoffen gruppieren.
   a) Abgewandelte Naturstoffe sind im allgemeinen pflanzlicher Herkunft. Hierzu gehören v.a. Fasern aus regenerierter Cellulose, wie Kupferseide, Viskosefasern, Modalfasern und Celluloseacetaten, wie Acetat, Triacetat, aus Alginaten, wie Alginatfasern und Polyisoprenen, wie Gummi. Fasern aus regenerierter Cellulose, gelegentlich auch solche aus Celluloseestern werden hierbei oft als Kunstseide oder Reyon bezeichnet.
   b) Unter Synthesefasern versteht man vollsynthetische Chemiefasern, die aus einfachen organischen Bausteinen (= Monomeren) durch Polymerisation, Polykondensation oder Polyaddition - also durch Polyreaktionen - hergestellt werden. Zu den Synthesefasern zählen beispielsweise die Elastofasern, wie Elastan, Elastodien; Fluorofasern; Polyacrylfasern, wie Polyacrylonitril, Modacryl; Polyamidfasern, wie Nylon, Aramid; Polychloridfasern, wie Polyvinylchlorid, Polyvinylidenchlorid; Polyesterfasern; Polyolefinfasern, wie Polyethylen, Polypropylen und Polyvinylalkoholfasern. c) Anorganische Chemiefasern können aus Glas, Kohlenstoff oder Metall sein. Sie werden zur Herstellung von Fasern in eine spinnfähige Form gebracht u. in diesem Zustand, beispielsweise in Lösung oder als Schmelze durch engporige Öffnungen, beispielsweise Düsen in ein verfestigendes Medium, z.B. Fällbad beim Nassspinnverfahren oder geheizter Spinnschacht beim Trockenspinnverfahren, gepresst bzw. in Schmelzspinn-Apparaten zu Filamenten geformt, ggf. verstreckt, gefärbt, nach verschiedenen Methoden zu Fasern versponnen und zu Garnen vereinigt.

Prinzipiell sind sowohl gewebte wie auch nicht gewebte Materialien einsetzbar, wie Vliesstoffe, Gewebe oder Gewirke aus den oben genannten Materialien oder andere geeignete Materialien oder Gemische davon. Außerdem sind auch Materialien wie u.a. Schaumstoffe, insbesondere offenporige Schaumstoffe, Mikrofasern, Nanofasern, Partikel, Agglomerate und/oder Folien als Materialien bzw. als Träger verwendbar, hierbei können die Partikel, Nanopartikel, Agglomerate, Pulver und/oder Gele aus dem gleichen Material wie die Fasern gebildet sein. Die Träger können insbesondere aus einem bestimmten Material oder aus Materialgemischen bestehen.

Die als Träger geeigneten Partikel, weisen vorzugsweise einen Partikeldurchmesser von 20 µm und 1 mm und vorzugsweise zwischen 200 µm und 0,6 mm, auf.

Der oder die organischen kovalent an das Trägermaterial gebundenen Liganden der vorliegenden Erfindung sind geeignet zur Ausbildung eines Bleichkatalysatorkomplexes durch Komplexierung eines Metalls, wobei der gebildete Komplex zur Katalyse von Bleichmitteln, insbesondere Peroxyverbindungen, geeignet ist. Vorzugsweise weist der mit wenigstens einem Liganden an einen Träger kovalent gebundene Bleichkatalysatorkomplex ein Übergangsmetall auf.

In einer weiteren Ausführungsform handelt es sich bei dem mit wenigstens einem Liganden an einen Träger kovalent gebundene Ligand um einen freien Liganden, der erst am Einsatzort mit einem Übergangsmetall, das von einer anderen Quelle, beispielsweise aus der Bleichmittelzusammensetzung und/oder dem verwendeten Wasser, abstammt, den Übergangsmetall aufweisenden Komplex ausbildet. Vorzugsweise wird dieser Übergangsmetall aufweisende Komplex in situ in dem Anwendungsmedium gebildet.

Der erfindungsgemäß verwendbare mit wenigstens einem Liganden, vorzugsweise organischem Liganden, an einen Träger kovalent gebundene Bleichkatalysatorkomplex weist wenigstens ein Übergangsmetall auf.

Das oder die Übergangsmetall(e) sind vorzugsweise ausgewählt aus der Gruppe umfassend: Mn in den Oxidationsstufen II-V, Fe I-IV, Cu I-III, Co I-III, Ni I-III, Cr II-VII, Ag I-II, Ti II-IV, W IV-VI, Pd II, Ru II-V, V II-V und/oder Mo II-VI.

Der trägerfixierte Bleichkatalysator kann bei einem pH-Wert von zwischen 7-14, vorzugsweise 8-10 und bevorzugt ≥9,5 regeneriert werden.

Erfindungsgemäß bevorzugt sind trägerfixierte Komplexe mit Übergangsmetallen verwendbar, wie Mangan, Eisen, Cobalt, Ruthenium, Molybdän, Titan, Vanadium und/oder Kupfer. Liganden, die mit einem Träger eine kovalente Bindung ausbilden können weisen die allgemeine nachstehende Formel I auf, 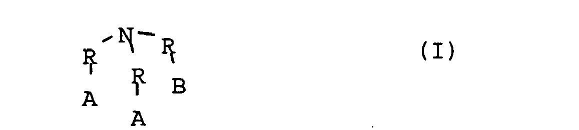 in denen R eine direkte Bindung oder eine gegebenenfalls aminogruppensubstituierte Alkylengruppe mit 1 bis 4 C-Atomen ist, A für ein kondensiertes oder nichtkondensiertes Ringsystem steht, welches mindestes ein Stickstoffatom enthält, und B Wasserstoff, eine OH-Gruppe oder A ist.

Derartige über solche Ligand(en) trägerfixiert(e) Komplexe können als Katalysatoren, insbesondere für anorganische Persauerstoffverbindungen in Oxidations-, Wasch-, Reinigungs- oder Desinfektionslösungen, verwendet werden. In den Alkylengruppen R können eine oder mehrere nicht benachbarte CH₂-Einheiten, die nicht direkt an das zentrale N-Atom gebunden sind, gegebenenfalls durch NH-Einheiten ersetzt sein.

Kovalente Fixierungen von Verbindungen der allgemeinen Formel I auf polymeren Trägern stellen eine besondere synthetische Herausforderung dar, weil im Gegensatz zu anderen Ligandensystemen (z. B. makrozyklischen Systemen) erst entsprechende Ankergruppen eingeführt werden müssen.

Verbindungen der allgemeinen Formel I können durch formale verknüpfende Modifikation ihrer B-Einheiten zu Liganden der allgemeinen Formel II umgewandelt werden, 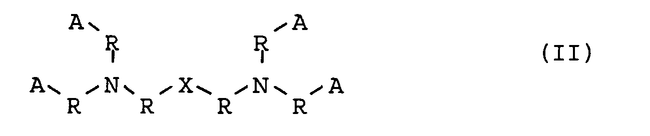 in denen A und R die oben gegebene Bedeutung haben und X ein gegebenenfalls hydroxy- und/oder C₁₋₄-alkylsubstituierter Phenylenring oder eine gegebenenfalls hydroxysubstituierte C₁₋₄-Alkylengruppe ist, und die in der Lage sein können, jeweils zwei Übergangsmetallatome komplex zu binden. Ein weiterer Gegenstand der Erfindung ist daher die Verwendung von gegebenenfalls zwei- oder mehrkernigen Komplexen, die über wenigstens einen Liganden kovalent trägerfixiert sind, der Übergangsmetalle Mangan, Eisen, Cobalt, Ruthenium, Molybdän, Titan, Vanadium und/oder Kupfer, die einen oder mehrere der Liganden der allgemeinen Formel II aufweisen. Derartig trägerfixierte erfindungsgemäße Komplexe können als Katalysatoren für insbesondere anorganische Persauerstoffverbindungen in Oxidations-, Wasch-, Reinigungs- oder Desinfektionslösungen, eingesetzt werden.

In dem Teil R-B der Verbindungen gemäß Formel I ist R vorzugsweise eine direkte Bindung, wenn B Wasserstoff ist, und vorzugsweise keine direkte Bindung, wenn B eine Hydroxylgruppe ist. Es müssen nicht sämtliche Gruppen R in einem Ligandenmolekül identisch sein. Zu den bevorzugten stickstoffhaltigen Ringsystemen (A in den Formeln I und II) gehören die 2-Pyridylgruppe, die 2-Imidazolylgruppe, die 1-Methyl-2-Imidazolylgruppe und die 2-Benzimidazolylgruppe. Es müssen nicht sämtliche Reste A in einem Ligandenmolekül identisch sein. R in den Verbindungen der Formeln I oder II ist vorzugsweise eine Methylengruppe. X in den Verbindungen gemäß Formel II wird vorzugsweise unter der 1,3-Phenylengruppe, der 2-Hydroxy-1,3-Phenylengruppe, der 2-Hydroxy-5-Methyl-1,3-Phenylengruppe und der Hydroxymethylengruppe ausgewählt.

Liganden gemäß den allgemeinen Formeln I oder II können nach im Prinzip bekannten Verfahren, wie zum Beispiel in dem Übersichtsartikel von K. Wieghardt in Angew. Chem. 101 (1995), S. 1179 - 1198 und den dort zitierten Originalarbeiten beschrieben, hergestellt werden. Üblicherweise werden kommerziell verfügbare Rohstoffe über Kondensationsreaktionen, beispielsweise unter Abspaltung von Halogenwasserstoffen, zu den gewünschten Liganden umgesetzt. Diese können anschließend, üblicherweise in gängigen Lösungsmitteln, mit Salzen entsprechender Übergangsmetalle umgesetzt werden. Die erfindungsgemäß als Bleichkatalysatoren zu verwendenden Komplexe bilden sich in der Regel bereits bei Raumtemperatur und werden normalerweise aus gängigen Lösungsmitteln in kristalliner Form erhalten.

Die genannten Übergangsmetalle in den erfindungsgemäß zu verwendenden Bleichkatalysatoren liegen vorzugsweise in den Oxidationsstufen +2, +3 oder +4 vor. Bevorzugt werden Komplexe mit Übergangsmetallzentralatomen in den Oxidationsstufen +3 oder +4 verwendet. Systeme mit gemischten Oxidationszahlen sind möglich. Im Falle mehrkerniger Komplexe müssen nicht alle Metallatome im Komplex gleich sein. Zu den bevorzugt verwendeten Komplexen gehören diejenigen mit Eisen und/oder Mangan als Zentralatomen.

Außer den Liganden gemäß den allgemeinen Formeln I und II können die erfindungsgemäß zu verwendenden Übergangsmetallkomplexe noch weitere, in der Regel einfacher aufgebaute Liganden, insbesondere ein- oder mehrwertige Anionliganden, tragen. In Frage kommen beispielsweise Nitrat, Acetat, Formiat, Citrat, Perchlorat und die Halogenide wie Chlorid, Bromid und lodid sowie komplexe Anionen wie Hexafluorophosphat. Die Anionliganden sorgen für den Ladungsausgleich zwischen Übergangsmetall-Zentralatom und dem Ligandensystem. Auch die Anwesenheit von Oxo-Liganden, Peroxo-Liganden und Imino-Liganden ist möglich. Diese zusätzlichen Liganden können auch verbrückend wirken, so daß mehrkernige Komplexe mit mindestens einem Liganden gemäß den allgemeinen Formeln I oder II entstehen.

Der oder die Liganden können über reaktive Gruppen wie OH, N, H, Halogen, Mehrfachbindungen, insbesondere Doppelbindungen oder dergleichen mit wenigstens einer reaktiven Gruppe des Trägers unter Ausbildung einer kovalenten Bindung trägerfixiert werden. Kovalente Einfachbindungen zwischen Träger und Ligand können beispielsweise über N-Atom des Liganden mittels Quarternisierung erreicht werden. Die Ausbildung von Mehrfach- und/oder Einfachbindungen zwischen Ligand(en) und Träger umfassen Substitutions- und/oder Kondensationsreaktionen. Zwischen dem Träger und dem Liganden kann eine Esterbindung oder auch eine Etherbindung ausgebildet werden. Prinzipiell sind alle Einfach- und Mehrfachbindungen zwischen Träger und Ligand möglich, solange der Ligand noch koordinierungsfähig bleibt. Bevorzugt ist, wenn das Trägerpolymer wenigstens eine zur Ausbildung einer kovalenten Bindung geeigneten Substituenten und/oder funktionelle Gruppe enthält, vorzugsweise ausgewählt aus der Gruppe umfassend -H, -OH, -NH₂, -NH-R, -Halogen, -SH, -Si-OR, -C=C, -C≡C, -OR, -NCO, -COOH, -COOR, -CHO, -CN, -NH-C=O, -O=C-O-C=O und/oder Epoxid.

Zu den bevorzugten Liganden, die kovalent an einen Träger bindbar sind, zwecks Bildung von Bleichkatalysatoren gemäß der Erfindung, gehören solche mit dem:
Tris-(2-pyridylmethyl)amin-Liganden, 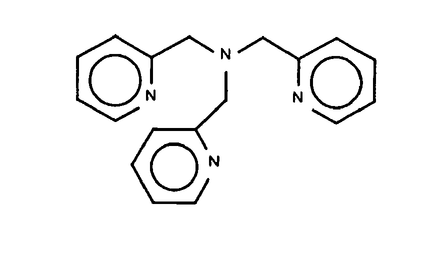 mit dem (Bis-((1-methylimidazol-2-yl)-methyl))-(2-pyridylmethyl)-amin-Liganden, 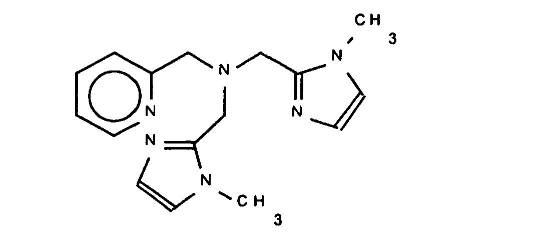 mit dem N-Bis-(2-benzimodazolylmethyl)-aminoethanol-Liganden, 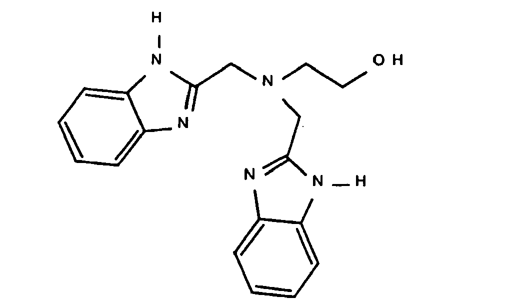 und mit dem N,N'-(Bis-(1-methylimidazol-2-yl)-methyl)-ethylendiamin-Liganden,

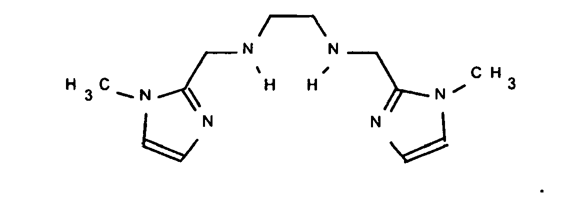

Zu den bevorzugten Liganden gemäß allgemeiner Formel II gehören 2,6-Bis-(bis-(2-benzimidazolylmethyl)aminomethyl)-4-methylphenol, 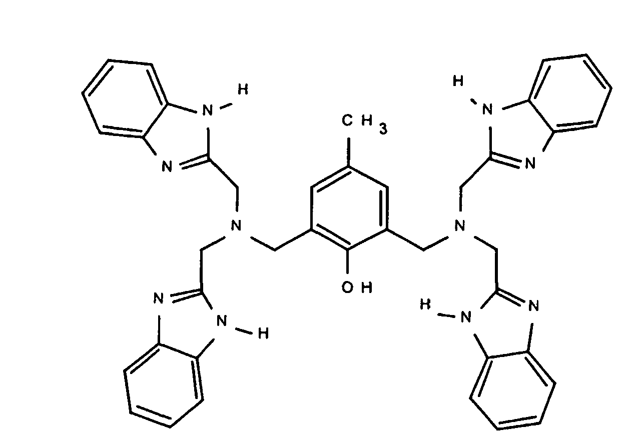 N,N,N',N'-Tetrakis-(2-benzimidazolylmethyl)-2-hydroxy-1,3-diaminopropan, 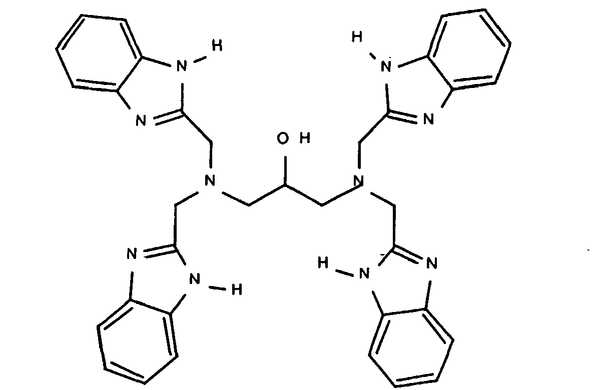 1,3-Bis-(bis-(2-benzimidazolylmethyl)aminomethyl)-benzol, 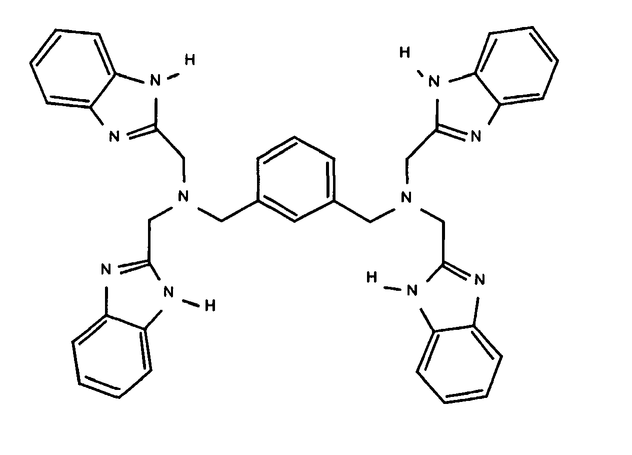 und 2,6-Bis-(bis-(2-pyridylmethyl)aminomethyl)-4-methylphenol,

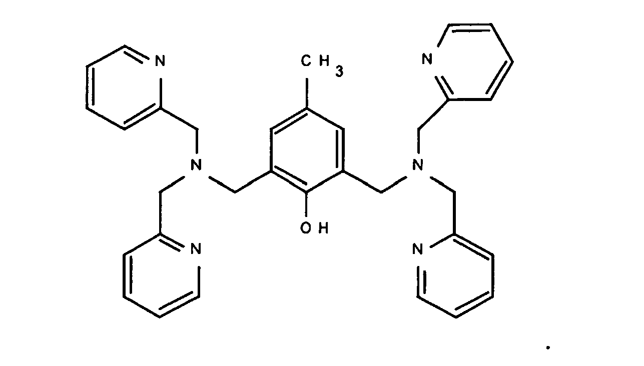

Ein trägerfixierter Übergangsmetall-Bleichkatalysator, mit den kovalent an einen Träger bindbaren Liganden gemäß Formel I oder Formel II, wird zum Bleichen von farbigen Anschmutzungen, wie Farbanschmutzungen beim Waschen von Textilien, insbesondere in wäßriger, tensidhaltiger Flotte, verwendet. Die Formulierung "Bleichen von Farbanschmutzungen" ist dabei in ihrer weitesten Bedeutung zu verstehen und umfaßt sowohl das Bleichen von sich auf dem Textil befindendem Schmutz, das Bleichen von in der Waschflotte befindlichem, vom Textil abgelöstem Schmutz als auch das oxidative Zerstören von sich in der Waschflotte befindenden Textilfarben, die sich unter den Waschbedingungen von Textilien ablösen, bevor sie auf andersfarbige Textilien aufziehen können.

Eine weitere Anwendungsform gemäß der Erfindung ist die erfindungsgemäße Verwendung der trägerfixierten Übergangsmetall-Bleichkatalysatoren, mit den kovalent an einen Träger bindbaren Liganden gemäß Formel I oder Formel II, in Reinigungslösungen für harte Oberflächen, insbesondere für Geschirr, zum Bleichen von gefärbten Anschmutzungen. Auch dabei wird unter dem Begriff der Bleiche sowohl das Bleichen von sich auf der harten Oberfläche befindendem Schmutz als auch das Bleichen von in der Geschirrspülflotte befindlichem, von der harten Oberfläche abgelöstem Schmutz verstanden.

Erfindungsgemäß bevorzugt verwendbare dipodale Liganden, bei denen die kovalente Bindung mit dem Träger, beispielsweise einem Merrifield-Harz, über das zentrale sekundäre Amin des Liganden ausbildbar ist, sind nachfolgend dargestellt: 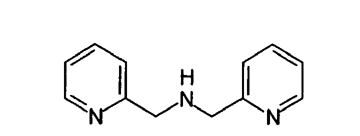 Bis(2-pyridylmethyl)amin 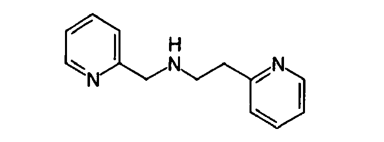 (2-Pyridylmethyl)(2-(2-pyridyl)-ethyl)amin 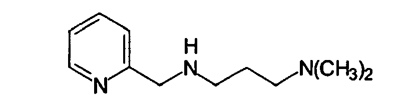 (2-Pyridylmethyl)(3-(N,N-dimethylamino)-propyl)amin 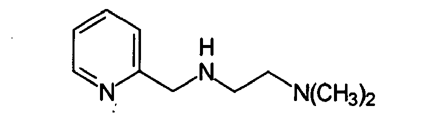 (2-Pyridylmethyl)(2-(N,N-dimethylamino)-ethyl)amin 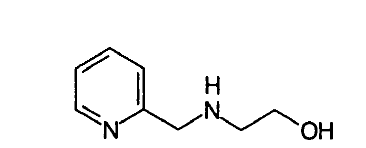 (2-Pyridylmethyl)(2-hydroxyethyl)amin 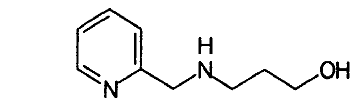 (2-Pyridylmethyl)(3-hydroxypropyl)amin 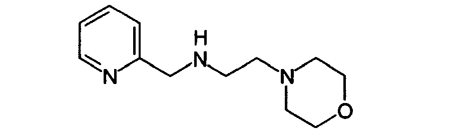 (2-Pyridylmethyl)(2-N-morpholinoethyl)amin 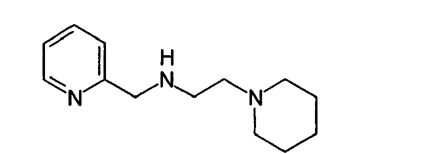 (2-Pyridylmethyl)(2-N-piperidinoethyl)amin 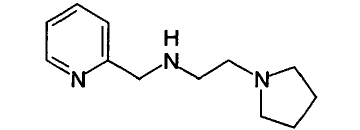 (2-Pyridylmethyl)(2-N-pyrrolidinoethyl)amin 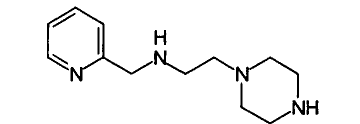 (2-Pyridylmethyl)(2-N-piperazinoethyl)amin 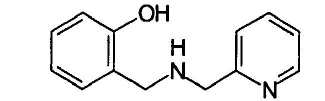 (2-Hydroxybenzyl)(2-pyridylmethyl)amin 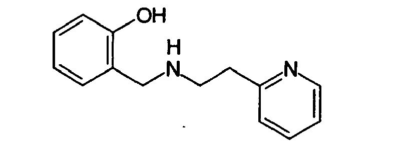 (2-Hydroxybenzyl)(2-(2-pyridyl)-ethyl)amin 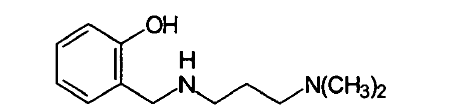 (2-Hydroxybenzyl)(3-(N,N-dimethylamino)-propyl)amin 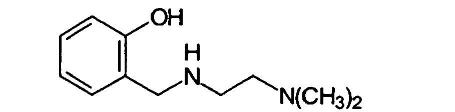 (2-Hydroxybenzyl)(2-(N,N-dimethylamino)-ethyl)amin 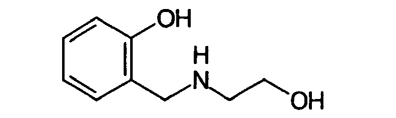 (2-Hydroxybenzyl)(2-hydroxyethyl)amin 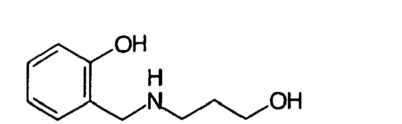 (2-Hydroxybenzyl)(3-hydroxypropyl)amin 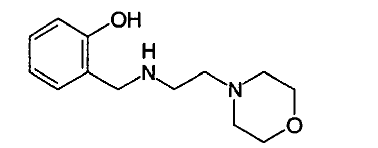 (2-Hydroxybenzyl)(2-N-morpholinoethyl)amin 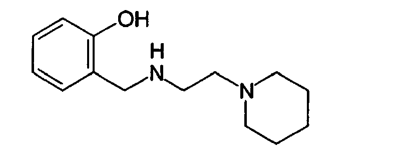 (2-Hydroxybenzyl)(2-N-piperidinoethyl)amin 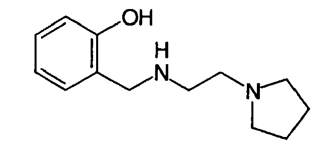 (2-Hydroxybenzyl)(2-N-pyrrolidinoethyl)amin 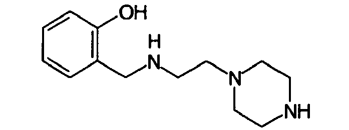 (2-Hydroxybenzyl)(2-N-piperazinoethyl)amin

Nachfolgend sind erfindungsgemäß besonders bevorzugt verwendbare tripodale Liganden, wobei die kovalente Bindung zwischen dem Träger, beispielsweise ein Merrifield-Harz, und den nachstehenden tripodalen Liganden, bevorzugt über die Methylgruppe in 6-Position des tripodalen Liganden, ausbildbar ist, aufgeführt: 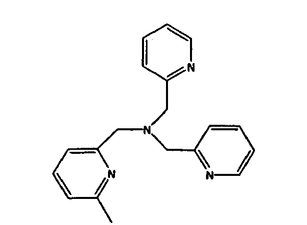 ((6-Methyl-2-pyridyl)methyl)bis(2-pyridylmethyl)amin 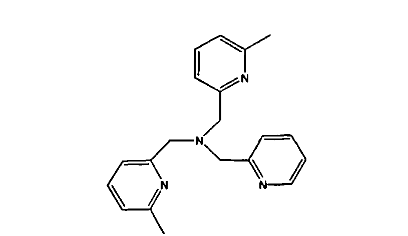 Bis((6-Methyl-2-pyridyl)methyl)(2-pyridylmethyl)amin 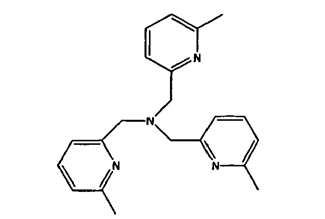 Tris((6-methyl-2-pyridyl)methyl)amin 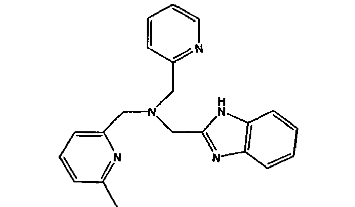 [(Benzimidazol-2-yl)methyl][(6-Methyl-2-pyridyl)methyl)(2-pyridyl)methyl]amin 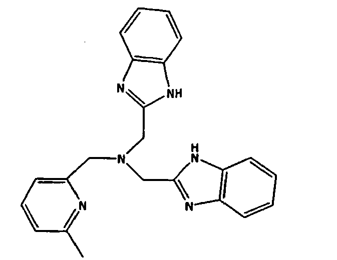 Bis[(Benzimidazol-2-yl)methyl][(6-Methyl-2-pyridyl)methyl)]amin 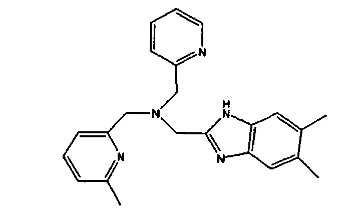 [(5,6-dimethylbenzimidazol-2-yl)methyl][(6-Methyl-2-pyridyl)methyl)(2-pyridyl)methyl]amin 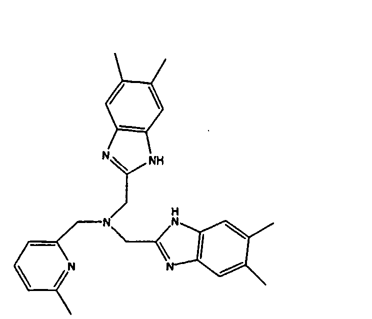 Bis[(5,6-dimethylbenzimidazol-2-yl)methyl][(6-Methyl-2-pyridyl)methyl)]amin 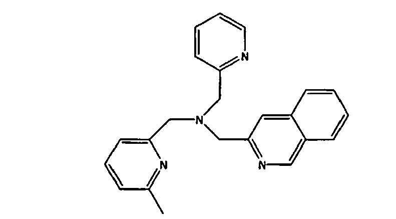 [(2-pyridyl)methyl(6-Methyl-2-pyridyl)(2-chinolyl)methyl]amin 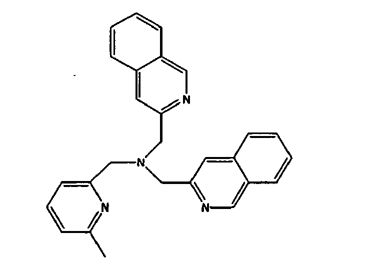 Bis[(2-chinolyl)(6-Methyl-2-pyridyl) methyl]amin 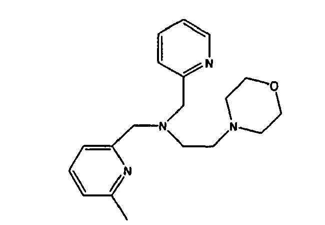 [(2-pyridyl)methyl](2-N-morpholinoethyl)][(6-methyl-2-pyridyl)-methyl]amin 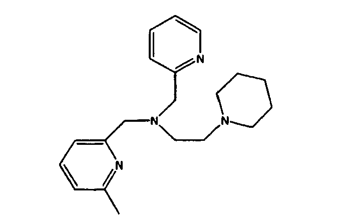 [(2-pyridyl)methyl](2-N-piperidinoethyl)][(6-methyl-2-pyridyl)-methyl]amin 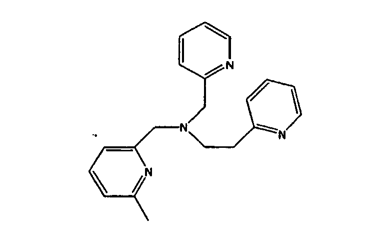 *pmap* [2-(2-pyridyl)ethyl][(2-pyridyl)methyl])][(6-methyl-2-pyridyl)-methyl]amin 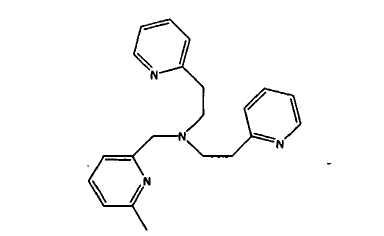 *pmea* [(2-pyridyl)methyl][2-(2-pyridyl)ethyl])][(6-methyl-2-pyridyl)-methyl]amin

Erfindungsgemäß bevorzugt verwendbare Heptadentate Liganden, bei denen die kovalente Bindung mit dem Träger, beispielsweise ein Merrifield-Harz, über die zentrale OH-Gruppe des Liganden ausbildbar ist, sind nachfolgend dargestellt: 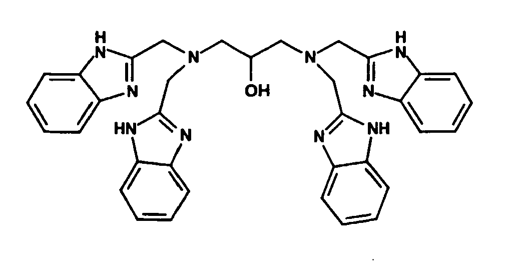 N,N,N',N'-Tetrakis[2-benzimidazolylmethyl]-1,3-diamino-2-propanol 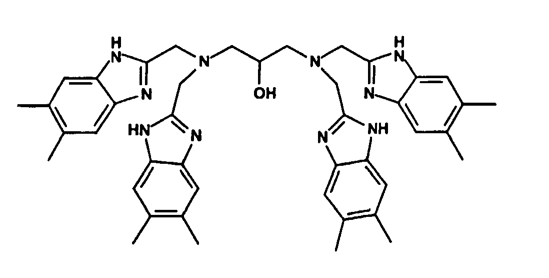 N,N,N',N'-Tetrakis[2-(5,6-dimethyl)-benzimidazolylmethyl]-1,3-diamino-2-propanol 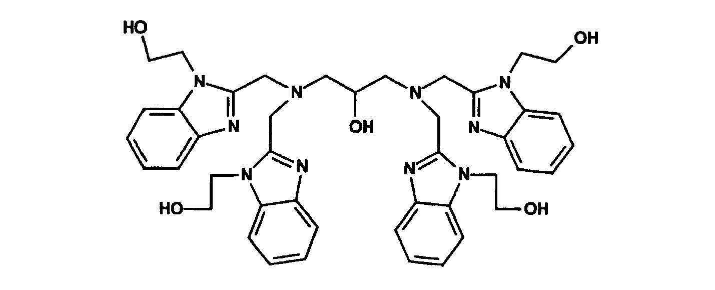 N,N,N',N'-Tetrakis[2-(2-hydroxyethyl)-benzimidazolylmethyl]--1,3-diamino-2-propanol 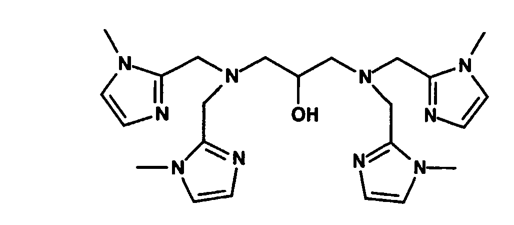 N,N,N',N'-Tetrakis[2-(1-methyl)-imidazolylmethyl]-1,3-diamino-2-propanol

Weitere erfindungsgemäß bevorzugt verwendbare Bleichkatalysatorkomplex(e) die mit wenigstens einem organischem Liganden an einen Träger kovalent gebunden werden können, weisen die nachstehende allgemeine Formel (A1) auf:

[MₐLₖXₙ]Yₘ

worin:
M steht für ein Metall ausgewählt aus der Gruppe: Mn(II)-(III)-(IV)-(V), Cu(I)-(II)-(III), Fe(I)-(II)-(III)-(IV), Co(I)-(II)-(III), Ni(I)-(II)-(III), Cr(II)-(III)-(IV)-(V)-(VI)-(VII), Ti(II)-(III)-(IV), V(II)-(III)-(IV)-(V), Mo(II)-(III)-(IV)-(V)-(VI), W(IV)-(V)-(VI), Pd(II), Ru(II)-(III)-(IV)-(V) und/oder Ag(I)-(II); vorzugsweise Mn(II)-(III)-(IV)-(V), Cu(I)-(II), Fe(II)-(III)-(IV) und/oder Co(I)-(II)-(III);
L steht für einen in der Beschreibung aufgeführten Liganden, der gegebenfalls protoniert oder deprotoniert sein kann;
X steht für ein koordinierfähiges Molekül und/oder eine Gruppe, umfassend mono-, bi- oder tri-geladene Anionen und/oder ein beliebiges neutrales Molekül geeignet zur mono-, bi- oder tridentat-Koordination eines Metalls, wie O²⁻, RBO₂²⁻, RCOO⁻, RCONR⁻, OH⁻, NO₃⁻, NO₂⁻, NO, CO, S²⁻, RS⁻, PO₄³⁻, PO₃OR³⁻, H₂O, CO₃²⁻, HCO³⁻, ROH, NRR'R", RCN, Cl⁻, Br⁻, OCN⁻, SCN⁻, CN⁻, N3⁻, F⁻, l⁻, RO⁻, ClO₄⁻, SO₄²⁻, HSO₄⁻, SO₃²⁻ und/oder RSO₃⁻, und besonders bevorzugt O²⁻, RBO₂²⁻, RCOO⁻, OH⁻, NO₃⁻, NO₂⁻, NO, CO, CN⁻, S²⁻, RS⁻, PO₄³⁻, H₂O, CO₃²⁻, HCO³⁻, ROH, NRR'R", RCN, Cl⁻, Br⁻, OCN⁻, SCN⁻, N3⁻, F⁻, l⁻, RO⁻, ClO₄⁻, SO₄²⁻, HSO₄⁻, SO₃²⁻ und/oder RSO₃⁻ und noch bevorzugter CF₃SO₃⁻;
Y steht für ein nicht koordinierendes Gegenion, vorzugsweise ausgewählt aus der Gruppe, umfassend ClO₄⁻, BR₄⁻, [FeCl₄]⁻, PF₆⁻, RCOO⁻, NO₃⁻, NO₂⁻, RO⁻, N⁺RR'R"R"', Cl⁻, Br⁻, F⁻, l⁻, RSO₃⁻, S₂O₆²⁻, OCN⁻, SCN⁻, Li⁺, Ba²⁺, Na⁺, Mg²⁺, K⁺, Ca ²⁺, Cs⁺, PR₄⁺, RBO₂²⁻, SO₄²⁻, HSO₄⁻, SO₃²⁻, SbCl₆⁻, CuCl₄²⁻, CN, PO₄³⁻, HPO₄²⁻, H₂PO₄⁻, CO₃ ²⁻, HCO₃⁻ und/oder BF₄⁻ und noch bevorzugter ClO₄⁻, BR₄⁻, [FeCl₄]⁻, PF₆⁻, RCOO⁻, NO₃⁻, NO₂⁻, RO⁻, N⁺RR'R"R"', Cl⁻, Br⁻, F⁻, l⁻, RSO₃⁻, CF₃SO₃⁻, S₂O₆²⁻, OCN⁻, SCN⁻, Li⁺, Ba²⁺, Na⁺, Mg²⁺, K⁺, Ca²⁺, PR₄⁺, SO₄²⁻, HSO₄⁻, SO₃²⁻ und/oder BF₄⁻;
R, R', R", R'" stehen unabhängig voneinander für -H, -OH, -OR (worin R=Alkyl, Alkenyl, Cycloalkyl, Heterocycloalkyl, Aryl, Heteroaryl oder Carbonyl - Gruppe), - OAr, Alkyl, Alkenyl, Cycloalkyl, Heterocycloalkyl, Aryl, Heteroaryl und/oder Carbonyl - Gruppe, jedes R, Ar, Alkyl, Alkenyl, Cycloalkyl, Heterocycloalkyl, Aryl, Heteroaryl und/oder Carbonyl - Gruppe kann optional durch wenigstens eine Gruppe E substituiert sein oder R6 zusammen mit R7 und/oder unabhängig davon R8 zusammen mit R9 stehen für O, worin E für funktionelle Gruppen steht umfassend O, S, P, N, Se, Halogen und/oder jeden beliebigen Elektronen Donor- und/oder ziehende Gruppen steht; und R, R', R", R'" vorzugsweise steht für H, substituiertes Alkyl oder substituiertes Aryl, noch bevorzugter für H oder substituiertes Phenyl, Naphthyl oder C1-4-alkyl;

a = steht für eine ganze Zahl von zwischen 1 bis 10, vorzugsweise 1 bis 4;
k = steht für eine ganze Zahl von zwischen 1 bis 10;
n = 0 oder steht für eine ganze Zahl von zwischen 1 bis 10, vorzugsweise 1 bis 4;
m = 0 oder steht für eine ganze Zahl von zwischen 1 bis 20, vorzugsweise 1 bis 8.

Vorzugsweise weist der kovalent mit dem Träger verbindbare organische Ligand L die allgemeine Formel (BI) auf: 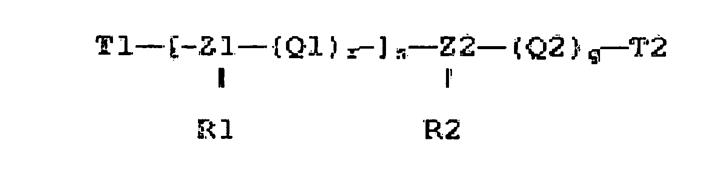 worin:
g = 0 oder steht für eine ganze Zahl von zwischen 1 bis 6;
r = steht für eine ganze Zahl von zwischen 1 bis 6;
s = 0 oder steht für eine ganze Zahl von zwischen 1 bis 6;
Z1, Z2 stehen unabhängig voneinander für ein Heteroatom oder einen heterocyclischen oder heteroaromatischen Ring, Z1 und/oder Z2 können optional substituiert sein mit wenigstens einer funktionalen Gruppe E, wie unten angegeben;
Q1, Q2 stehen unabhängig für eine Gruppe mit der nachstehenden Formel: 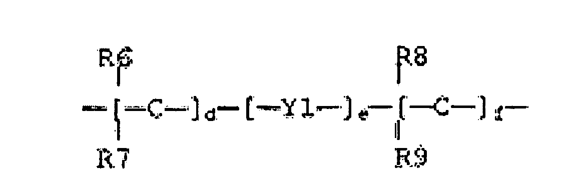 worin:
10>d+e+f>1; d=0-9; e=0-9; f=0-9;
jedes Y1 ist unabhängig voneinander ausgewählt aus der Gruppe -O-, -S-, -SO-, - SO₂⁻, -(G¹)N-, -( G¹)( G²)N- (worin G¹ und/oder G² nachfolgend definiert sind), - C(O)-, Arylene, Alkylene, Heteroarylene, -P- und/oder -P(O)-;
wenn s>1, jede -[-Z1 (R1)-(Q1 )r-]- Gruppe ist unabhängig voneinander definiert;
R1, R2, R6, R7, R8, R9 stehen unabhängig voneinander für -H, -OH, -OR (worin R=Alkyl, Alkenyl, Cycloalkyl, Heterocycloalkyl, Aryl, Heteroaryl oder Carbonyl - Gruppe), -OAr, Alkyl, Alkenyl, Cycloalkyl, Heterocycloalkyl, Aryl, Heteroaryl und/oder Carbonyl - Gruppe, jedes R, Ar, Alkyl, Alkenyl, Cycloalkyl, Heterocycloalkyl, Aryl, Heteroaryl und/oder Carbonyl - Gruppe können durch wenigstens eine funktionelle Gruppe E substituiert sein, oder R6 zusammen mit R7 und/oder unabhängig davon R8 zusammen mit R9 Sauerstoff sein;
E steht für funktionelle Gruppen, enthaltend O, S, P, N, Se, Halogen, und/oder jeden beliebigen Elektronendonator und/oder jede beliebige elektronenziehende Gruppe, vorzugsweise steht E für -OH, mono- oder Polycarboxylat - Derivate, Aryl, Heteroaryl, Sulphonat, Thiol (-RSH), Thioether (-R-S-R'), Disulphide (-RSSR'), Dithiolene, Mono- oder Polyphosphonate, Mono- der Polyphosphate, Elektronen-Donor-Gruppen und/oder elektronenziehende Gruppen, und/oder Gruppen mit der Formel (G¹)( G²)N-, (G¹)( G²) (G³)N-, (G¹)( G²)N-C(O)-, G³O- und/oder G³C(O)-,
worin jedes G¹, G² und/oder G³ unabhängig voneinander steht für H, Alkyl, Electronen-Donor-Groupen und/oder Elektronen ziehende Gruppen, einschließlich der vorgenannten;
R1-R9 kann eine verbrückende Gruppe sein, vorzugsweise gebunden mit einem anderen Teil eines Vertreters der selben allgemeinen Formel;

T1, T2 stehen unabhängig voneinander für R4 oder R5, worin R4 oder R5 definiert ist wie R1-R9, oder wenn g=0 und/oder s>0, R1 mit R4, und/oder R2 mit R5, können unabhängig voneinander für =CH-R10 stehen, worin R10 definiert ist wie R1-R9; oder
T1, T2 kann -T2-T1- sein, optional können sie eine kovalente Bindung ausbilden, wenn s>1 und/oder g>0;
wenn Z1 und/oder Z2 = N oder T1 und T2 stehen für eine für eine Einfachbindung und/oder R1 und/oder R2 fehlen, Q1 und/oder Q2 stehen unabhängig voneinander für eine Gruppe der allgemeinen Formel:

=CH-[-Y1-]e-CH=,

worin wenigstens zwei von R1, R2, R6, R7, R8 und/oder R9 unabhängig voneinander mittels einer kovalenten Bindung verbunden sind;
wenn Z1 und/oder Z2 = Sauerstoff, können R1 und/oder R2 fehlen;
wenn Z1 und/oder Z2 = S, N, P, B oder Si dann können R1 und/oder R2 fehlen; wenn Z1 und/oder Z2 = für ein Heteroatom stehen, substituiert mit einer funktionalen Gruppe E, dann können R1 und/oder R2 und/oder R4 und/oder R5 fehlen.

Die Gruppen Z1 und/oder Z2 stehen für ein Heteroatom wie N, P, 0, S, B und/oder Si, vorzugsweise für einen substituierten heterocyclischen Ring oder gegebenenfalls für einen substituierten heteroaromatischen Ring ausgewählt aus der Gruppe, umfassend Pyridine, Pyrimidine, Pyrazine, Pyrazidine, Pyrazole, Pyrrole, Imidazole, Benzimidazole, Quinoline, Isoquinoline, Carbazole, Triazole, Indole, Isoindole, Furane, Thiophene, Oxazole und/oder Thiazole.

Die Gruppe R1-R9 ist vorzugsweise -H, Hydroxy-C₀-C₂₀-alkyl, Halo- C₀-C₂₀-alkyl, Nitroso, Formyl- C₀-C₂₀-alkyl, Carboxyl- C₀-C₂₀-alkyl und/oder Ester und/oder Salze davon, Carbamoyl- C₀-C₂₀-alkyl, Sulpho- C₀-C₂₀-alkyl und/oder Ester und/oder Salze davon, Sulphamoyl- C₀-C₂₀-alkyl, Amino- C₀-C₂₀-alkyl, Aryl- C₀-C₂₀-alkyl, Heteroaryl- C₀-C₂₀-alkyl, C₀-C₂₀-Alkyl, Alkoxy- C₀-C₈-alkyl, Carbonyl- C₀-C₆-alkoxy und/oder Aryl- C₀-C₆-alkyl und/oder C₀-C₂₀-alkylamide.

Wenigstens ein R1-R9 kann eine verbrückende Gruppe sein, welches ein Ligandmolekül mit einem Molekül eines anderen Liganden verbindet, vorzugsweise mit einem Liganden der gleichen Formel.

Die verbrückende Gruppe hat vorzugsweise die Formel -Cn'(R11)(R12)-(D)p-Cm'(R11)(R12)- und verbindet die beiden Moleküle, worin p is 0 oder 1, D ist ein Heteroatom oder eine ein Heteroatom-enthaltende Gruppe, oder ist Bestandteil eines aromatischen oder gesättigten homonuclearen oder heteronuclearen Rings, n' ist eine ganze Zahl von zwischen 1 bis 4, m' ist eine ganze Zahl von zwischen 1 bis 4, mit der Ausnahme, dass wenn n'+m' ≤ 4 ist, R11 und/oder R12 sind unabhängig ausgewählt aus der Gruppe, umfassend -H, NR13, und/oder OR14, Alkyl, Aryl, optional substituiert, und R13 und/oder R14 = -H, Alkyl, Aryl, beide optional substituiert. Alternativ oder zusätzlich können wenigstens zwei von R1-R9 Brückengruppen verbindende Atome sein, vorzugsweise Heteroatome, wobei die verbrückende Gruppe Alkylen, Hydroxyalkylen oder Heteroaryl aufweist. Eine weitere erfindungsgemäß verwendbare modifizierte Gruppe basiert auf der Formel (BI), wobei die Gruppen T1 und/oder T2 eine Einfachbindung ausbilden und s>1 ist, gemäß der allgemeinen Formel (BII): 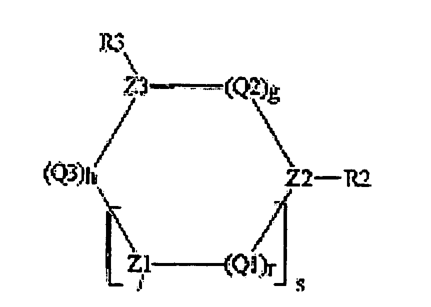 worin Z3 unabhängig voneinander für eine Gruppe, definiert wie Z1 oder Z2; R3 unabhängig voneinander für eine Gruppe, definiert wie R1-R9; Q3 unabhängig voneinander für eine Gruppe definiert wie Q1, Q2; h = 0 oder 1 bis 6; und/oder s'=s-1, stehen.

Vorzugsweise erfindungsgemäß verwendbar sind organische Liganden gemäß der allgemeinen Formel (BII), worin s'=1, 2 oder 3; r=g=h=1; d=2 oder 3; e=f=0; R6=R7=H ist, vorzugsweise so, dass der Ligand(en) eine der nachfolgenden Strukturen aufweist:

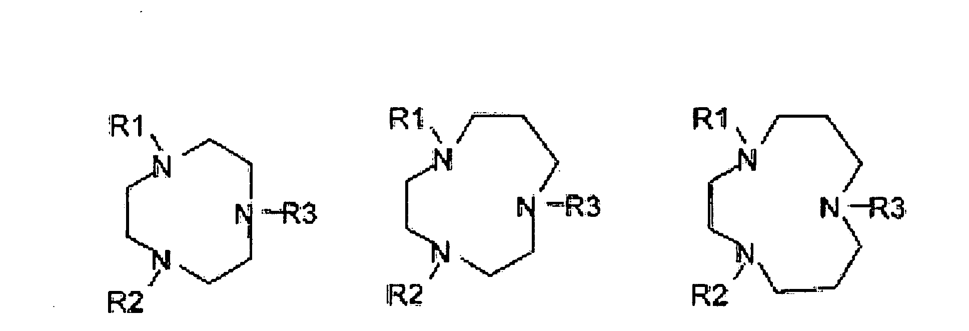

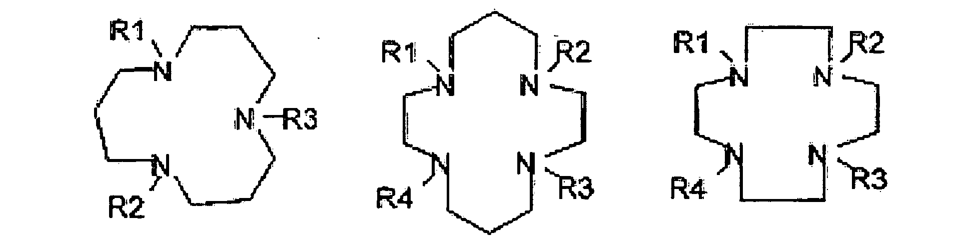

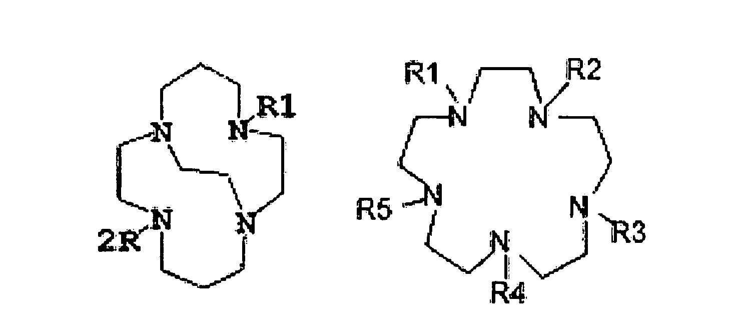

Bei diesen bevorzugten Beispielen ist R1, R2, R3 und/oder R4 unabhängig ausgewählt aus der Gruppe, umfassend -H, Alkyl, Aryl, Heteroaryl, und/oder wenigstens eins der R1-R4 steht für eine verbrückende Gruppe gebunden zu einem Molekül der gleichen Formel und/oder wenigstens zwei der R1-R4 bilden zusammen eine verbrückende Gruppe verbunden mit N Atomen der gleichen Verbindung, vorzugsweise ist die verbrückende Gruppe Alkylen oder Hydroxy-Alkylen oder eine Heteroaryl enthaltende Brücke, vorzugsweise Heteroarylen. Noch bevorzugter, R1, R2, R3 und/oder R4 sind unabhängig voneinander ausgewählt aus der Gruppe, umfassend -H, Methyl, Ethyl, Isopropyl, Stickstoff enthaltendes Heteroaryl, oder eine das Molekül der selben allgemeinen Formel verbrückende Gruppe oder eine das Molekül der selben allgemeinen Formel über N Atome verbrückende Gruppe, wobei diese vorzugsweise Alkylen oder Hydroxy-Alkylen ist.

Der vorgenannte Komplex [MₐLₖXₙ]Yₘ umfasst vorzugsweise:
M=Mn(II)-(IV), Cu(I)-(III), Fe(II)-(III), Co(II)-(III);
X=CH₃CN, OH₂, Cl⁻, Br⁻, OCN⁻, N₃⁻, SCN⁻, OH⁻, O₂⁻, PO₄³⁻, C₆H₅BO₂²⁻, RCOO⁻;
Y=ClO₄⁻, BPh₄⁻, Br⁻, Cl⁻, [FeCl₄]⁻, PF₆⁻, NO₃⁻
a=1, 2, 3, 4;
n=0, 1, 2, 3, 4, 5, 6, 7, 8, 9;
m=1, 2, 3, 4; und/oder
k=1, 2, 4.

Gemäß der allgemeinen Formel (BII) bei der s'=2; r=g=h=1; d=f=0; e=1; und/oder jedes Y1 ist unabhängig voneinander Alkylen oder Heteroarylen. Der Ligand hat vorzugsweise die allgemeine Formel: 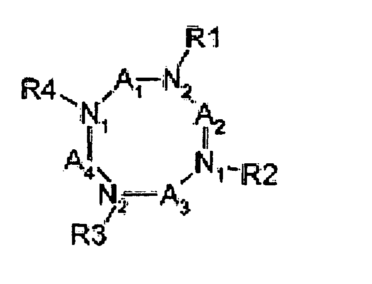 worin:
A1, A2, A3, A4 sind unabhängig voneinander ausgewählt aus der Gruppe, umfassend C1-9-Alkylen oder Heteroarylen Gruppen; und/oder N1 und/oder N2 stehen unabhängig voneinander für ein Heteroatom oder eine Heteroarylen Gruppe.

Vorzugsweise N1 steht für einen aliphatischen Stickstoff, N2 steht für eine Heteroarylen Gruppe, R1, R2, R3, R4 stehen jedes unabhängig voneinander für - H, Alkyl, Aryl oder Heteroaryl, und/oder A1, A2, A3, A4 steht jedes für -CH2-.

Eines der R1-R4 kann für eine das Molekül der selben allgemeinen Formel verbrückende Gruppe stehen und/oder wenigstens zwei der R1-R4 können für eine das Molekül der selben allgemeinen Formel über N-Atome verbrückende Gruppe stehen, vorzugsweise ist die verbrückende Gruppe Alkylen oder Hydroxy-Alkylen oder eine Heteroaryl enthaltende Brücke, vorzugsweise Heteroarylen. Vorzugsweise R1, R2, R3 und/oder R4 sind unabhängig voneinander ausgewählt aus der Gruppe, umfassend -H, Methyl, Ethyl, Isopropyl, Stickstoff enthaltendes Heteroaryl, oder eine das Molekül der selben allgemeinen Formel verbrückende Gruppe oder eine das Molekül der selben allgemeinen Formel über N Atome verbrückende Alkylen oder Hydroxy-Alkylen Gruppe.

Vorzugsweise hat der Ligand die allgemeine Formel: 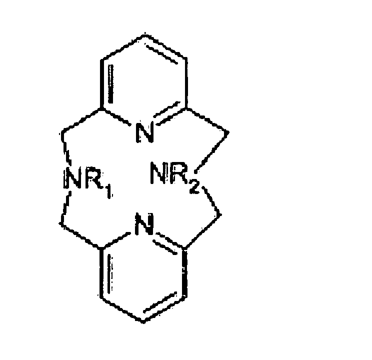 worin jedes R1, R2 unabhängig voneinander stehen für -H, Alkyl, Aryl oder Heteroaryl.

Der Komplex [MₐLₖXₙ]Yₘ umfaßt vorzugsweise:
M=Fe(II)-(III), Mn(II)-(IV), Cu(II), Co(II)-(III);
X= CH₃CN, OH₂, Cl⁻, Br⁻, OCN⁻, N₃⁻, SCN⁻, OH⁻, O₂⁻, PO₄³⁻, C₆H₅BO₂²⁻, RCOO⁻;
Y= ClO₄⁻, BPh₄⁻, Br⁻, Cl-, [FeCl₄]⁻, PF₆⁻, NO₃⁻;
a=1, 2, 3, 4;
n=0, 1, 2, 3, 4, 5, 6, 7, 8, 9;
m=1, 2, 3, 4; und/oder
k=1, 2, 4.

Ein weiterer erfindungsgemäß verwendbarer Ligand der sich kovalent an den Träger verbinden läßt basiert auf der allgemeinen Formel(BII), mit s'=2 und/oder r=g=h=1, gemäß der allgemeinen Formel:
worin: 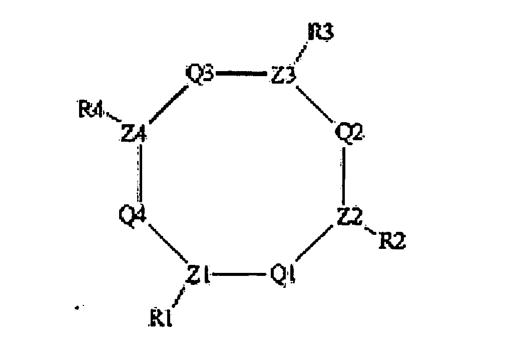 vorzugsweise steht jedes Z1-Z4 für einen heteroaromatischen Ring; e=f=0; d=1; und/oder R7 fehlt, vorzugsweise ist R1=R2=R3=R4 = 2,4,6-Trimethyl-3-SO₃Na-phenyl, 2,6-DiCl-3(oder 4)-SO₃Na-phenyl.

Alternativ, jedes Z1-Z4 steht für N; R1-R4 fehlt; beide Q1 und/oder Q3 stehen für =CH-[-Y1-]ₑ-CH=; und/oder beide Q2 und/oder Q4 stehen für -CH2-[-Y1-]ₙ-CH2-.

Vorzugsweise weist der erfindungsgemäß verwendbare Ligand, der sich kovalent an den Träger binden läßt, die allgemeine Formel auf: 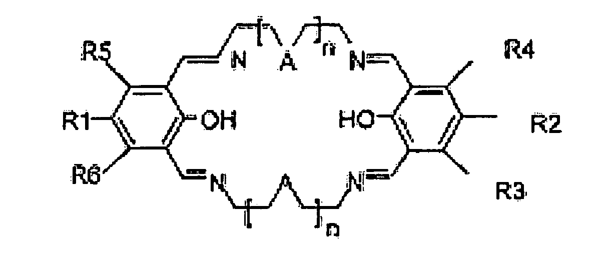 worin:
A steht für ein optional substituiertes Alkylen, optional unterbrochen durch ein Heteroatom; und/oder n ist Null oder eine ganze Zahl von zwischen 1 bis 5.

Vorzugsweise stehen R1-R6 für Wasserstoff, n=1 und/oder A=-CH2-, -CHOH-, - CH2N(R)CH2- oder -CH2CH2N(R)CH2CH2-, worin R steht für Wasserstoff oder Alkyl, weiter bevorzugt steht A für -CH2-, -CHOH- oder -CH2CH2NHCH2CH2-.

Weiter ist bevorzugt wenn der entsprechende Komplex [MₐLₖXₙ]Yₘ umfaßt:
M=Mn(II)-(IV), Co(II)-(III), Fe(II)-(III);
X= CH₃CN, OH₂, Cl⁻, Br⁻, OCN⁻, N₃⁻, SCN⁻, OH⁻, O₂⁻, PO₄³⁻, C₆H₅BO₂²⁻, RCOO⁻;
Y= ClO₄⁻, BPh₄⁻, Br⁻, Cl⁻, [FeCl₄]⁻, PF₆⁻, NO₃⁻;
a=1, 2, 3, 4;
n=0, 1, 2, 3, 4, 5, 6, 7, 8, 9;
m=1, 2, 3, 4; und/oder
k=1,2,4.

Bei einer weiteren Modifikation von (BI), stehen T1 und/oder T2 unabhängig voneinander für die Gruppen R4, R5, wie definiert für R1-R9, gemäß der allgemeinen Formel(BIII):

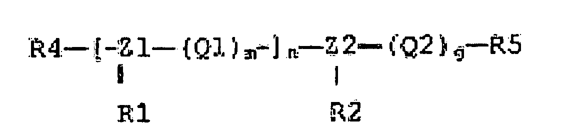

Bei dieser allgemeinen Formel(BIII), ist s=1; r=1; g=0; d=f=1; e=1-4; Y1=-CH2-; und/oder R1 zusammen mit R4, und/oder R2 zusammen mit R5, stehen 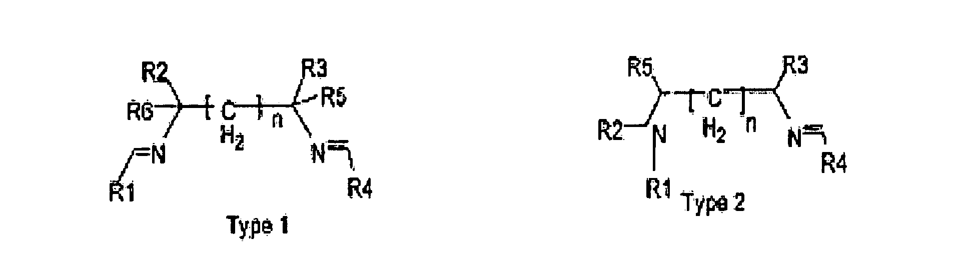 unabhängig voneinander für =CH-R10, worin R10 wie R1-R9 definiert ist. R2 kann zusammen mit R5 für =CH-R10, und R1 und/oder R4 für zwei getrennte Gruppen, stehen. Alternativ können beide R1 zusammen mit R4, und/oder R2 zusammen mit R5 unabhängig voneinander stehen für =CH-R10. Ein bevorzugter, erfindungsgemäß verwendbarer Ligand, der mit einem Träger kovalent verknüpfbar ist, weist die nachfolgende Formel auf:

Vorzugsweise ist dieser Ligand ausgewählt aus der Gruppe umfassend: 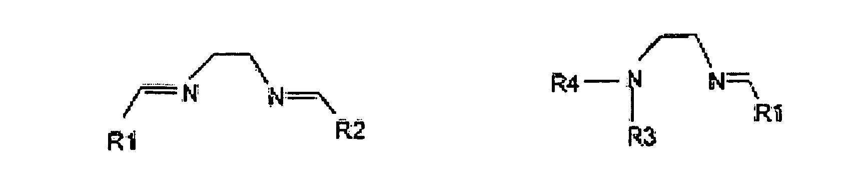 worin:
R1 und/oder R2 ausgewählt sind aus der Gruppe, umfassend Phenole, Heteroaryl-C₀-C₂₀-alkyle, optional substituiert; R3 und/oder R4 sind ausgewählt aus der Gruppe, umfassend -H, Alkyl, Aryl, optional substituierte Phenole, Heteroaryl-C₀-C₂₀-alkyle, Alkylaryl, Aminoalkyl, Alkoxy; weiter bevorzugt R1 und/oder R2 sind ausgewählt aus der Gruppe, umfassend optional substituierte Phenole, Heteroaryl- C₀-C₂-alkyle, R3 und/oder R4 sind ausgewählt aus der Gruppe, umfassend -H, Alkyl, Aryl, optional substituierte Phenole und/oder Stickstoff-Heteroaryl-C₀-C₂-alkyl.

Der entsprechende Komplex [MₐLₖXₙ]Yₘ kann vorzugsweise umfassen:
M=Mn(II)-(IV), Co(II)-(III), Fe(II)-(III);
X= CH₃CN, OH₂, Cl⁻, Br⁻, OCN⁻, N₃⁻, SCN⁻, OH⁻, O₂⁻, PO₄³⁻, C₆H₅BO₂²⁻, RCOO⁻;
Y= ClO₄⁻, BPh₄⁻, Br⁻, Cl⁻, [FeCl₄]⁻, PF₆⁻, NO₃⁻;
a=1, 2, 3, 4;
n=0, 1, 2, 3, 4, 5, 6, 7, 8, 9;
m=1, 2, 3, 4; und/oder
k=1, 2, 4.

Bei einer weiteren Variante gemäß der allgemeinen Formel(BIII), ist s=1; r=1; g=0; d=f=1; e=1-4; Y1=-C(R')(R"), worin R' und/oder R", unabhängig voneinander definiert, wie R1-R9, sind.

Vorzugsweise weist der erfindungsgemäß verwendbare Ligand die allgemeine Formel auf:

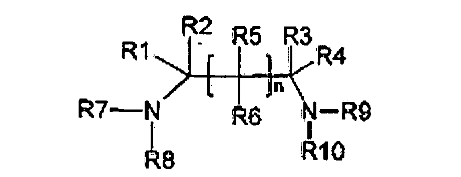

Die Gruppen R1, R2, R3, R4, R5 in dieser Formel sind vorzugsweise -H oder C₀-C₂₀-Alkyl, n=0 oder 1, R6 ist -H, Alkyl, -OH oder -SH, und/oder R7, R8, R9, R10 sind vorzugsweise jedes unabhängig voneinander ausgewählt aus der Gruppe, umfassend -H, C₀-C₂₀-Alkyl, Heteroaryl- C₀-C₂₀-Alkyl, Alkoxy- C₀-C₈-Alkyl und/oder Amino-C₀-C₂₀-Alkyl.

Der entsprechende Komplex [MₐLₖXₙ]Yₘ umfaßt vorzugsweise:
M=Mn(II)-(IV), Fe(II)-(III), Cu(II), Co(II)-(III);
X= CH₃CN, OH₂, Cl⁻, Br⁻, OCN⁻, N₃⁻, SCN⁻, OH⁻, O₂⁻, PO₄³⁻, C₆H₅BO₂²⁻, RCOO ;
Y= ClO₄⁻, BPh₄⁻, Br⁻, Cl⁻, [FeCl₄]⁻, PF₆⁻, NO₃⁻;
a=1, 2, 3, 4;
n=0, 1, 2, 3, 4;
m=0, 1, 2, 3, 4, 5, 6, 7, 8; und/oder
k=1,2,3,4.

Ein weiterer erfindungsgemäß geeigneter Ligand, der kovalent an einen Träger bindet, besitzt die allgemeine Formel(BIII), s=0; g=1; d=e=0; f=1-4. Vorzugsweise hat dieser Ligand die allgemeine Formel:

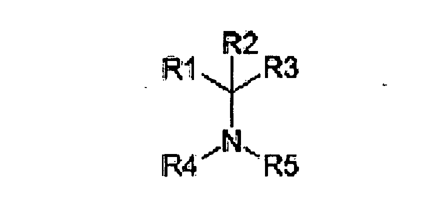

Noch bevorzugter ist, wenn der Ligand die nachfolgende allgemeine Formel aufweist: 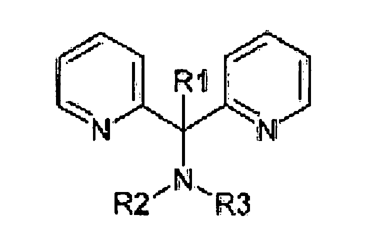 worin:
R1, R2, R3 definiert sind, wie R2, R4, R5.

Der entsprechende Komplex [MₐLₖXₙ]Yₘ ist nachfolgend angegeben:
M=Mn(II)-(IV), Fe(II)-(III), Cu(II), Co(II)-(III);
X= CH₃CN, OH₂, Cl⁻, Br⁻, OCN⁻, N₃⁻, SCN⁻, OH⁻, O₂⁻, PO₄³⁻, C₆H₅BO₂²⁻, RCOO⁻;
Y= ClO₄⁻, BPh₄⁻, Br⁻, Cl⁻, [FeCl₄]⁻, PF₆⁻, NO₃⁻;

a=1, 2, 3, 4;
n=0, 1, 2, 3, 4;
m=0, 1, 2, 3, 4, 5, 6, 7, 8; und/oder
k=1, 2, 3, 4.

Noch ein weiterer erfindungsgemäß verwendbarer Komplex, der über wenigstens einen organischen Liganden an einen Träger kovalent bindbar ist, weist die allgemeine Formel(A) auf:

[LMXₙ]^{z}Y_{q}

worin
M steht für Fe in der Oxidationsstufe II, III, IV oder V, Mn in der Oxidationsstufe II, III, IV, VI oder VII, Cu in der Oxidationsstufe I, II oder III, Co in der Oxidationsstufe II, III oder IV, oder Cr in der Oxidationsstufe II-VI;
X steht für ein koordinationsfähiges Atom oder Molekül;
n steht für Null oder eine ganze Zahl im Bereich von zwischen 0 bis 3;
z steht für die Ladung des Komplexes und ist eine ganze Zahl, welche positiv, ull oder negativ sein kann;
Y steht für ein Gegenion, wobei das Gegenion abhängig von der Ladung des Komplexes ist;
q=z/[Ladung Y]; und/oder
L steht für einen pentadentaten Liganden der allgemeinen Formel(B): 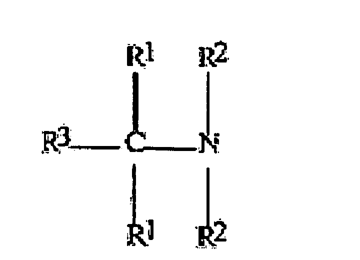
worin:
jedes R¹, R² unabhängig voneinander, stehen für -R4-R5,
R³ steht für Wasserstoff, optional substituiertes Alkyl, Aryl oder Arylalkyl, oder -R⁴-R⁵,
jedes R⁴ stehen unabhängig voneinander für eine Einfachbindung oder optional substituiertes Alkylen, Alkenylen, Oxyalkylen, Aminoalkylen, Alkylen Ether, Carboxylester oder Carboxylamid, und/oder
jedes R⁵ steht unabhängig voneinander für eine optional N-substituierte Aminoalkyl - Gruppe oder eine optional substituierte Heteroaryl - Gruppe ausgewählt aus der Gruppe, umfassend Pyridinyl, Pyrazinyl, Pyrazolyl, Pyrrolyl, Imidazolyl, Benzimidazolyl, Pyrimidinyl, Triazolyl und/oder Thiazolyl.

Der Ligand L hat die allgemeine Formel(B), wie oben angegeben, und ist ein pentadentater Ligand. Der Begriff 'pentadentat' bedeutet im Sinne dieser Erfindung, dass fünf Heteroatome das Metall M - lon in dem Metall-Komplex koordinieren können.
Gemäß der Formel (B), ist ein koordinierendes Heteroatom der Stickstoff, der in der Methylamin - Kette (Peptidkette) enthalten ist, und/oder ein koordinierendes Heteroatom ist vorzugsweise enthalten in jedem der vier R¹ und/oder R² SeitenGruppen. Vorzugsweise sind alle koordinierenden Heteroatome Stickstoffatome.

Der Ligand L der Formel (B) umfaßt vorzugsweise wenigstens zwei substituierte oder unsubstituierte Heteroaryl-Gruppen in den vier Seitengruppen. Die Heteroaryl-Gruppe ist vorzugsweise eine Pyridin-2-yl Gruppe und/oder, wenn substituiert, vorzugsweise eine Methyl- oder Ethyl-substituierte Pyridin-2-yl Gruppe. Noch bevorzugter, die Heteroaryl Gruppe ist eine unsubstituierte Pyridin-2-yl Gruppe. Vorzugsweise ist die Heteroaryl-Gruppe mit Methylamin, vorzugsweise über das N Atom davon, mittels einer Methylen - Gruppe, verbunden. Vorzugsweise enthält der Ligand L der Formel (B) wenigstens eine substituierte Amino-Alkyl Seiten-Gruppe, weiter bevorzugt zwei Aminoethyl SeitenGruppen, noch bevorzugter 2-(N-Alkyl)aminoethyl oder 2-(N,N-dialkyl)aminoethyl.

In Formel (B) steht vorzugsweise R¹ für Pyridin-2-yl oder R² steht für Pyridin-2-yl-methyl. vorzugsweise stehen R² oder R¹ für 2-Aminoethyl, 2-(N-(m)Ethyl)aminoethyl oder 2-(N,N-di(m)Ethyl)aminoethyl. Wenn substituiert, steht R⁵ vorzugsweise für 3-Methylpyridin-2-yl. R³ steht vorzugsweise für Wasserstoff, Benzyl oder Methyl.

Beispiele bevorzugter erfindungsgemäß verwendbarer Liganden L gemäß Formel (B) in deren einfachsten Form sind:
(i) Pyridin-2-yl enthaltende Liganden wie:
   N,N-Bis(pyridin-2-yl-Methyl)-bis(pyridin-2-yl)methylamin;
   N,N-Bis(pyrazol-1-yl-Methyl)-bis(pyridin-2-yl)methylamin;
   N,N-Bis(imidazol-2-yl-Methyl)-bis(pyridin-2-yl)methylamin;
   N,N-Bis(1,2,4-triazol-1-yl-Methyl)-bis(pyridin-2-yl)methylamin;
   N,N-Bis(pyridin-2-yl-Methyl)-bis(pyrazol-1-yl)methylamin;
   N,N-Bis(pyridin-2-yl-Methyl)-bis(imidazol-2-yl)methylamin;
   N,N-Bis(pyridin-2-yl-Methyl)-bis(1,2,4-triazol-1-yl)methylamin;
   N,N-Bis(pyridin-2-yl-Methyl)-1,1-bis(pyridin-2-yl)-1-aminoethan;
   N,N-Bis(pyridin-2-yl-Methyl)-1,1-bis(pyridin-2-yl)-2-phenyl-1-aminoethan;
   N,N-Bis(pyrazol-1-yl-Methyl)-1,1-bis(pyridin-2-yl)-1-aminoethan;
   N,N-Bis(pyrazol-1-yl-Methyl)-1,1-bis(pyridin-2-yl)-2-phenyl-1-aminoethan;
   N,N-Bis(imidazol-2-yl-Methyl)-1,1-bis(pyridin-2-yl)-1-aminoethan;
   N,N-Bis(imidazol-2-yl-Methyl)-1,1 -bis(pyridin-2-yl)-2-phenyl-1 -aminoethan;
   N,N-Bis(1,2,4-triazol-1-yl-Methyl)-1,1-bis(pyridin-2-yl)-1-aminoethan;
   N,N-Bis(1,2,4-triazol-1-yl-Methyl)-1,1-bis(pyridin-2-yl)-2-phenyl-1-aminoethan;
   N,N-Bis(pyridin-2-yl-Methyl)-1,1-bis(pyrazol-1-yl)-1-aminoethan;
   N,N-Bis(pyridin-2-yl-Methyl)-1,1-bis(pyrazol-1-yl)-2-phenyl-1-aminoethan;
   N,N-Bis(pyridin-2-y)-Methyl)-1,1-bis(imidazo)-2-yl)-1-aminoethan;
   N,N-Bis(pyridin-2-y)-Methyl)-1,1 -bis(imidazol-2-yl)-2-phenyl-1-aminoethan;
   N,N-Bis(pyridin-2-yl-Methyl)-1,1-bis(1,2,4-triazol-1-yl)-1-aminoethan;
   N,N-Bis(pyridin-2-yl-Methyl)-1,1-bis(1,2,4-triazol-1-yl)-1-aminoethan;
   N,N-Bis(pyridin-2-yl-Methyl)-1,1-bis(pyridin-2-yl)-1-aminoethan;
   N,N-Bis(pyridin-2-yl-Methyl)-1,1-bis(pyridin-2-yl)-1-aminohexan;
   N,N-Bis(pyridin-2-yl-Methyl)-1,1-bis(pyridin-2-yl)-2-phenyl-1-aminoethan;
   N,N-Bis(pyridin-2-yl-Methyl)-1,1-bis(pyridin-2-yl)-2-(4-sulphonic acid-phenyl)-1-aminoethan;
   N,N-Bis(pyridin-2-yl-Methyl)-1,1-bis(pyridin-2-yl)-2-(pyridin-2-yl)-1-aminoethan;
   N,N-Bis(pyridin-2-yl-Methyl)-1,1-bis(pyridin-2-yl)-2-(pyridin-3-yl)-1-aminoethan;
   N,N-Bis(pyridin-2-yl-Methyl)-1,1-bis(pyridin-2-yl)-2-(pyridin-4-yl)-1-aminoethan;
   N,N-Bis(pyridin-2-yl-Methyl)-1,1-bis(pyridin-2-yl)-2-(1-Alkyl-pyridinium-4-yl)-1-minoethan;
   N,N-Bis(pyridin-2-yl-Methyl)-1,1-bis(pyridin-2-yl)-2-(1-Alkyl-pyridinium-3-yl)-1-aminoethan;
   N,N-Bis(pyridin-2-yl-Methyl)-1,1-bis(pyridin-2-yl)-2-(1-Alkyl-pyridinium-2-yl)-1-aminoethan;
(ii) 2-Aminoethyl enthaltende Liganden wie:
   N,N-Bis(2-(N-Alkyl)amino-Ethyl)-bis(pyridin-2-yl)methylamin;
   N,N-Bis(2-(N-Alkyl)amino-Ethyl)-bis(pyrazol-1-yl)methylamin;
   N,N-Bis(2-(N-Alkyl)amino-Ethyl)-bis(imidazol-2-yl)methylamin;
   N,N-Bis(2-(N-Alkyl)amino-Ethyl)-bis(1,2,4-triazol-1-yl)methylamin;
   N,N-Bis(2-(N,N-dialkyl)amino-Ethyl)-bis(pyridin-2-yl)methylamin;
   N,N-Bis(2-(N,N-dialkyl)amino-Ethyl)-bis(pyrazol-1-yl)methylamin;
   N,N-Bis(2-(N,N-dialkyl)amino-Ethyl)-bis(imidazol-2-yl)methylamin;
   N,N-Bis(2-(N,N-dialkyl)amino-Ethyl)-bis(1,2,4-triazol-1-yl)methylamin;
   N,N-Bis(pyridin-2-yl-Methyl)-bis(2-amino-Ethyl)methylamin;
   N,N-Bis(pyrazol-1-yl-Methyl)-bis(2-amino-Ethyl)methylamin;
   N,N-Bis(imidazol-2-yl-Methyl)-bis(2-amino-Ethyl)methylamin;
   N,N-Bis(1,2,4-triazol-1-yl-Methyl)-bis(2-amino-Ethyl)methylamin.

Besonders bevorzugte Liganden:
N,N-Bis(pyridin-2-yl-Methyl)-bis(pyridin-2-yl)methylamin, nachfolgend bezeichnet als N4Py;
N,N-Bis(pyridin-2-yl-Methyl)-1,1-bis(pyridin-2-yl)-1-aminoethan, nachfolgend bezeichnet als MeN4Py;
N,N-Bis(pyridin-2-yl-methyl)-1,1-bis(pyridin-2-yl)-2-phenyl-1-aminoethane, nachfolgend bezeichnet als BzN4Py.

Ein weiterer erfindungsgemäß verwendbarer Ligandenkomplex basiert auf der allgemeinen Komplex-Formel (A) und Ligand (B) wie oben angegeben, aber mit der Ausnahme das R³ für Wasserstoff steht.

Alternativ bildet die organische Substanz einen Komplex der allgemeine Formel (A), wie oben angegeben, aus, aber mit der Ausnahme das L für einen pentadentaten oder hexadentaten Ligand der allgemeinen Formel (C) steht:

R¹R¹N-W-N R¹R²

worin:
jedes R¹ unabhängig voneinander steht für -R³ -V, worin R³ steht für optional substituiertes Alkylen, Alkenylen, Oxyalkylen, Aminoalkylen oder Alkylen Ether, und/oder V steht für eine optional substituierte Heteroaryl Gruppe ausgewählt aus der Gruppe, umfassend Pyridinyl, Pyrazinyl, Pyrazolyl, Pyrrolyl, Imidazolyl, Benzimidazolyl, Pyrimidinyl, Triazolyl und/oder Thiazolyl;
W steht für eine optional substituierte Alkylen-Brücken-Gruppe ausgewählt aus der Gruppe, umfassend -CH2CH2-, -CH2CH2CH2-, -CH2CH2CH2CH2-, -CH2-C6H4-CH2-, -CH2- C6H10-CH2-, und/oder-CH2-C10H6-CH2-; und/oder
R² steht für ein Gruppe ausgewählt aus der Gruppe, umfassend R¹, und/oder Alkyl, Aryl und/oder Arylalkyl - Gruppen, optional substituiert mit einem Substituenten ausgewählt aus der Gruppe, umfassend Hydroxy, Alkoxy, Phenoxy, Carboxylat, Carboxamid, Carboxylester, Sulphonat, Amin, Alkylamin und/oder N⁺(R⁴)₃, worin R⁴ ist ausgewählt aus der Gruppe, umfassend Wasserstoff, Alkanyl, Alkenyl, Arylalkanyl, Arylalkenyl, Oxyalkanyl, Oxyalkenyl, Aminoalkanyl, Aminoalkenyl, Alkanylether und/oder Alkenylether.

Der Ligand L hat die allgemeine Formel(C), wie oben angegeben, und ist ein pentadentater Ligand oder, wenn R¹= R², kann dieser ein hexadentater Ligand sein. Der Begriff 'pentadentat' bedeutet im Sinne dieser Erfindung, wie oben angegeben, das fünf Heteroatome das Metall M - Ion in dem Metall-Komplex koordinieren können. Der Begriff 'hexadentat' bedeutet im Sinne dieser Erfindung, das im Prinzip sechs Heteroatome das Metall M - lon in dem Metall-Komplex koordinieren können. Bei hexadentaten Liganden koordinieren in der Regel aber meistens nur maximal fünf der sechs Liganden.

In der Formel (C), sind zwei Heteroatome durch eine Brücken-Gruppe W verbunden und/oder wenigstens ein koordinierendes Heteroatom ist in jeder der drei R¹ Gruppen enthalten. Vorzugsweise ist das koordinierende Heteroatom ein Stickstoffatom.

Der Ligand L der Formel (C) umfaßt wenigstens eine optional substituierte Heteroaryl -Gruppe in jeder der drei R¹ Gruppen. Vorzugsweise ist die Heteroaryl-Gruppe eine Pyridin-2-yl-Gruppe, insbesondere eine Methyl- oder Ethyl-substituierte Pyridin-2-yl-Gruppe. Die Heteroaryl-Gruppe ist über ein N Atom in Formel (C) verbunden, vorzugsweise über eine Alkylen - Gruppe und weiter bevorzugt über eine Methylen-Gruppe. Am meisten bevorzugt ist die Heteroaryl - Gruppe eine 3-Methyl-pyridin-2-yl - Gruppe, welche zu einem N Atom über Methylen verbunden ist.

Die Gruppe R² in Formel (C) ist eine substituierte oder unsubstituierte Alkyl, Aryl oder Arylalkyl Gruppe, oder eine Gruppe R¹. Vorzugsweise ist R² unterschiedlich zu jeder der R¹ Gruppen in der oben angegebenen Formel. Vorzugsweise ist R² Methyl, Ethyl, Benzyl, 2-Hydroxyethyl oder 2-Methoxyethyl. Noch bevorzugter, R² ist Methyl oder Ethyl.
Die Brückengruppe W kann eine substituierte oder unsubstituierte Alkylen Gruppe ausgewählt aus der Gruppe, umfassend -CH2CH2-, -CH2CH2CH2-, -CH2CH-2CH2CH2-, -CH2-C6H4-CH2-, -CH2-C6H10-CH2-, und/oder -CH2-C10H6-CH2-, sein (worin -C6H4-, -C6H10-, -C10H6- kann ortho-, para-, oder meta-C6H4-, - C6H10-, -C10H6- sein). Vorzugsweise ist die Brückengruppe W eine Ethylen oder 1,4-Butylen Gruppe, noch bevorzugter eine Ethylen-Gruppe.

Vorzugsweise steht V für substituiertes Pyridin-2-yl, insbesondere für Methylsubstituiertes oder Ethyl-substituiertes Pyridin-2-yl, und/oder noch bevorzugter steht V für 3-Methylpyridin-2-yl.

Beispiele bevorzugter Liganden der Formel (C) in ihrer einfachsten Form sind:
N-Methyl-N,N',N'-tris(3-Methyl-pyridin-2-ylmethyl)ethylen-1,2-diamin;
N-Ethyl-N,N',N'-tris(3-Methyl-pyridin-2-ylmethyl)ethylen-1,2-diamin;
N-Benzyl-N,N', N'-tris(3-Methyl-pyridin-2-ylmethyl)ethylen-1,2-diamin;
N-(2-hydroxyethyl)-N,N',N'-tris(3-Methyl-pyridin-2-ylmethyl)ethylen-1,2-diamin;
N-(2-methoxyethyl)-N,N',N'-tris(3-Methyl-pyridin-2-ylmethyl)ethylen-1,2-diamin;
N-Methyl-N,N',N'-tris(5-Methyl-pyridin-2-ylmethyl)ethylen-1,2-diamin;
N-Ethyl-N, N',N'-tris(5-Methyl-pyridin-2-ylmethyl)ethylen-1,2-diamin;
N-benzyl-N,N',N'-tris(5-Methyl-pyridin-2-ylmethyl)ethylen-1,2-diamin;
N-(2-hydroxyethyl)-N,N',N'-tris(5-Methyl-pyridin-2-ylmethyl)ethylen-1,2-diamin;
N-(2-methoxyethyl)-N,N',N'-tris(5-Methyl-pyridin-2-ylmethyl)ethylen-1,2-diamin;
N-Methyl-N,N',N'-tris(3-Ethyl-pyridin-2-ylmethyl)ethylen-1,2-diamin;
N-Ethyl-N,N',N'-tris(3-Ethyl-pyridin-2-ylmethyl)ethylen-1,2-diamin;
N-benzyl-N,N',N'-tris(3-Ethyl-pyridin-2-ylmethyl)ethylen-1,2-diamin;
N-(2-hydroxyethyl)-N,N',N'-tris(3-Ethyl-pyridin-2-ylmethyl)ethylen-1,2-diamin;
N-(2-methoxyethyl)-N,N',N'-tris(3-Ethyl-pyridin-2-ylmethyl)ethylen-1,2-diamin;
N-Methyl-N,N',N'-tris(5-Ethyl-pyridin-2-ylmethyl)ethylen-1,2-diamin;
N-Ethyl-N,N',N'-tris(5-Ethyl-pyridin-2-ylmethyl)ethylen-1,2-diamin;
N-Benzyl-N,N',N'-tris(5-Ethyl-pyridin-2-ylmethyl)ethylen-1,2-diamin; und/oder
N-(2-methoxyethyl)-N,N',N'-tris(5-Ethyl-pyridin-2-ylmethyl)ethylen-1,2-diamin.

Besonders bevorzugte erfindungsgemäß verwendbare Liganden, die kovalent an einen Träger gebunden werden können, sind:
N-Methyl-N,N',N'-tris(3-Methyl-pyridin-2-ylmethyl)ethylen-1,2-diamin;
N-Ethyl-N,N',N'-tris(3-Methyl-pyridin-2-ylmethyl)ethylen-1,2-diamin;
N-benzyl-N,N',N'-tris(3-Methyl-pyridin-2-ylmethyl)ethylen-1,2-diamin;
N-(2-Hydroxyethyl)-N,N',N'-tris(3-Methyl-pyridin-2-ylmethyl)ethylen-1,2-diamin; und/oder
N-(2-methoxyethyl)-N,N',N'-tris(3-Methyl-pyridin-2-ylmethyl)ethylen-1,2-diamin.

Am meisten bevorzugte erfindungsgemäß verwendbare Liganden, die kovalent an einen Träger gebunden werden können, sind:
N-Methyl-N,N',N'-tris(3-Methyl-pyridin-2-ylmethyl)ethylen-1,2-diamin; und/oder
N-Ethyl-N, N',N'-tris(3-Methyl-pyridin-2-ylmethyl)ethylen-1,2-diamin.

Vorzugsweise ist das Metall M in Formel (A) Fe oder Mn, noch bevorzugter Fe. Bevorzugt koordinierende Atome/Moleküle X in Formel (A) können ausgewählt sein aus der Gruppe, umfassend R⁶OH, NR⁶₃, R⁶CN, R⁶OO-, R⁶S-, R⁶O-, R⁶COO-, OCN⁻, SCN⁻, N₃⁻, CN⁻, F⁻, Cl⁻, Br⁻, l⁻, O₂⁻, NO₃⁻, NO₂⁻, SO₄²⁻, SO₃²⁻, PO₄³⁻ und/oder aromatische N Donor ausgewählt aus der Gruppe, umfassend Pyridine, Pyrazine, Pyrazole, Pyrrole, Imidazole, Benzimidazole, Pyrimidine, Triazole und/oder Thiazole, worin R⁶ vorzugsweise ausgewählt ist aus der Gruppe, umfassend Wasserstoff, optional substituiertes Alkyl und/oder optional substituiertes Aryl. X kann außerdem LMO- oder LMOO- sein, worin M ein Übergangsmetall und L ein Ligand ist, wie oben angegeben. Das koordinierungsfähige X ist vorzugsweise ausgewählt aus der Gruppe, umfassend CH₃CN, H₂O, F⁻, Cl⁻, Br⁻, OOH⁶, R⁶COO⁻, R⁶O⁻, LMO⁻, und/oder LMOO⁻ worin R⁶ für Wasserstoff oder optional substituiertes Phenyl, Naphthyl, oder C1-C4 Alkyl, steht.
Das Gegenion Y in Formel (A) gleicht die Ladung z des durch den Liganden L gebildeten Komplex, des Metall M und/oder des koordinierenden X, aus. Wenn die Ladung z positiv ist, kann Y ein Anion, beispielsweise R⁷COO⁻, BPh₄⁻, ClO₄⁻, BF₄⁻, PF₆⁻, R⁷SO₃⁻, R⁷SO₄⁻, SO₄²⁻, NO₃⁻, F⁻, Cl⁻, Br⁻, oder I⁻, mit R⁷ = Wasserstoff, optional substituiertes Alkyl oder optional substituiertes Aryl. Wenn z ist negativ, ist Y ein übliches Kation, beispielsweise ein Alkalimetall, Erdalkalimetall oder ein (Alkyl)ammoniumkation.
Geeignete Gegenionen Y sind auch solche die die Bildung lagerstabiler Feststoffe bewirken. Bevorzugte Gegenionen bevorzugter Metallkomplexe sind ausgewählt aus der Gruppe, umfassend R⁷COO⁻, ClO₄⁻, BF₄⁻, PF₆⁻, R⁷SO₃⁻ (insbesondere CF₃SO₃⁻), R⁷SO₄⁻, SO₄²⁻, NO₃⁻, F⁻, Cl⁻, Br⁻, und/oder I⁻, worin R⁷steht für Wasserstoff oder optional substituiertes Phenyl, Naphthyl oder C1-C4 Alkyl. Ein weiterer geeigneter erfindungsgemäß verwendbarer, an einen Träger über wenigstens einen Liganden kovalent bindbarer Ligandenkomplex, weist die nachstehende allgemeine Formel(D) auf:

[{M' ₐL}_{b}X_{c}]^{z}Y_{q}

worin:
M' steht für Wasserstoff oder ein Metall ausgewählt aus der Gruppe, umfassend Ti, V, Co, Zn, Mg, Ca, Sr, Ba, Na, K, und/oder Li;
X steht für eine koordinierendes Atom/Molekül;
a steht für eine ganze Zahl im Bereich von zwischen 1 bis 5;
b steht für eine ganze Zahl im Bereich von zwischen 1 bis 4;
c steht für Null oder eine ganze Zahl im Bereich von zwischen 0 bis 5;
z steht für die Ladung der Verbindung und ist eine ganze Zahl , welche positiv, Null oder negativ sein kann;
Y steht für ein Gegenion, das abhängig von der Ladung der Verbindung ausgewählt ist;
q=z/[Ladung Y]; und/oder
L steht für ein pentadentaten Liganden der allgemeinen Formel (B) oder (C) wie oben angegeben.

Eine geeignete organische Substanz umfaßt einen macrocyclischen Ligand der Formel (E): 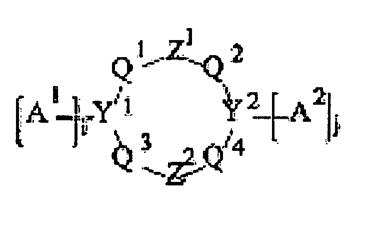 worin:
Z¹ und/oder Z² unabhängig voneinander ausgewählt sind aus der Gruppe, umfassend monocyclische oder polycyclische aromatische Ringstrukturen, die optional wenigstens ein Heteroatom aufweisen, wobei jede aromatische Ringstruktur mit wenigstens einem Substituenten substituiert sein kann; Y¹ und/oder Y² sind unabhängig voneinander ausgewählt aus der Gruppe, umfassend C, N, O, Si, P und/oder S Atome;
A¹ und/oder A² sind unabhängig voneinander ausgewählt aus der Gruppe, umfassend Wasserstoff, Alkyl, Alkenyl und/oder Cycloalkyl, wobei jedes Alkyl, Alkenyl und/oder Cycloalkyl kann optional substituiert sein durch wenigstens eine Gruppe ausgewählt aus der Gruppe, umfassend Hydroxy, Aryl, Heteroaryl, Sulphonate, Phosphate, Elektronen-Donor-Gruppen und/oder elektronenziehende Gruppen, und/oder Gruppen der Formel (G¹)(G²N-, G³OC(O)-, G³O- und/oder G³C(O)-, worin jedes G¹, G² und/oder G³ unabhängig voneinander ausgewählt ist aus der Gruppe, umfassend Wasserstoff und/oder Alkyl, und/oder Elektron-Donor-Gruppe und/oder elektronenziehende Gruppen (zusätzlich zu den vorgenannten);
i und/oder j sind ausgewählt aus der Gruppe, umfassend 0, 1 und/oder 2 entsprechend der Valenz Y¹ und/oder Y²;
jedes Q¹-Q⁴ ist unabhängig voneinander ausgewählt aus der Gruppe gemäß der nachstehenden Formel: 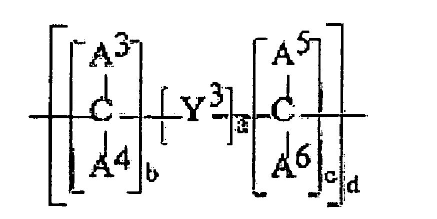
worin:
10>a+b+c>2 und/oder d≥1;
jedes Y³ ist unabhängig voneinander ausgewählt aus der Gruppe, umfassend -O-, -S-, -SO-, -SO₂-, -(G¹)N- (worin G¹ wie oben angegeben ist), -C(O)-, Arylen, Heteroarylen, -P- und/oder -P(O)-;
jedes A³-A⁶ ist unabhängig voneinander ausgewählt aus der Gruppe, umfassend die oben angegeben Gruppen A¹ und/oder A²; und
   worin wenigstens zwei der A¹-A⁶ eine Brücken-Gruppe ausbilden, vorausgesetzt das A¹ und/oder A² gebunden sind, ohne gleichzeitig an A³-A⁶ gebunden zu sein; die A¹ und/oder A² verbindende Brückengruppe kann wenigstens eine CarbonylGruppe aufweisen.
Der Ligand der Formel (E), wenn nicht anders angegeben, umfaßt alle Alkyl, Hydroxyalkyl Alkoxy, und/oder Alkenyl Gruppen, vorzugsweise mit 1 bis 6, weiter bevorzugt mit 1 bis 4 Kohlenstoffatomen.

Bevorzugte Elektronen-Donor-Gruppen umfassen Alkyl (z.B. Methyl), Alkoxy (z.B. Methoxy), Phenoxy, und/oder unsubstituierte, monosubstituierte und/oder disubstituierte Amin - Gruppen. Bevorzugte Elektronen ziehende Gruppen umfassen Nitro, Carboxy, Sulphonyl und/oder Halogen.

Der Ligand der Formel (E) kann als Ligandenkomplex mit Übergangsmetallatom oder ohne Übergangsmetallatom vorliegen. In der nicht Übergangsmetall komplexierten Form kann das Übergangsmetall aus der gebrauchsfertigen Zusammensetzung, d.h. dem Mittel, oder beispielsweise aus einem zusammen mit dem Mittel am Einsatzort verwendeten fluiden Medium, wie Wasser, stammen. Leitungswasser weisen üblicherweise ausreichende Spuren an Übergangsmetallen auf.

Ein weiterer erfindungsgemäß verwendbarer Ligand, der kovalent an einen Träger bindbar ist, weist die nachstehende Formel (E) mit einem Gegenion der Formel (F) auf:

[HₓL]^{z}Y_{q}

worin:
H ist ein Wasserstoffatom;
Y ist ein Gegenanion, wobei das Gegenion abhängig von der Ladung des Komplexes ist;
x ist eine ganze Zahl, so dass wenigstens ein Stickstoff Atom von L protoniert ist;
z steht für die Ladung des Komplexes und/oder ist eine ganze Zahl, die positiv oder Null ist;
q=z/[Ladung von Y]; und/oder
L ist ein Ligand der Formel (E) wie oben angegeben.

In einer weiter erfindungsgemäß verwendbaren Ausführungsform bildet die organische Substanz einen Metalkomplex der Formel (G), basierend auf der lonenpaarbildung der Formel (F):

[MₓL]^{z}Y_{q}

worin:
L, Y, x, z und/oder q wie in Formel (F) oben definiert sind und/oder M ist ein Metall ausgewählt aus der Gruppe, umfassend Mn in Oxidationsstufen II-V, Fe II-V, Cu I-III, Co I-III, Ni I-III, Cr II-VI, W IV-VI, Pd V, Ru II-IV, V III-IV und/oder Mo IV-VI. Bevorzugt ist ein Komplex der Formel (G) worin M für Mn, Co, Fe oder Cu steht. Weiterhin ist eine organische Substanz bevorzugt die einen Komplex der Formel 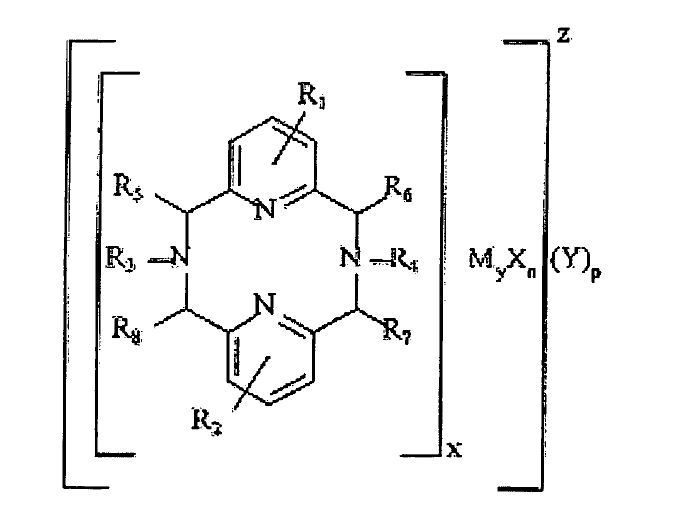 (H) ausbildet:
worin:
M steht für ein Fe Atom mit der Oxidationsstufe II oder III, Mn Atom mit der Oxidationsstufe II, III, IV oder V, Cu Atom mit der Oxidationsstufe I, II oder III, oder Co Atom mit der Oxidationsstufe II, III oder IV;
X ist eine verbrückende Gruppe oder eine nicht verbrückende Gruppe zwischen M, vorzugsweise Fe Atom(en), Y ist ein Gegenion, x und/oder y sind ≥1, 0=<n=<3 und z ist die Ladung des Metallkomplexes, und p="z/Ladung" von Y; R1 und/oder R2 stehen unabhängig voneinander für wenigstens einen Ringsubstituenten, ausgewählt aus der Gruppe, umfassend Wasserstoff und/oder Elektronen-Donor und/oder Elektronen ziehende Gruppen, R3 bis R8 stehen unabhängig voneinander für Wasserstoff, Alkyl, Hydroxyalkyl, Alkenyl oder Derivate davon, wenn diese durch wenigstens eine Elektronen-Donor und/oder Elektronen ziehende Gruppen substituiert sind.

In dem Komplex der Formel (H) steht M vorzugsweise für ein Fe Atom in der Oxidationsstufe II oder III oder ein Mn Atom in der Oxidationsstufe II, III, IV, oder V. Vorzugsweise ist die Oxidationsstufe von M = III.
Wenn M = Fe ist, liegt der Komplex der Formel (H) vorzugsweise in der Form eines Fe-Salzes vor, wie (im oxidiertem Zustand) Dihalo-2,11-diazo[3.3](2,6)pyridinophan, Dihalo-4-methoxy-2,11-diazo[ 3.3](2,6)pyridinophan und/oder Mischungen davon, insbesondere in der Form eines Chloridsalzes.

Wenn M = Mn ist, liegt der Komplex der Formel (H) vorzugsweise in der Form eines Mn-Salzes vor (im oxidiertem Zusatand), wie N,N'-Dimethyl-2,11-diazo[3.3](2,6)pyridinophan, insbesondere in der Form eines Monohexafluorophosphat-Salz.

Vorzugsweise ist X ausgewählt aus der Gruppe, umfassend H₂O, OH⁻, O₂⁻, SH⁻, S²⁻, SO₄²⁻, NR₉R₁₀⁻, RCOO⁻, NR₉R₁₀R₁₁, Cl⁻, Br⁻, F⁻, N₃⁻ und/oder Kombinationen davon, worin R₉, R₁₀ und/oder R₁₁ unabhängig voneinander ausgewählt sind aus der Gruppe, umfassend -H, C1-4 Alkyl und/oder Aryl, optional mit wenigstens einer Electronen ziehenden und/oder Elektronen-Donor Gruppe. Noch bevorzugter ist X ein Halogen, insbesondere ein Fluoridion.

In den Formeln (F), (G) und/oder (H) ist das anionische Gegenion Y vorzugsweise ausgewählt aus der Gruppe, umfassend Cl⁻, Br⁻, I⁻, NO₃⁻, ClO₄⁻, SCN⁻, PF₆⁻, RSO₃⁻, RSO₄⁻, CF₃SO₃⁻, BPh₄⁻, und/oder OAc⁻. Ein kationisches Gegenion ist vorzugsweise nicht vorhanden.

In Formel (H), R1 und/oder R2 sind vorzugsweise beide Wasserstoff; R3 und/oder R4 sind vorzugsweise C1-4 Alkyl, insbesondere Methyl; R5-R8 sind vorzugsweise jeweils Wasserstoff.
Der Fe oder Mn haltige Katalysator der Formel (H) kann als ein Monomer, Dimer oder Oligomer vorliegen. Ein weiterer erfindungsgemäß verwendbarer Ligand kann den Komplex der allgemeinen Formel (A1) ausbilden, worin L für einen Liganden der allgemeinen Formel steht, oder ein protoniertes oder deprotoniertes Analogon davon ist: 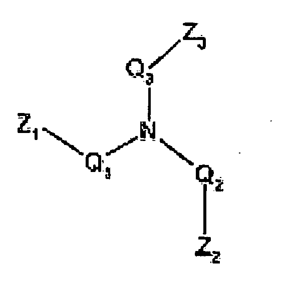 worin**:**
Z1, Z2 und/oder Z3 unabhängig voneinander für eine Koordinationsgruppe stehen, ausgewählt aus der Gruppe, umfassend Carboxylat, Amido, -NH-C(NH)NH2, Hydroxyphenyl, optional substituierten heterocyclischen Ring oder optional substituierten heteroaromatischen Ring, ausgewählt aus der Gruppe, umfassend Pyridin, Pyrimidin, Pyrazin, Pyrazol, Imidazol, Benzimidazol, Quinolin, Quinoxalin, Triazol, Isoquinolin, Carbazol, Indol, Isoindol, Oxazol und/oder Thiazol; Q1, Q2 und/oder Q3 stehen unabhängig voneinander für eine Gruppe mit der Formel: 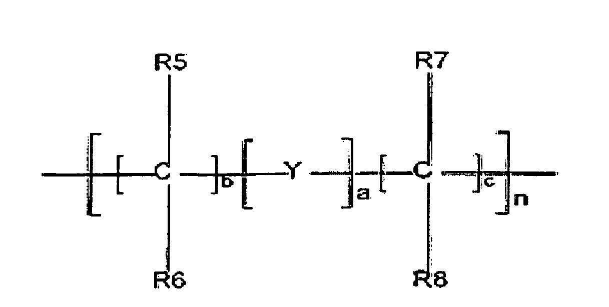
worin
5≥a+b+c≥1; a=0-5; b=0-5; c=0-5; n=1 oder 2 ist. Vorzugsweise ist Q1, Q2 und/oder Q3 so definiert, dass a=b=0, c=1 oder 2, und/oder n=1 ist. Vorzugsweise stehen Q1, Q2 und/oder Q3 unabhängig voneinander für eine Gruppe, ausgewählt aus der Gruppe umfassend, -CH2- und/oder -CH2CH2-. Y steht unabhängig voneinander für ein Gruppe, ausgewählt aus der Gruppe, umfassend -O-, -S-, - SO-, -S02-, -C(O)-, Arylen, Alkylen, Heteroarylen, Heterocycloalkylen, -(G)P-, - P(O)- und/oder -(G)N-, worin G ausgewählt ist aus der Gruppe, umfassend Wasserstoff, Alkyl, Aryl, Arylalkyl, Cycloalkyl, jede, ausgenommen Wasserstoff, kann durch wenigstens eine Gruppen E funktionalisiert sein;
R5, R6, R7, R8 stehen unabhängig voneinander für eine Gruppe ausgewählt aus der Gruppe, umfassend Wasserstoff, Hydroxyl, Halogen, -R und/oder -OR, worin R steht für Alkyl, Alkenyl, Cycloalkyl, Heterocycloalkyl, Aryl, Heteroaryl oder eine Carbonyl-Derivat Gruppe, R kann durch wenigstens eine Gruppen E funktionalisiert sein, oder R5 zusammen mit R6, oder R7 zusammen mit R8, oder beide, steht für Sauerstoff, oder R5 zusammen mit R7 und/oder unabhängig R6 zusammen mit R8, oder R5 zusammen mit R8 und/oder unabhängig R6 zusammen mit R7, steht für C1-6-Alkylen, gegebenenfalls substituiert durch C1-4-Alkyl, -F, -Cl, -Br oder -I; und/oder E steht unabhängig voneinander für eine functionale Gruppe ausgewählt aus der Gruppe, umfassend -F, -Cl, -Br, -I, -OH, - OR', NH₂, -NHR', -N(R')₂, -N(R')₃⁺, -C(O)R', -OC(O)R', -COOH, -COO- (Na⁺, K⁺), - COOR', -C(O)NH₂, -C(O)NHR', -C(O)N(R')₂, Heteroaryl, -R', -SR', -SH, -P(R')₂, - P(O)(R')₂-P(O)(OH)₂, -P(O)(OR')₂, -NO₂, -SO₃H, -SO₃- (Na⁺, K⁺) -S(O)₂R', - NHC(O)R', und/oder -N(R')C(O)R', worin R' steht für Cycloalkyl, Aryl, Arylalkyl, oder Alkyl optional substituiert durch -F, -Cl, -Br, -I, -NH₃⁺, -SO₃H, -SO₃- (Na⁺,K⁺), -COOH, -COO-(Na⁺,K⁺), -P(O)(OH)₂, oder -P(O) (O-(Na⁺,K⁺))₂.

Bevorzugt sind Liganden, wie oben angegeben, mit Z1, Z2 und/oder Z, die unabhängig voneinander stehen für eine koordinierende Gruppe, ausgewählt aus der Gruppe, umfassend optional substituiertes Pyridin-2-yl, optional substituiertes Imidazol-2-yl, optional substituiertes Imidazol-4-yl, optional substituiertes Pyrazol-1-yl, und/oder optional substituiertes Quinolin-2-yl. Noch bevorzugter sind Liganden, wie oben angegeben, mit Z1, Z1 und/oder Z3 enthaltend optional substituiertes Pyridin-2-yl Gruppen. Am meisten bevorzugt sind die nachfolgenden Liganden L umfassend Tris(Pyridin-2-ylmethyl)amin, Tris(3-Methyl-pyridin-2-ylmethyl)amin, Tris(5-Methyl-pyridin-2-ylmethyl)amin, und/oder Tris(6-Methyl-pyridin-2-ylmethyl)amin.

Der entsprechend geeignete Komplex ist nachfolgend angegeben:

[MₐLₖXₙ]Yₘ

vorzugsweise:
M steht für ein Metal ausgewählt aus der Gruppe, umfassend Mn(II)-(III)(IV)-(V), Cu(I)-(II)-(III), Fe(II)-(III)-(IV)-(V), Co(I)-(II)-(III), Ti(II)-(III)-(IV), V(II)-(III)-(IV)-(V), Mo(II)-(III)-(IV)-(V)-(VI) und/oder W(IV)-(V)-(VI);
X steht für ein koordinierendes Atom/Molekül ausgewählt aus der Gruppe, umfassend jedes mono, bi oder tri - Ladungandenanion und/oder jedes neutrale Molekül, dass zur mono, bi oder tridentaten Koordination von Metall geeignet ist;
Y steht für jedes nicht-koordinierendes Gegenion;
a steht für eine ganze Zahl von zwischen 1 bis 10;
k steht für eine ganze Zahl von zwischen 1 bis 10;
n steht für eine ganze Zahl von zwischen 1 bis 10;
m steht für Null oder eine ganze Zahl von zwischen 1 bis 20.

Am meisten bevorzugt ist der erfindungsgemäß verwendbare über wenigstens einen Liganden an einen Träger kovalent gebundene Bleichmittelkomplex, ausgewählt aus der Gruppe, umfassend Dimangan-tris-µ-oxo-Bis(1,4,7-trimethyl-1,4,7-triazacyclononan) bis(hexafluorophosphat), Dimangan-Bis-µ-oxo-µ-acetato-1,2-bis(4,7-dimethyl-1,4,7-triaza-1-cyclononyl)ethan-bis(hexafluorophosphate), Eisen-N,N'-bis(pyridin-2-ylmethylen)-1,1,-bis(pyridin-2-yl)aminoethan-bis-chlorid, Cobalt-pentamine-µ-acetatdichlorid, Eisen-(N-methyl-N,N',N'-tris(3-methyl-pyridin-2ylmethyl)-ethylendiamin)chloride-hexafluorphosphat und/oder Mischungen davon.

Geeignet verwendbare über wenigstens einen Liganden an einen Träger kovalent verbindbare Bleichmittelkomplexe mit makrocyclischer Struktur sind außerdem beschrieben in EP-A-408 131, EP-A-384503, EP-A-458 398, U.S. Pat. No. 5,194,416, WO 96/06157 und/oder WO 98/39405 Weitere geeignet verwendbare über wenigstens einen Liganden an einen Träger kovalent verbindbare Bleichmittelkomplexe mit linearer Struktur sind beschrieben in EP-A-392592, WO97/48710, U.S. Pat. No. 5,580,485 und/oder EP-909 809. U.S. Pat. No. 5,705.

Weitere Gegenstände der Erfindung ist die Verwendung von obengenannter Übergangsmetall-Bleichkatalysatoren mit einem vorbeschriebenen Liganden enthalten in Mitteln insbesondere Wasch-Reinigungs- und Desinfektionsmittel und ein Verfahren zur Aktivierung von Persauerstoffverbindungen unter Einsatz eines derartigen über einen solchen Liganden trägerfixierten Bleichkatalysators. Die Mittel können dabei auch portioniert, d.h. schon für die übliche Anwendungsmenge separat vorportioniert, vorliegen.

Bei dem erfindungsgemäßen Verfahren und im Rahmen einer erfindungsgemäßen Verwendung kann der trägerfixierte Bleichkatalysator im Sinne eines Katalysators überall dort eingesetzt werden, wo es auf eine materialschonende Bleichwirkung ankommt, beispielsweise bei der Bleiche von Textilien oder Haaren, bei der Oxidation organischer oder anorganischer Zwischenprodukte und bei der Desinfektion.

Die erfindungsgemäße Verwendung besteht im wesentlichen darin, Bedingungen zu schaffen, unter denen die Persauerstoffverbindung und der trägerfixierte Bleichkatalysator miteinander reagieren können, mit dem Ziel, stärker oxidierend wirkende Folgeprodukte zu erhalten. Solche Bedingungen liegen insbesondere dann vor, wenn beide Reaktionspartner in wäßriger Lösung aufeinandertreffen. Dies kann durch separate Zugabe der Persauerstoffverbindung und des trägerfixierten Bleichkatalysators zu einer gegebenenfalls wasch- oder reinigungsmittelhaltigen Lösung geschehen. Besonders vorteilhaft wird das erfindungsgemäße Verfahren jedoch unter Verwendung eines erfindungsgemäßen Wasch-, Reinigungs- oder Desinfektionsmittels, das den trägerfixierten Bleichkatalysator und gegebenenfalls ein peroxidisches Oxidationsmittel enthält, durchgeführt. Die Persauerstoffverbindung kann auch separat, in Substanz oder als vorzugsweise wäßrige Lösung oder Suspension, zur Lösung zugegeben werden, wenn ein persauerstofffreies Mittel verwendet wird.

Je nach Verwendungszweck können die Bedingungen weit variiert werden. So kommen neben rein wäßrigen Lösungen auch Mischungen aus Wasser und geeigneten organischen Lösungsmitteln als Reaktionsmedium in Frage. Die Einsatzmengen an Persauerstoffverbindungen werden im allgemeinen so gewählt, daß in den Lösungen zwischen 10 ppm und 10 % Aktivsauerstoff, vorzugsweise zwischen 50 und 5000 ppm Aktivsauerstoff vorhanden sind. Auch die verwendete Menge an trägerfixiertem Bleichkatalysator, bezogen auf den Bleichkatalysator ohne Träger, hängt vom Anwendungszweck ab. Je nach gewünschtem Aktivierungsgrad werden 0,00001 Mol bis 0,025 Mol, vorzugsweise 0,0001 Mol bis 0,002 Mol Katalysator pro Mol Persauerstoffverbindung verwendet, doch können in besonderen Fällen diese Grenzen auch über- oder unterschritten werden.

Ein Wasch-, Reinigungs- oder Desinfektionsmittel enthält, wobei die Gew.-% Angaben auf den Bleichkatalysator ohne Träger bezogen sind, vorzugsweise 0,0025 Gew.-% bis 0,25 Gew.-%, insbesondere 0,01 Gew.-% bis 0,1 Gew.-% des Bleichkatalysators. Vorzugsweise weisen derartige Wasch-, Reinigungs- oder Desinfektionsmittel Bleichkatalysator(en) mit Ligand(en) gemäß Formel I oder Formel II neben üblichen, mit dem Bleichkatalysator verträglichen Inhaltsstoffen auf.

Der trägerfixierte Bleichkatalysator kann in im Prinzip bekannter Weise an Trägerstoffe adsorbiert und/oder in Hüllsubstanzen eingebettet sein.

Die Wasch-, Reinigungs- und Desinfektionsmittel, die als insbesondere pulverförmige Feststoffe, in nachverdichteter Teilchenform, als homogene Lösungen oder Suspensionen vorliegen können, können außer dem erfindungsgemäß verwendeten trägerfixiertem Bleichkatalysator im Prinzip alle bekannten und in derartigen Mitteln üblichen Inhaltsstoffe enthalten. Die erfindungsgemäßen Mittel können anionische Tenside, kationische Tenside, amphotere Tenside, Buildersubstanzen, Bleichmittel, Bleichaktivatoren, Bleichstabilisatoren, weitere Bleichkatalysatoren, Enzyme, Polymere, Cobuilder, Alkalisierungsmittel, Acidifizierungsmittel, Antiredepositionsmittel, Silberschutzmittel, Färbemittel, optische Aufheller, UV-Schutzsubstanzen, Weichspüler, Duftstoffe, schmutzabweisende Stoffe, Anti-Knitter-Stoffe, antibakterielle Stoffe, Farbschutzstoffe, Verfärbungsinhibitoren, Vitamine, Schichtsilikate, geruchskomplexierende Substanzen, Klarspüler, Schauminhibitoren, Schäumungsmittel, Konservierungsmittel und/oder Hilfsmittel aufweisen.

Bevorzugt weisen die Mittel insbesondere Buildersubstanzen, oberflächenaktive Tenside, organische und/oder anorganische Persauerstoffverbindungen, wassermischbare organische Lösungsmittel, Enzyme, Sequestrierungsmittel, Elektrolyte, pH-Regulatoren und weitere Hilfsstoffe, wie optische Aufheller, Vergrauungsinhibitoren, Farbübertragungsinhibitoren, Schaumregulatoren, zusätzliche Persauerstoff-Aktivatoren, Farb- und Duftstoffe auf.

Ein Desinfektionsmittel kann zur Verstärkung der Desinfektionswirkung gegenüber speziellen Keimen zusätzlich zu den bisher genannten Inhaltsstoffen übliche antimikrobielle Wirkstoffe enthalten. Derartige antimikrobielle Zusatzstoffe sind in den erfindungsgemäßen Desinfektionsmitteln vorzugsweise nicht über 10 Gew.-%, besonders bevorzugt von 0,1 Gew.-% bis 5 Gew.-%, enthalten.

Zusätzlich zu den Übergangsmetall-Bleichkatalysatoren mit den vorbeschriebenen Liganden, insbesondere in Kombination mit anorganischen Persauerstoffverbindungen, können in den Mitteln konventionelle Bleichaktivatoren, das heißt Verbindungen, die unter Perhydrolysebedingungen gegebenenfalls substituierte Perbenzoesäure und/oder aliphatische Peroxocarbonsäuren mit 1 bis 10 C-Atomen, insbesondere 2 bis 4 C-Atomen ergeben, eingesetzt werden.

In den Mittel(n) verwendbare Tensid(e), umfassen anionische, nichtionische, kationische und/oder amphotere Tenside. Bevorzugt sind aus anwendungstechnischer Sicht bei Textilwaschmitteln Mischungen aus anionischen und nichtionischen Tensiden, wobei der Anteil der anionischen Tenside größer sein sollte als der Anteil an nichtionischen Tensiden. Der Gesamttensidgehalt des Mittels, beispielsweise bei Wasch-, Pflege- oder Reinigungsmittelzusammensetzung, liegt vorzugsweise unterhalb von 30 Gew.-%, bezogen auf das gesamte Mittel.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO, 4 EO oder 7 EO, C₉-₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Hierbei können Blockcopolymere mit EO-PO-Blockeinheiten bzw. PO-EO-Blockeinheiten eingesetzt werden, aber auch EO-PO-EO-Copolymere bzw. PO-EO-PO-Copolymere. Selbstverständlich sind auch gemischt alkoxylierte Niotenside einsetzbar, in denen EO- und PO-Einheiten nicht blockweise sondern statistisch verteilt sind. Solche Produkte sind durch gleichzeitige Einwirkung von Ethylen- und Propylenoxid auf Fettalkohole erhältlich.

Außerdem können als weitere nichtionische Tenside auch Alkylglykoside der allgemeinen Formel RO(G)ₓ eingesetzt werden, in der R einen primären geradkettigen oder methylverzweigten, insbesondere in 2-Stellung methylverzweigten aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl zwischen 1 und 10; vorzugsweise liegt x bei 1,2 bis 1,4.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind Polyhydroxyfettsäureamide der Formel (TI), 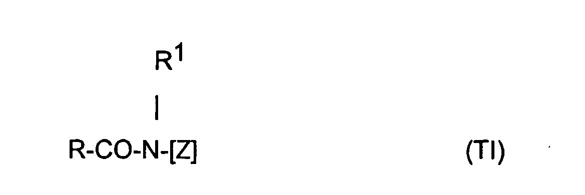 in der RCO für einen aliphatischen Acylrest mit 6 bis 22 Kohlenstoffatomen, R¹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel (TII), 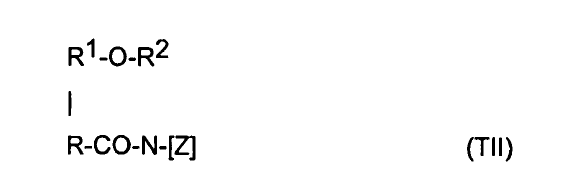 in der R für einen linearen oder verzweigten Alkyl- oder Alkenylrest mit 7 bis 12 Kohlenstoffatomen, R¹ für einen linearen, verzweigten oder cyclischen Alkylrest oder einen Arylrest mit 2 bis 8 Kohlenstoffatomen und R² für einen linearen, verzweigten oder cyclischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propxylierte Derivate dieses Restes.

[Z] wird vorzugsweise durch reduktive Aminierung eines Zuckers erhalten, beispielsweise Glucose, Fructose, Maltose, Lactose, Galactose, Mannose oder Xylose. Die N-Alkoxy- oder N-Aryloxy-substituierten Verbindungen können dann durch Umsetzung mit Fettsäuremethylestern in Gegenwart eines Alkoxids als Katalysator in die gewünschten Polyhydroxyfettsäureamide überführt werden.

Der Gehalt bevorzugter, für die Textilwäsche geeigneter Wasch-, Pflege- oder Reinigungsmittelzusammensetzungen an nichtionischen Tensiden beträgt 5 bis 20 Gew.-%, vorzugsweise 7 bis 15 Gew.-% und insbesondere 9 bis 14 Gew.-%, jeweils bezogen auf das gesamte Mittel.

In maschinellen Geschirrspülmitteln werden vorzugsweise schwachschäumende nichtionische Tenside eingesetzt. Mit besonderem Vorzug enthalten erfindungsgemäße maschinellen Geschirrspülmittel ein nichtionisches Tensid, das einen Schmelzpunkt oberhalb Raumtemperatur aufweist. Demzufolge sind bevorzugte Mittel dadurch gekennzeichnet, daß sie nichtionische(s) Tensid(e) mit einem Schmelzpunkt oberhalb von 20°C, vorzugsweise oberhalb von 25°C, besonders bevorzugt zwischen 25 und 60°C und insbesondere zwischen 26,6 und 43,3°C, enthalten.

Geeignete Niotenside, die Schmelz- bzw. Erweichungspunkte im genannten Temperaturbereich aufweisen, sind beispielsweise schwachschäumende nichtionische Tenside, die bei Raumtemperatur fest oder hochviskos sein können. Werden bei Raumtemperatur hochviskose Niotenside eingesetzt, so ist bevorzugt, daß diese eine Viskosität oberhalb von 20 Pas, vorzugsweise oberhalb von 35 Pas und insbesondere oberhalb 40 Pas aufweisen. Auch Niotenside, die bei Raumtemperatur wachsartige Konsistenz besitzen, sind bevorzugt.

Bevorzugt bei Raumtemperatur feste einzusetzende Niotenside sind ausgewählt aus den Gruppen der alkoxylierten Niotenside, insbesondere der ethoxylierten primären Alkohole und Mischungen dieser Tenside mit strukturell komplizierter aufgebauten Tensiden wie Polyoxypropylen/Polyoxyethylen/Polyoxypropylen (PO/EO/PO)-Tenside. Solche (PO/EO/PO)-Niotenside zeichnen sich darüber hinaus durch gute Schaumkontrolle aus.

In einer bevorzugten Ausführungsform der vorliegenden Erfindung ist das nichtionische Tensid mit einem Schmelzpunkt oberhalb Raumtemperatur ein ethoxyliertes Niotensid, das aus der Reaktion von einem Monohydroxyalkanol oder Alkylphenol mit 6 bis 20 C-Atomen mit vorzugsweise mindestens 12 Mol, besonders bevorzugt mindestens 15 Mol, insbesondere mindestens 20 Mol Ethylenoxid pro Mol Alkohol bzw. Alkylphenol hervorgegangen ist.

Ein besonders bevorzugtes bei Raumtemperatur festes, einzusetzendes Niotensid wird aus einem geradkettigen Fettalkohol mit 16 bis 20 Kohlenstoffatomen (C₁₆₋₂₀-Alkohol), vorzugsweise einem C₁₈-Alkohol und mindestens 12 Mol, vorzugsweise mindestens 15 Mol und insbesondere mindestens 20 Mol Ethylenoxid gewonnen. Hierunter sind die sogenannten "narrow range ethoxylates" (siehe oben) besonders bevorzugt.

Demnach enthalten besonders bevorzugte erfindungsgemäße Mittel ethoxylierte(s) Niotensid(e), das/die aus C₆₋₂₀-Monohydroxyalkanolen oder C₆₋₂₀-Alkylphenolen oder C₁₆₋₂₀-Fettalkoholen und mehr als 12 Mol, vorzugsweise mehr als 15 Mol und insbesondere mehr als 20 Mol Ethylenoxid pro Mol Alkohol gewonnen wurde(n).

Das Niotensid besitzt vorzugsweise zusätzlich Propylenoxideinheiten im Molekül. Vorzugsweise machen solche PO-Einheiten bis zu 25 Gew.-%, besonders bevorzugt bis zu 20 Gew.-% und insbesondere bis zu 15 Gew.-% der gesamten Molmasse des nichtionischen Tensids aus. Besonders bevorzugte nichtionische Tenside sind ethoxylierte Monohydroxyalkanole oder Alkylphenole, die zusätzlich Polyoxyethylen-Polyoxypropylen Blockcopolymereinheiten aufweisen. Der Alkohol- bzw. Alkylphenolteil solcher Niotensidmoleküle macht dabei vorzugsweise mehr als 30 Gew.-%, besonders bevorzugt mehr als 50 Gew.-% und insbesondere mehr als 70 Gew.-% der gesamten Molmasse solcher Niotenside aus. Bevorzugte Klarspülmittel sind dadurch gekennzeichnet, daß sie ethoxylierte und propoxylierte Niotenside enthalten, bei denen die Propylenoxideinheiten im Molekül bis zu 25 Gew.-%, bevorzugt bis zu 20 Gew.-% und insbesondere bis zu 15 Gew.-% der gesamten Molmasse des nichtionischen Tensids ausmachen, enthalten.

Weitere besonders bevorzugt einzusetzende Niotenside mit Schmelzpunkten oberhalb Raumtemperatur enthalten 40 bis 70% eines Polyoxypropylen/Polyoxyethylen/Polyoxypropylen-Blockpolymerblends, der 75 Gew.-% eines umgekehrten Block-Copolymers von Polyoxyethylen und Polyoxypropylen mit 17 Mol Ethylenoxid und 44 Mol Propylenoxid und 25 Gew.-% eines Block-Copolymers von Polyoxyethylen und Polyoxypropylen, initiiert mit Trimethylolpropan und enthaltend 24 Mol Ethylenoxid und 99 Mol Propylenoxid pro Mol Trimethylolpropan.

Nichtionische Tenside, die mit besonderem Vorzug eingesetzt werden können, sind beispielsweise unter dem Namen Poly Tergent^{®} SLF-18 von der Firma Olin Chemicals erhältlich.

Ein weiter bevorzugtes Wasch-, Pflege- oder Reinigungsmittel enthält nichtionische Tenside der Formel

R¹O[CH₂CH(CH₃)O]ₓ[CH₂CH₂O]_{y}[CH₂CH(OH)R²],

in der R¹ für einen linearen oder verzweigten aliphatischen Kohlenwasserstoffrest mit 4 bis 18 Kohlenstoffatomen oder Mischungen hieraus steht, R² einen linearen oder verzweigten Kohlenwasserstoffrest mit 2 bis 26 Kohlenstoffatomen oder Mischungen hieraus bezeichnet und x für Werte zwischen 0,5 und 1,5 und y für einen Wert von mindestens 15 steht.

Weitere bevorzugt einsetzbare Niotenside sind die endgruppenverschlossenen Poly(oxyalkylierten) Niotenside der Formel

R¹O[CH₂CH(R³)O]ₓ[CH₂]ₖCH(OH)[CH₂]ⱼOR²

in der R¹ und R² für lineare oder verzweigte, gesättigte oder ungesättigte, aliphatische oder aromatische Kohlenwasserstoffreste mit 1 bis 30 Kohlenstoffatomen stehen, R³ für H oder einen Methyl-, Ethyl-, n-Propyl-, isoPropyl, n-Butyl-, 2-Butyl- oder 2-Methyl-2-Butylrest steht, x für Werte zwischen 1 und 30, k und j für Werte zwischen 1 und 12, vorzugsweise zwischen 1 und 5 stehen. Wenn der Wert x ≥ 2 ist, kann jedes R³ in der obenstehenden Formel unterschiedlich sein. R¹ und R² sind vorzugsweise lineare oder verzweigte, gesättigte oder ungesättigte, aliphatische oder aromatische Kohlenwasserstoffreste mit 6 bis 22 Kohlenstoffatomen, wobei Reste mit 8 bis 18 C-Atomen besonders bevorzugt sind. Für den Rest R³ sind H, -CH₃ oder -CH₂CH₃ besonders bevorzugt. Besonders bevorzugte Werte für x liegen im Bereich von 1 bis 20, insbesondere von 6 bis 15.

Wie vorstehend beschrieben, kann jedes R³ in der obenstehenden Formel unterschiedlich sein, falls x ≥2 ist. Hierdurch kann die Alkylenoxideinheit in der eckigen Klammer variiert werden. Steht x beispielsweise für 3, kann der Rest R³ ausgewählt werden, um Ethylenoxid- (R³ = H) oder Propylenoxid- (R³ = CH₃) Einheiten zu bilden, die in jedweder Reihenfolge aneinandergefügt sein können, beispielsweise (EO)(PO)(EO), (EO)(EO)(PO), (EO)(EO)(EO), (PO)(EO)(PO), (PO)(PO)(EO) und (PO)(PO)(PO). Der Wert 3 für x ist hierbei beispielhaft gewählt worden und kann durchaus größer sein, wobei die Variationsbreite mit steigenden x-Werten zunimmt und beispielsweise eine große Anzahl (EO)-Gruppen, kombiniert mit einer geringen Anzahl (PO)-Gruppen einschließt, oder umgekehrt.

Insbesondere bevorzugte endgruppenverschlossenen Poly(oxyalkylierte) Alkohole der obenstehenden Formel weisen Werte von k = 1 und j = 1 auf, so daß sich die vorstehende Formel zu

R¹O[CH₂CH(R³)O]ₓCH₂CH(OH)CH₂OR²

vereinfacht. In der letztgenannten Formel sind R¹, R² und R³ wie oben definiert und x steht für Zahlen von 1 bis 30, vorzugsweise von 1 bis 20 und insbesonders von 6 bis 18. Besonders bevorzugt sind Tenside, bei denen die Reste R¹ und R² 9 bis 14 C-Atome aufweisen, R³ für H steht und x Werte von 6 bis 15 annimmt.

Faßt man die letztgenannten Aussagen zusammen, sind Wasch-, Pflege- oder Reinigungsmittel bevorzugt, die endgruppenverschlossenen Poly(oxyalkylierten) Niotenside der Formel

R¹O[CH₂CH(R³)O]ₓ[CH₂]ₖCH(OH)[CH₂]ⱼOR²

enthalten, in der R¹ und R² für lineare oder verzweigte, gesättigte oder ungesättigte, aliphatische oder aromatische Kohlenwasserstoffreste mit 1 bis 30 Kohlenstoffatomen stehen, R³ für H oder einen Methyl-, Ethyl-, n-Propyl-, isoPropyl, n-Butyl-, 2-Butyl- oder 2-Methyl-2-Butylrest steht, x für Werte zwischen 1 und 30, k und j für Werte zwischen 1 und 12, vorzugsweise zwischen 1 und 5 stehen, wobei Tenside des Typs

R¹O[CH₂CH(R³)O]ₓCH₂CH(OH)CH₂OR²

in denen x für Zahlen von 1 bis 30, vorzugsweise von 1 bis 20 und insbesonder von 6 bis 18 steht, besonders bevorzugt sind.

In Verbindung mit den genannten Tensiden können auch anionische, kationische und/oder amphotere Tenside eingesetzt werden, wobei diese wegen ihres Schaumverhaltens in maschinellen Geschirrspülmitteln nur untergeordnete Bedeutung besitzen und zumeist nur in Mengen unterhalb von 10 Gew.-%, meistens sogar unterhalb von 5 Gew.-%, beispielsweise von 0,01 bis 2,5 Gew.-%, jeweils bezogen auf das Mittel, eingesetzt werden. Die Mittel können somit als Tensidkomponente auch anionische, kationische und/oder amphotere Tenside enthalten.

Als kationische Aktivsubstanzen können die erfindungsgemäßen Mittel beispielsweise kationische Verbindungen der Formeln (TIII), (TIV) oder (TV) enthalten: 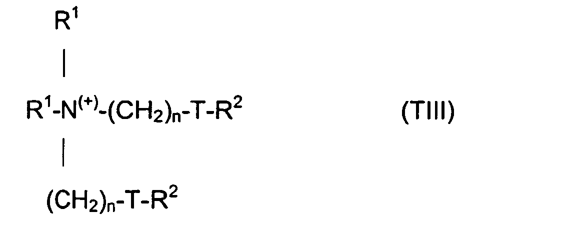 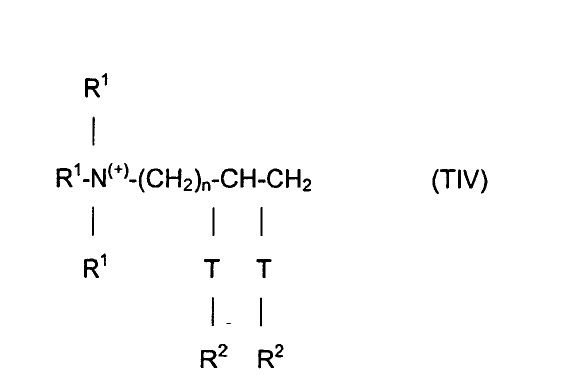 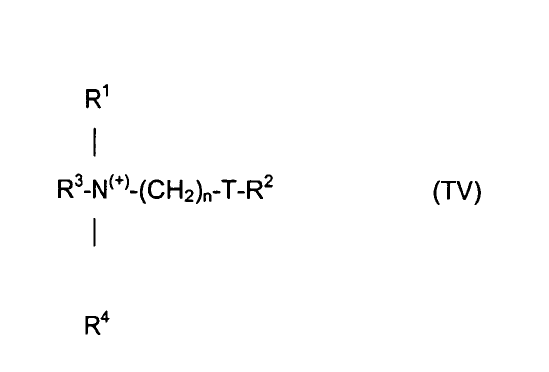 worin jede Gruppe R¹ unabhängig voneinander ausgewählt ist aus C₁₋₆-Alkyl-, -Alkenyl- oder -Hydroxyalkylgruppen; jede Gruppe R² unabhängig voneinander ausgewählt ist aus C₈₋₂₈-Alkyl- oder -Alkenylgruppen; R³ = R¹ oder (CH2)ₙ-T-R²; R⁴ = R¹ oder R² oder (CH₂)ₙ-T-R²; T = -CH₂-, -O-CO- oder -CO-O- und n eine ganze Zahl von 0 bis 5 ist.

Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C₁₂₋₁₈-Alkanen beispielsweise durch Sulfochlorierung oder Sulfoxidation mit anschließender Hydrolyse bzw. Neutralisation gewonnen werden. Ebenso sind auch die Ester von α-Sulfofettsäuren (Estersulfonate), z.B. die α-sulfonierten Methylester der hydrierten Kokos-, Palmkern- oder Talgfettsäuren geeignet.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate, welche beispielsweise gemäß den US-Patentschriften 3,234,258 oder 5,075,041 hergestellt werden und als Handelsprodukte der Shell Oil Company unter dem Namen DAN^{®} erhalten werden können, sind geeignete Aniontenside.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet. Sie werden in Reinigungsmitteln aufgrund ihres hohen Schaumverhaltens nur in relativ geringen Mengen, beispielsweise in Mengen von 1 bis 5 Gew.-%, eingesetzt.

Weitere geeignete Aniontenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C₈₋₁₈-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen (Beschreibung siehe unten). Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.

Als weitere anionische Tenside kommen insbesondere Seifen in Betracht. Geeignet sind gesättigte und ungesättigte Fettsäureseifen, wie die Salze der Laurinsäure, Myristinsäure, Palmitinsäure, Stearinsäure, (hydrierten) Erucasäure und Behensäure sowie insbesondere aus natürlichen Fettsäuren, z.B. Kokos-, Palmkern-, Olivenöl- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Der Gehalt bevorzugter erfindungsgemäßer Textilwaschmittel an anionischen Tensiden beträgt 5 bis 25 Gew.-%, vorzugsweise 7 bis 22 Gew.-% und insbesondere 10 bis 20 Gew.-%, jeweils bezogen auf das gesamte Mittel.

Im Rahmen der vorliegenden Erfindung enthalten bevorzugt verwendete Mittel zusätzlich einen oder mehrere Stoffe aus der Gruppe der Gerüststoffe, Bleichmittel, Bleichaktivatoren, Enzyme, Elektrolyte, nichtwäßrigen Lösungsmittel, pH-Stellmittel, Duftstoffe, Parfümträger, Fluoreszenzmittel, Farbstoffe, Hydrotope, Schauminhibitoren, Silikonöle, Antiredepositionsmittel, optischen Aufheller, Vergrauungsinhibitoren, Einlaufverhinderer, Knitterschutzmittel, Farbübertragungsinhibitoren, antimikrobiellen Wirkstoffe, Germizide, Fungizide, Antioxidantien, Korrosionsinhibitoren, Antistatika, Bügelhilfsmittel, Phobier- und Imprägniermittel, Quell- und Schiebefestmittel sowie UV-Absorber.

Als Gerüststoffe, die in den Mitteln enthalten sein können, sind insbesondere Phosphate, Silikate, Aluminiumsilikate (insbesondere Zeolithe), Carbonate, Salze organischer Di- und Polycarbonsäuren sowie Mischungen dieser Stoffe zu nennen.

Der Einsatz der allgemein bekannten Phosphate als Buildersubstanzen ist möglich, sofern ein derartiger Einsatz nicht aus ökologischen Gründen vermieden werden sollte. Unter der Vielzahl der kommerziell erhältlichen Phosphate haben die Alkalimetallphosphate unter besonderer Bevorzugung von Pentanatrium- bzw. Pentakaliumtriphosphat (Natrium- bzw. Kaliumtripolyphosphat) in der Wasch- und Reinigungsmittel-Industrie die größte Bedeutung.

Alkalimetallphosphate ist dabei die summarische Bezeichnung für die Alkalimetall-(insbesondere Natrium- und Kalium-) -Salze der verschiedenen Phosphorsäuren, bei denen man Metaphosphorsäuren (HPO₃)ₙ und Orthophosphorsäure H₃PO₄ neben höhermolekularen Vertretern unterscheiden kann. Die Phosphate vereinen dabei mehrere Vorteile in sich: Sie wirken als Alkaliträger, verhindern Kalkbeläge auf Maschinenteilen bzw. Kalkinkrustationen in Geweben und tragen überdies zur Reinigungsleistung bei.

Natriumdihydrogenphosphat, NaH₂PO₄, existiert als Dihydrat (Dichte 1,91 gcm⁻³, Schmelzpunkt 60°) und als Monohydrat (Dichte 2,04 gcm⁻³). Beide Salze sind weiße, in Wasser sehr leicht lösliche Pulver, die beim Erhitzen das Kristallwasser verlieren und bei 200°C in das schwach saure Diphosphat (Dinatriumhydrogendiphosphat, Na₂H₂P₂O₇), bei höherer Temperatur in Natiumtrimetaphosphat (Na₃P₃O₉) und Maddrellsches Salz (siehe unten), übergehen. NaH₂PO₄ reagiert sauer; es entsteht, wenn Phosphorsäure mit Natronlauge auf einen pH-Wert von 4,5 eingestellt und die Maische versprüht wird. Kaliumdihydrogenphosphat (primäres oder einbasiges Kaliumphosphat, Kaliumbiphosphat, KDP), KH₂PO₄, ist ein weißes Salz der Dichte 2,33 gcm⁻³, hat einen Schmelzpunkt 253° [Zersetzung unter Bildung von Kaliumpolyphosphat (KPO₃)ₓ] und ist leicht löslich in Wasser.

Dinatriumhydrogenphosphat (sekundäres Natriumphosphat), Na₂HPO₄, ist ein farbloses, sehr leicht wasserlösliches kristallines Salz. Es existiert wasserfrei und mit 2 Mol. (Dichte 2,066 gcm⁻³, Wasserverlust bei 95°), 7 Mol. (Dichte 1,68 gcm⁻³, Schmelzpunkt 48° unter Verlust von 5 H₂O) und 12 Mol. Wasser (Dichte 1,52 gcm-³, Schmelzpunkt 35° unter Verlust von 5 H₂O), wird bei 100° wasserfrei und geht bei stärkerem Erhitzen in das Diphosphat Na₄P₂O₇ über. Dinatriumhydrogenphosphat wird durch Neutralisation von Phosphorsäure mit Sodalösung unter Verwendung von Phenolphthalein als Indikator hergestellt. Dikaliumhydrogenphosphat (sekundäres od. zweibasiges Kaliumphosphat), K₂HPO₄, ist ein amorphes, weißes Salz, das in Wasser leicht löslich ist.

Trinatriumphosphat, tertiäres Natriumphosphat, Na₃PO₄, sind farblose Kristalle, die als Dodecahydrat eine Dichte von 1,62 gcm⁻³ und einen Schmelzpunkt von 73-76°C (Zersetzung), als Decahydrat (entsprechend 19-20% P₂O₅) einen Schmelzpunkt von 100°C und in wasserfreier Form (entsprechend 39-40% P₂O₅) eine Dichte von 2,536 gcm⁻³ aufweisen. Trinatriumphosphat ist in Wasser unter alkalischer Reaktion leicht löslich und wird durch Eindampfen einer Lösung aus genau 1 Mol Dinatriumphosphat und 1 Mol NaOH hergestellt. Trikaliumphosphat (tertiäres oder dreibasiges Kaliumphosphat), K₃PO₄, ist ein weißes, zerfließliches, körniges Pulver der Dichte 2,56 gcm⁻³, hat einen Schmelzpunkt von 1340° und ist in Wasser mit alkalischer Reaktion leicht löslich. Es entsteht z.B. beim Erhitzen von Thomasschlacke mit Kohle und Kaliumsulfat. Trotz des höheren Preises werden in der Reinigungsmittel-Industrie die leichter löslichen, daher hochwirksamen, Kaliumphosphate gegenüber entsprechenden Natrium-Verbindungen vielfach bevorzugt.

Tetranatriumdiphosphat (Natriumpyrophosphat), Na₄P₂O₇, existiert in wasserfreier Form (Dichte 2,534 gcm-³, Schmelzpunkt 988°, auch 880° angegeben) und als Decahydrat (Dichte 1,815-1,836 gcm⁻³, Schmelzpunkt 94° unter Wasserverlust). Bei Substanzen sind farblose, in Wasser mit alkalischer Reaktion lösliche Kristalle. Na₄P₂O₇ entsteht beim Erhitzen von Dinatriumphosphat auf >200° oder indem man Phosphorsäure mit Soda im stöchiometrischem Verhältnis umsetzt und die Lösung durch Versprühen entwässert. Das Decahydrat komplexiert Schwermetall-Salze und Härtebildner und verringert daher die Härte des Wassers. Kaliumdiphosphat (Kaliumpyrophosphat), K₄P₂O₇, existiert in Form des Trihydrats und stellt ein farbloses, hygroskopisches Pulver mit der Dichte 2,33 gcm⁻³ dar, das in Wasser löslich ist, wobei der pH-Wert der 1%igen Lösung bei 25° 10,4 beträgt. Durch Kondensation des NaH₂PO₄ bzw. des KH₂PO₄ entstehen höhermol. Natrium- und Kaliumphosphate, bei denen man cyclische Vertreter, die Natrium- bzw. Kaliummetaphosphate und kettenförmige Typen, die Natrium- bzw. Kaliumpolyphosphate, unterscheiden kann. Insbesondere für letztere sind eine Vielzahl von Bezeichnungen in Gebrauch: Schmelz- oder Glühphosphate, Grahamsches Salz, Kurrolsches und Maddrellsches Salz. Alle höheren Natrium- und Kaliumphosphate werden gemeinsam als kondensierte Phosphate bezeichnet.

Das technisch wichtige Pentanatriumtriphosphat, Na₅P₃O₁₀ (Natriumtripolyphosphat), ist ein wasserfrei oder mit 6 H₂O kristallisierendes, nicht hygroskopisches, weißes, wasserlösliches Salz der allgemeinen Formel NaO-[P(O)(ONa)-O]ₙ-Na mit n=3. In 100 g Wasser lösen sich bei Zimmertemperatur etwa 17 g, bei 60° ca. 20 g, bei 100° rund 32 g des kristallwasserfreien Salzes; nach zweistündigem Erhitzen der Lösung auf 100° entstehen durch Hydrolyse etwa 8% Orthophosphat und 15% Diphosphat. Bei der Herstellung von Pentanatriumtriphosphat wird Phosphorsäure mit Sodalösung oder Natronlauge im stöchiometrischen Verhältnis zur Reaktion gebracht und die Lsg. durch Versprühen entwässert. Ähnlich wie Grahamsches Salz und Natriumdiphosphat löst Pentanatriumtriphosphat viele unlösliche Metall-Verbindungen (auch Kalkseifen usw.). Pentakaliumtriphosphat, K₅P₃O₁₀ (Kaliumtripolyphosphat), kommt beispielsweise in Form einer 50 Gew.-%igen Lösung (> 23% P₂O₅, 25% K₂O) in den Handel. Die Kaliumpolyphosphate finden in der Wasch- und Reinigungsmittel-Industrie breite Verwendung. Weiter existieren auch Natriumkaliumtripolyphosphate, welche ebenfalls im Rahmen der vorliegenden Erfindung einsetzbar sind. Diese entstehen beispielsweise, wenn man Natriumtrimetaphosphat mit KOH hydrolysiert:

(NaPO₃)₃ + 2 KOH → Na₃K₂P₃O₁₀ + H₂O

Diese sind genau wie Natriumtripolyphosphat, Kaliumtripolyphosphat oder Mischungen aus diesen beiden einsetzbar; auch Mischungen aus Natriumtripolyphosphat und Natriumkaliumtripolyphosphat oder Mischungen aus Kaliumtripolyphosphat und Natriumkaliumtripolyphosphat oder Gemische aus Natriumtripolyphosphat und Kaliumtripolyphosphat und Natriumkaliumtripolyphosphat sind einsetzbar.

Geeignete kristalline, schichtförmige Natriumsilikate besitzen die allgemeine Formel NaMSiₓO₂ₓ₊₁ H₂O, wobei M Natrium oder Wasserstoff bedeutet, x eine Zahl von 1,9 bis 4 und y eine Zahl von 0 bis 20 ist und bevorzugte Werte für x 2, 3 oder 4 sind. Bevorzugte kristalline Schichtsilikate der angegebenen Formel sind solche, in denen M für Natrium steht und x die Werte 2 oder 3 annimmt. Insbesondere sind sowohl β- als auch δ-Natriumdisilikate Na₂Si₂O₅·yH₂O bevorzugt.

Einsetzbar sind auch amorphe Natriumsilikate mit einem Modul Na₂O: SiO₂ von 1:2 bis 1:3,3, vorzugsweise von 1:2 bis 1:2,8 und insbesondere von 1:2 bis 1:2,6, welche löseverzögert sind und Sekundärwascheigenschaften aufweisen. Die Löseverzögerung gegenüber herkömmlichen amorphen Natriumsilikaten kann dabei auf verschiedene Weise, beispielsweise durch Oberflächenbehandlung, Compoundierung, Kompaktierung/ Verdichtung oder durch Übertrocknung hervorgerufen worden sein. lm Rahmen dieser Erfindung wird unter dem Begriff "amorph" auch "röntgenamorph" verstanden. Dies heißt, daß die Silikate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Buildereigenschaften führen, wenn die Silikatpartikel bei Elektronenbeugungsexperimenten verwaschene oder sogar scharfe Beugungsmaxima liefern. Dies ist so zu interpretieren, daß die Produkte mikrokristalline Bereiche der Größe 10 bis einige Hundert nm aufweisen, wobei Werte bis max. 50 nm und insbesondere bis max. 20 nm bevorzugt sind. Derartige sogenannte röntgenamorphe Silikate, weisen ebenfalls eine Löseverzögerung gegenüber den herkömmlichen Wassergläsern auf. Insbesondere bevorzugt sind verdichtete/kompaktierte amorphe Silikate, compoundierte amorphe Silikate und übertrocknete röntgenamorphe Silikate.

Der eingesetzte feinkristalline, synthetische und gebundenes Wasser enthaltende Zeolith ist vorzugsweise Zeolith A und/oder P. Als Zeolith P wird Zeolith MAP^{®} (Handelsprodukt der Firma Crosfield) besonders bevorzugt. Geeignet sind jedoch auch Zeolith X sowie Mischungen aus A, X und/oder P. Kommerziell erhältlich und im Rahmen der vorliegenden Erfindung bevorzugt einsetzbar ist beispielsweise auch ein Co-Kristallisat aus Zeolith X und Zeolith A (ca. 80 Gew.-% Zeolith X), das von der Firma CONDEA Augusta S.p.A. unter dem Markennamen VEGOBOND AX^{®} vertrieben wird und durch die Formel

nNa₂O · (1-n)K₂O · Al₂O₃ · (2 - 2,5)SiO₂ · (3,5 - 5,5) H₂O

beschrieben werden kann. Der Zeolith kann als sprühgetrocknetes Pulver oder auch als ungetrocknete, von ihrer Herstellung noch feuchte, stabilisierte Suspension zum Einsatz kommen. Für den Fall, daß der Zeolith als Suspension eingesetzt wird, kann diese geringe Zusätze an nichtionischen Tensiden als Stabilisatoren enthalten, beispielsweise 1 bis 3 Gew.-%, bezogen auf Zeolith, an ethoxylierten C₁₂-C₁₈-Fettalkoholen mit 2 bis 5 Ethylenoxidgruppen, C₁₂-C₁₄-Fettalkoholen mit 4 bis 5 Ethylenoxidgruppen oder ethoxylierten Isotridecanolen. Geeignete Zeolithe weisen eine mittlere Teilchengröße von weniger als 10 µm (Volumenverteilung; Meßmethode: Coulter Counter) auf und enthalten vorzugsweise 18 bis 22 Gew.-%, insbesondere 20 bis 22 Gew.-% an gebundenem Wasser.

Weitere wichtige Gerüststoffe sind insbesondere die Carbonate, Citrate und Silikate. Bevorzugt werden Trinatriumcitrat und/oder Pentanatriumtripolyphosphat und/oder Natriumcarbonat und/oder Natriumbicarbonat und/oder Gluconate und/oder silikatische Builder aus der Klasse der Disilikate und/oder Metasilikate eingesetzt.

Als weitere Bestandteile können Alkaliträger zugegen sein. Als Alkaliträger gelten Alkalimetallhydroxide, Alkalimetallcarbonate, Alkalimetallhydrogencarbonate, Alkalimetallsesquicarbonate, Alkalisilikate, Alkalimetasilikate, und Mischungen der vorgenannten Stoffe, wobei im Sinne dieser Erfindung bevorzugt die Alkalicarbonate, insbesondere Natriumcarbonat, Natriumhydrogencarbonat oder Natriumsesquicarbonat eingesetzt werden.

Besonders bevorzugt ist ein Buildersystem enthaltend eine Mischung aus Tripolyphosphat und Natriumcarbonat.

Ebenfalls besonders bevorzugt ist ein Buildersystem enthaltend eine Mischung aus Tripolyphosphat und Natriumcarbonat und Natriumdisilikat.

Daneben können weitere Inhaltsstoffe zugegen sein, wobei Wasch-, Pflege- oder Reinigungsmittel bevorzugt sind, die zusätzlich einen oder mehrere Stoffe aus der Gruppe der Acidifizierungsmittel, Chelatkomplexbildner oder der belagsinhibierenden Polymere enthalten.

Als Acidifizierungsmittel bieten sich sowohl anorganische Säuren als auch organische Säuren an, sofern diese mit den übrigen Inhaltsstoffen verträglich sind. Aus Gründen des Verbraucherschutzes und der Handhabungssicherheit sind insbesondere die festen Mono-, Oligo- und Polycarbonsäuren einsetzbar. Aus dieser Gruppe wiederum bevorzugt sind Citronensäure, Weinsäure, Bernsteinsäure, Malonsäure, Adipinsäure, Maleinsäure, Fumarsäure, Oxalsäure sowie Polyacrylsäure. Auch die Anhydride dieser Säuren können als Acidifizierungsmittel eingesetzt werden, wobei insbesondere Maleinsäureanhydrid und Bernsteinsäureanhydrid kommerziell verfügbar sind. Organische Sulfonsäuren wie Amidosulfonsäure sind ebenfalls einsetzbar. Kommerziell erhältlich und als Acidifizierungsmittel im Rahmen der vorliegenden Erfindung ebenfalls bevorzugt einsetzbar ist Sokalan^{®} DCS (Warenzeichen der BASF), ein Gemisch aus Bernsteinsäure (max. 31 Gew.-%), Glutarsäure (max. 50 Gew.-%) und Adipinsäure (max. 33 Gew.-%).

Eine weitere mögliche Gruppe von Inhaltsstoffen stellen die Chelatkomplexbildner dar. Chelatkomplexbildner sind Stoffe, die mit Metallionen cyclische Verbindungen bilden, wobei ein einzelner Ligand mehr als eine Koordinationsstelle an einem Zentralatom besetzt, d. h. mind. "zweizähnig" ist. In diesem Falle werden also normalerweise gestreckte Verbindungen durch Komplexbildung über ein lon zu Ringen geschlossen. Die Zahl der gebundenen Liganden hängt von der Koordinationszahl des zentralen lons ab.

Gebräuchliche und im Rahmen der vorliegenden Erfindung bevorzugte Chelatkomplexbilder sind beispielsweise Polyoxycarbonsäuren, Polyamine, Ethylendiamintetraessigsäure (EDTA) und Nitrilotriessigsäure (NTA). Auch komplexbildende Polymere, also Polymere, die entweder in der Hauptkette selbst oder seitenständig zu dieser funktionelle Gruppen tragen, die als Liganden wirken können und mit geeigneten Metall-Atomen in der Regel unter Bildung von ChelatKomplexen reagieren, sind einsetzbar. Die Polymergebundenen Liganden der entstehenden Metall-Komplexe können dabei aus nur einem Makromolekül stammen oder aber zu verschiedenen Polymerketten gehören. Letzteres führt zur Vernetzung des Materials, sofern die komplexbildenden Polymere nicht bereits zuvor über kovalente Bindungen vernetzt waren.

Komplexierende Gruppen (Liganden) üblicher komplexbildender Polymere sind Iminodi-essigsäure-, Hydroxychinolin-, Thioharnstoff-, Guanidin-, Dithiocarbamat-, Hydroxamsäure-, Amidoxim-, Aminophosphorsäure-, (cycl.) Polyamino-, Mercapto-, 1,3-Dicarbonyl- und Kronenether-Reste mit z. T. sehr spezif. Aktivitäten gegenüber Ionen unterschiedlicher Metalle. Basispolymere vieler auch kommerziell bedeutender komplexbildender Polymere sind Polystyrol, Polyacrylate, Polyacrylnitrile, Polyvinylalkohole, Polyvinylpyridine und Polyethylenimine. Auch natürliche Polymere wie Cellulose, Stärke od. Chitin sind komplexbildende Polymere. Darüber hinaus können diese durch polymeranaloge Umwandlungen mit weiteren Ligand-Funktionalitäten versehen werden.

Besonders bevorzugt sind Mittel, insbesondere Wasch-, Pflege- und/oder Reinigungsmittel, die ein oder mehrere Chelatkomplexbildner aus den Gruppen der
(i) Polycarbonsäuren, bei denen die Summe der Carboxyl- und gegebenenfalls Hydroxylgruppen mindestens 5 beträgt,
(ii) stickstoffhaltigen Mono- oder Polycarbonsäuren,
(iii) geminalen Diphosphonsäuren,
(iv) Aminophosphonsäuren,
(v) Phosphonopolycarbonsäuren,
(vi) Cyclodextrine
in Mengen oberhalb von 0,1 Gew.-%, vorzugsweise oberhalb von 0,5 Gew.-%, besonders bevorzugt oberhalb von 1 Gew.-% und insbesondere oberhalb von 2,5 Gew.-%, jeweils bezogen auf das Gewicht des Geschirrspülmittels, enthalten.

Alle Komplexbildner des Standes der Technik können eingesetzt werden. Diese können unterschiedlichen chemischen Gruppen angehören. Vorzugsweise werden einzeln oder im Gemisch miteinander eingesetzt:
a) Polycarbonsäuren, bei denen die Summe der Carboxyl- und gegebenenfalls Hydroxylgruppen mindestens 5 beträgt wie Gluconsäure,
b) stickstoffhaltige Mono- oder Polycarbonsäuren wie Ethylendiamintetraessigsäure (EDTA), N-Hydroxyethylethylendiamintriessigsäure, Diethylentriaminpentaessigsäure, Hydroxyethyliminodiessigsäure, Nitridodiessigsäure-3-propionsäure, Isoserindiessigsäure, N,N-Di-(β-hydroxyethyl)-glycin, N-(1,2-Dicarboxy-2-hydroxyethyl)-glycin, N-(1,2-Dicarboxy-2-hydroxyethyl)-asparaginsäure oder Nitrilotriessigsäure (NTA),
c) geminale Diphosphonsäuren wie 1-Hydroxyethan-1,1-diphosphonsäure (HEDP), deren höhere Homologe mit bis zu 8 Kohlenstoffatomen sowie Hydroxy- oder Aminogruppen-haltige Derivate hiervon und 1-Aminoethan-1,1-diphosphonsäure, deren höhere Homologe mit bis zu 8 Kohlenstoffatomen sowie Hydroxy- oder Aminogruppen-haltige Derivate hiervon,
d) Aminophosphonsäuren wie Ethylendiamintetra(methylenphosphonsäure), Di-ethylen-triaminpenta(methylenphosphonsäure) oder Nitrilotri(methylen-phosphonsäure),
e) Phosphonopolycarbonsäuren wie 2-Phosphonobutan-1,2,4-tricarbonsäure sowie
f) Cyclodextrine.

Als Polycarbonsäuren a) werden im Rahmen dieser Patentanmeldung Carbonsäuren -auch Monocarbonsäuren- verstanden, bei denen die Summe aus Carboxyl- und den im Molekül enthaltenen Hydroxylgruppen mindestens 5 beträgt. Komplexbildner aus der Gruppe der stickstoffhaltigen Polycarbonsäuren, insbesondere EDTA, sind bevorzugt. Bei den erfindungsgemäß erforderlichen alkalischen pH-Werten der Behandlungslösungen liegen diese Komplexbildner zumindest teilweise als Anionen vor. Es ist unwesentlich, ob sie in Form der Säuren oder in Form von Salzen eingebracht werden. Im Falle des Einsatzes als Salze sind Alkali-, Ammonium- oder Alkylammoniumsalze, insbesondere Natriumsalze, bevorzugt.

Belagsinhibierende Polymere können ebenfalls in den erfindungsgemäßen Mitteln enthalten sein. Diese Stoffe, die chemisch verschieden aufgebaut sein könne, stammen beispielsweise aus den Gruppen der niedermolekularen Polyacrylate mit Molmassen zwischen 1000 und 20.000 Dalton, wobei Polymere mit Molmassen unter 15.000 Dalton bevorzugt sind.

Belagsinhibierende Polymere können auch Cobuildereigenschaften aufweisen. Als organische Cobuilder können in den erfindungsgemäßen maschinellen Geschirrspülmitteln insbesondere Polycarboxylate / Polycarbonsäuren, polymere Polycarboxylate, Asparaginsäure, Polyacetale, Dextrine, weitere organische Cobuilder (siehe unten) sowie Phosphonate eingesetzt werden. Diese Stoffklassen werden nachfolgend beschrieben.

Brauchbare organische Gerüstsubstanzen sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen.

Auch die Säuren an sich können eingesetzt werden. Die Säuren besitzen neben ihrer Builderwirkung typischerweise auch die Eigenschaft einer Säuerungskomponente und dienen somit auch zur Einstellung eines niedrigeren und milderen pH-Wertes des Mittels, wie von Wasch- oder Reinigungsmitteln. Insbesondere sind hierbei Citronensäure, Bernsteinsäure, Glutarsäure, Adipinsäure, Gluconsäure und beliebige Mischungen aus diesen zu nennen.

Als Builder bzw. Belagsinhibitor sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70000 g/mol.

Bei den für polymere Polycarboxylate angegebenen Molmassen handelt es sich im Sinne dieser Schrift um gewichtsmittlere Molmassen M_{w} der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Geeignete Polymere sind insbesondere Polyacrylate, die bevorzugt eine Molekülmasse von 500 bis 20000 g/mol aufweisen. Aufgrund ihrer überlegenen Löslichkeit können aus dieser Gruppe wiederum die kurzkettigen Polyacrylate, die Molmassen von 1000 bis 10000 g/mol, und besonders bevorzugt von 1000 bis 4000 g/mol, aufweisen, bevorzugt sein.

Besonders bevorzugt werden in den Mitteln sowohl Polyacrylate als auch Copolymere aus ungesättigten Carbonsäuren, Sulfonsäuregruppen-haltigen Monomeren sowie gegebenenfalls weiteren ionischen oder nichtionogenen Monomeren eingesetzt. Die Sulfonsäuregruppen-haltigen Copolymere werden weiter unten ausführlich beschrieben.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 2000 bis 70000 g/mol, vorzugsweise 20000 bis 50000 g/mol und insbesondere 30000 bis 40000 g/mol.

Die (co-)polymeren Polycarboxylate können entweder als Pulver oder als wäßrige Lösung eingesetzt werden. Der Gehalt der Mittel an (co-)polymeren Polycarboxylaten beträgt vorzugsweise 0,5 bis 20 Gew.-%, insbesondere 3 bis 10 Gew.-%.

Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol bzw. Vinylalkohol-Derivate oder die als Monomere Salze der Acrylsäure und der 2-Alkylallylsulfonsäure sowie Zucker-Derivate enthalten. Weitere bevorzugte Copolymere sind solche, die als Monomere vorzugsweise Acrolein und Acrylsäure/Acrylsäuresalze bzw. Acrolein und Vinylacetat aufweisen.

Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren bzw. deren Salze und Derivate, die neben Cobuilder-Eigenschaften auch eine bleichstabilisierende Wirkung aufweisen.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere bzw. Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500000 g/mol. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose, welche ein DE von 100 besitzt, ist. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2000 bis 30000 g/mol.

Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Ein an C₆ des Saccharidrings oxidiertes Produkt kann besonders vorteilhaft sein.

Auch Oxydisuccinate und andere Derivate von Disuccinaten, vorzugsweise Ethylendiamindisuccinat, sind weitere geeignete Cobuilder. Dabei wird Ethylendiamin-N,N'-disuccinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate. Geeignete Einsatzmengen liegen in zeolithhaltigen und/oder silicathaltigen Formulierungen bei 3 bis 15 Gew.%.

Weitere brauchbare organische Cobuilder sind beispielsweise acetylierte Hydroxycarbonsäuren bzw. deren Salze, welche gegebenenfalls auch in Lactonform vorliegen können und welche mindestens 4 Kohlenstoffatome und mindestens eine Hydroxygruppe sowie maximal zwei Säuregruppen enthalten.

Eine weitere Substanzklasse mit Cobuildereigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Zusätzlich zu den Stoffen aus den genannten Stoffklassen können die Mittel weitere übliche Inhaltsstoffe von Wasch-, Pflege- oder Reinigungsmitteln enthalten, wobei insbesondere Bleichmittel, Bleichaktivatoren, Enzyme, Silberschutzmittel, Farb- und Duftstoffe von Bedeutung sind. Diese Stoffe werden nachstehend beschrieben.

Unter den als Bleichmittel dienenden, in Wasser H₂O₂ liefernden Verbindungen haben das Natriumperborattetrahydrat und das Natriumperboratmonohydrat besondere Bedeutung. Weitere brauchbare Bleichmittel sind beispielsweise Natriumpercarbonat, Peroxypyrophosphate, Citratperhydrate sowie H₂O₂ liefernde persaure Salze oder Persäuren, wie Perbenzoate, Peroxophthalate, Diperazelainsäure, Phthaloiminopersäure oder Diperdodecandisäure.

Um beim Waschen bei Temperaturen von 60 °C und darunter eine verbesserte Bleichwirkung zu erreichen, können Bleichaktivatoren in die Wasch- und Reinigungsmittelformkörper eingearbeitet werden. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die 0- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Zusätzlich zu den konventionellen Bleichaktivatoren oder an deren Stelle können auch sogenannte Bleichkatalysatoren verwendet werden. Bei diesen Stoffen handelt es sich um bleichverstärkende Übergangsmetallsalze bzw. Übergangsmetallkomplexe wie beispielsweise Mn-, Fe-, Co-, Ru - oder Mo-Salenkomplexe oder -carbonylkomplexe. Auch Mn-, Fe-, Co-, Ru-, Mo-, Ti-, V- und Cu-Komplexe mit stickstoffhaltigen Tripod-Liganden sowie Co-, Fe-, Cu- und Ru-Amminkomplexe sind als Bleichkatalysatoren verwendbar.

Als Enzyme kommen insbesondere solche aus der Klassen der Hydrolasen wie der Proteasen, Esterasen, Lipasen bzw. lipolytisch wirkende Enzyme, Amylasen, Cellulasen bzw. andere Glykosylhydrolasen und Gemische der genannten Enzyme in Frage. Alle diese Hydrolasen tragen in der Wäsche zur Entfernung von Verfleckungen wie protein-, fett- oder stärkehaltigen Verfleckungen und Vergrauungen bei. Cellulasen und andere Glykosylhydrolasen können darüber hinaus durch das Entfernen von Pilling und Mikrofibrillen zur Farberhaltung und zur Erhöhung der Weichheit des Textils beitragen. Zur Bleiche bzw. zur Hemmung der Farbübertragung können auch Oxireduktasen eingesetzt werden. Besonders gut geeignet sind aus Bakterienstämmen oder Pilzen wie Bacillus subtilis, Bacillus licheniformis, Streptomyceus griseus und Humicola insolens gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase bzw. lipolytisch wirkenden Enzymen oder Protease und Cellulase oder aus Cellulase und Lipase bzw. lipolytisch wirkenden Enzymen oder aus Protease, Amylase und Lipase bzw. lipolytisch wirkenden Enzymen oder Protease, Lipase bzw. lipolytisch wirkenden Enzymen und Cellulase, insbesondere jedoch Protease und/oder Lipase-haltige Mischungen bzw. Mischungen mit lipolytisch wirkenden Enzymen von besonderem Interesse. Beispiele für derartige lipolytisch wirkende Enzyme sind die bekannten Cutinasen. Auch Peroxidasen oder Oxidasen haben sich in einigen Fällen als geeignet erwiesen. Zu den geeigneten Amylasen zählen insbesondere α-Amylasen, Iso-Amylasen, Pullulanasen und Pektinasen. Als Cellulasen werden vorzugsweise Cellobiohydrolasen, Endoglucanasen und β-Glucosidasen, die auch Cellobiasen genannt werden, bzw. Mischungen aus diesen eingesetzt. Da sich verschiedene Cellulase-Typen durch ihre CMCase- und Avicelase-Aktivitäten unterscheiden, können durch gezielte Mischungen der Cellulasen die gewünschten Aktivitäten eingestellt werden.

Die Enzyme können an Trägerstoffe adsorbiert oder in Hüllsubstanzen eingebettet sein, um sie gegen vorzeitige Zersetzung zu schützen. Der Anteil der Enzyme, Enzymmischungen oder Enzymgranulate kann beispielsweise etwa 0,1 bis 5 Gew.-%, vorzugsweise 0,12 bis etwa 2 Gew.-% betragen.

Enzyme werden nach dem Stand der Technik in erster Linie einer Reinigungsmittel-Zubereitung zugesetzt, insbesondere einem GeschirrPflegemittel zugesetzt, das für den Hauptspülgang bestimmt ist. Nachteil war dabei, dass das Wirkungsoptimum verwendeter Enzyme die Temperaturwahl beschränkte und auch Probleme bei der Stabilität der Enzyme im stark alkalischen Milieu auftraten. Mit den erfindungsgemäßen Wasch- oder Reinigungsmittel-Portionen ist es möglich, Enzyme in ein separates Kompartiment einzuführen und diese dann auch im Vorspülgang zu verwenden und damit den Vorspülgang zusätzlich zum Hauptspülgang für eine Enzymeinwirkung auf Verschmutzungen des Spülguts zu nutzen.

Besonders bevorzugt ist es, der für den Vorspülgang vorgesehenen waschaktiven Zubereitung oder Teilportion einer Reinigungsmittel- und/oder Pflegemittel-Portion Enzyme zuzusetzen und eine derartige Zubereitung dann - weiter bevorzugt - mit einem bereits bei niedriger Temperatur wasserlöslichen Material eines flexiblen, vorzugsweise elastischen, Hohlkörpers zu umfassen, um beispielsweise die enzymhaltige Zubereitung vor einem Wirkungsverlust durch Umgebungsbedingungen zu schützen. Die Enzyme sind weiter bevorzugt für den Einsatz unter den Bedingungen des Vorpflegegangs, also beispielsweise in kaltem Wasser, optimiert.

Vorteilhaft können die Reinigungsmittel dann sein, wenn die Enzymzubereitungen flüssig vorliegen, wie sie teilweise im Handel angeboten werden, weil dann eine schnelle Wirkung erwartet werden kann, die bereits im (relativ kurzen und in kaltem Wasser durchgeführten) Vorspülgang eintritt. Auch wenn- wie üblich- die Enzyme in fester Form eingesetzt werden und diese mit einer Hohlkörper-Umfassung aus einem wasserlöslichen Material versehen sind, das bereits in kaltem Wasser löslich ist, können die Enzyme bereits vor dem Hauptwaschgang bzw. Hauptreinigungsgang ihre Wirkung entfalten. Vorteil der Verwendung einer Umfassung aus wasserlöslichem Material, insbesondere aus kaltwasserlöslichem Material ist, dass das Enzym/die Enzyme in kaltem Wasser nach Auflösen der Umfassung schnell zur Wirkung kommt/kommen. Damit kann deren Wirkungszeit ausgedehnt werden, was dem Wasch- bzw. Spülergebnis zugute kommt.

Die Reinigungsmittel für das maschinelle Geschirrspülen können zum Schutze des Spülgutes oder der Maschine Korrosionsinhibitoren enthalten, wobei besonders Silberschutzmittel im Bereich des maschinellen Geschirrspülens eine besondere Bedeutung haben. Einsetzbar sind die bekannten Substanzen des Standes der Technik. Allgemein können vor allem Silberschutzmittel ausgewählt aus der Gruppe der Triazole, der Benzotriazole, der Bisbenzotriazole, der Aminotriazole, der Alkylaminotriazole und der Übergangsmetallsalze oder -komplexe eingesetzt werden. Besonders bevorzugt zu verwenden sind Benzotriazol und/oder Alkylaminotriazol. Man findet in Reinigerformulierungen darüber hinaus häufig aktivchlorhaltige Mittel, die das Korrodieren der Silberoberfläche deutlich vermindern können. In chlorfreien Reinigern werden besonders Sauerstoff- und stickstoffhaltige organische redoxaktive Verbindungen, wie zwei- und dreiwertige Phenole, z. B. Hydrochinon, Brenzkatechin, Hydroxyhydrochinon, Gallussäure, Phloroglucin, Pyrogallol bzw. Derivate dieser Verbindungsklassen. Auch salz- und komplexartige anorganische Verbindungen, wie Salze der Metalle Mn, Ti, Zr, Hf, V, Co und Ce finden häufig Verwendung. Bevorzugt sind hierbei die Übergangsmetallsalze, die ausgewählt sind aus der Gruppe der Mangan und/oder Cobaltsalze und/oder -komplexe, besonders bevorzugt der Cobalt(ammin)-Komplexe, der Cobalt(acetat)-Komplexe, der Cobalt-(Carbonyl)-Komplexe, der Chloride des Cobalts oder Mangans und des Mangansulfats. Ebenfalls können Zinkverbindungen zur Verhinderung der Korrosion am Spülgut eingesetzt werden.

Als Elektrolyte aus der Gruppe der anorganischen Salze kann eine breite Anzahl der verschiedensten Salze eingesetzt werden. Bevorzugte Kationen sind die Alkali- und Erdalkalimetalle, bevorzugte Anionen sind die Halogenide und Sulfate. Aus herstellungstechnischer Sicht ist der Einsatz von NaCl oder MgCl₂ in den erfindungsgemäßen Mitteln bevorzugt. Der Anteil an Elektrolyten in den erfindungsgemäßen Mitteln beträgt üblicherweise 0,5 bis 5 Gew.-%.

Nichtwäßrige Lösungsmittel, die in den Mitteln eingesetzt werden können, stammen beispielsweise aus der Gruppe ein- oder mehrwertigen Alkohole, Alkanolamine oder Glycolether, sofern sie im angegebenen Konzentrationsbereich mit Wasser mischbar sind. Vorzugsweise werden die Lösungsmittel ausgewählt aus Ethanol, n- oder i-Propanol, Butanolen, Glykol, Propan- oder Butandiol, Glycerin, Diglykol, Propyl- oder Butyldiglykol, Hexylenglycol, Ethylenglykolmethylether, Ethylenglykolethylether, Ethylenglykolpropylether, Ethylenglykolmono-n-butylether, Diethylenglykolmethylether, Diethylenglykolethylether, Propylenglykolmethyl-, -ethyl- oder -propylether, Dipropylenglykolmonomethyl-, oder -ethylether, Diisopropylenglykolmonomethyl-, oder -ethylether, Methoxy-, Ethoxy- oder Butoxytriglykol, 1-Butoxyethoxy-2-propanol, 3-Methyl-3-methoxybutanol, Propylenglykol-t-butylether sowie Mischungen dieser Lösungsmittel. Nichtwäßrige Lösungsmittel können in den erfindungsgemäßen Flüssigwaschmitteln in Mengen zwischen 0,5 und 10 Gew.-%, bevorzugt aber unter 5 Gew.-% und insbesondere unterhalb von 3 Gew.-% eingesetzt werden.

Um den pH-Wert der Mittel in den gewünschten Bereich zu bringen, kann der Einsatz von pH-Stellmitteln angezeigt sein.Einsetzbar sind hier sämtliche bekannten Säuren bzw. Laugen, sofern sich ihr Einsatz nicht aus anwendungstechnischen oder ökologischen Gründen bzw. aus Gründen des Verbraucherschutzes verbietet. Üblicherweise überschreitet die Menge dieser Stellmittel 5 Gew.-% der Gesamtformulierung nicht.

Um den ästhetischen Eindruck der Mittel zu verbessern, können sie mit geeigneten Farbstoffen eingefärbt werden. Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Mittel und gegen Licht sowie keine ausgeprägte Substantivität gegenüber Textilfasern, um diese nicht anzufärben.

Als Schauminhibitoren, die in den Mitteln eingesetzt werden können, kommen beispielsweise Seifen, Paraffine oder Silikonöle in Betracht, die gegebenenfalls auf Trägermaterialien aufgebracht sein können. Geeignete Antiredepositionsmittel, die auch als soil repellents bezeichnet werden, sind beispielsweise nichtionische Celluloseether wie Methylcellulose und Methylhydroxypropylcellulose mit einem Anteil an Methoxygruppen von 15 bis 30 Gew.-% und an Hydroxypropylgruppen von 1 bis 15 Gew.-%, jeweils bezogen auf den nichtionischen Celluloseether sowie die aus dem Stand der Technik bekannten Polymere der Phthalsäure und/oder Terephthalsäure bzw. von deren Derivaten, insbesondere Polymere aus Ethylenterephthalaten und/oder Polyethylenglycolterephthalaten oder anionisch und/oder nichtionisch modifizierten Derivaten von diesen. Insbesondere bevorzugt von diesen sind die sulfonierten Derivate der Phthalsäure- und Terephthalsäure-Polymere.

Optische Aufheller (sogenannte "Weißtöner") können den Mitteln zugesetzt werden, um Vergrauungen und Vergilbungen der behandelten Textilien zu beseitigen. Diese Stoffe ziehen auf die Faser auf und bewirken eine Aufhellung und vorgetäuschte Bleichwirkung, indem sie unsichtbare Ultraviolettstrahlung in sichtbares längerwelliges Licht umwandeln, wobei das aus dem Sonnenlicht absorbierte ultraviolette Licht als schwach bläuliche Fluoreszenz abgestrahlt wird und mit dem Gelbton der vergrauten bzw. vergilbten Wäsche reines Weiß ergibt. Geeignete Verbindungen stammen beispielsweise aus den Substanzklassen der 4,4'-Diamino-2,2'-stilbendisulfonsäuren (Flavonsäuren), 4,4'-Distyryl-biphenylen, Methylumbelliferone, Cumarine, Dihydrochinolinone, 1,3-Diarylpyrazoline, Naphthalsäureimide, Benzoxazol-, Benzisoxazol- und Benzimidazol-Systeme sowie der durch Heterocyclen substituierten Pyrenderivate. Die optischen Aufheller werden üblicherweise in Mengen zwischen 0,05 und 0,3 Gew.-%, bezogen auf das fertige Mittel, eingesetzt.

Vergrauungsinhibitoren haben die Aufgabe, den von der Faser abgelösten Schmutz in der Flotte suspendiert zu halten und so das Wiederaufziehen des Schmutzes zu verhindern. Hierzu sind wasserlösliche Kolloide meist organischer Natur geeignet, beispielsweise Leim, Gelatine, Salze von Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich lösliche Stärkepräparate und andere als die obengenannten Stärkeprodukte verwenden, z.B. abgebaute Stärke, Aldehydstärken usw. Auch Polyvinylpyrrolidon ist brauchbar. Bevorzugt werden jedoch Celluloseether wie Carboxymethylcellulose (Na-Salz), Methylcellulose, Hydroxyalkylcellulose und Mischether wie Methylhydroxyethylcellulose, Methylhydroxypropylcellulose, Methylcarboxymethylcellulose und deren Gemische in Mengen von 0,1 bis 5 Gew.-%, bezogen auf die Mittel, eingesetzt

Werden die Mittel als Mittel für das maschinelle Geschirrspülen konfektioniert, so können weitere Inhaltsstoffe eingesetzt werden. An maschinell gespültes Geschirr werden heute häufig höhere Anforderungen gestellt als an manuell gespültes Geschirr. So wird auch ein von Speiseresten völlig gereinigtes Geschirr dann als nicht einwandfrei bewertet, wenn es nach dem maschinellen Geschirrspülen noch weißliche, auf Wasserhärte oder anderen mineralischen Salzen beruhende Flecken aufweist, die mangels Netzmittel aus eingetrockneten Wassertropfen stammen. Um glasklares und fleckenloses Geschirr zu erhalten, setzt man daher heute mit Erfolg Klarspüler ein. Der Zusatz von Klarspüler am Ende des Spülprogramms sorgt dafür, daß das Wasser möglichst vollständig vom Spülgut abläuft, so daß die unterschiedlichen Oberflächen am Ende des Spülprogramms rückstandsfrei und makellos glänzend sind. Das maschinelle Reinigen von Geschirr in Haushaltsgeschirrspülmaschinen umfaßt üblicherweise einen Vorspülgang, einen Hauptspülgang und einen Klarspülgang, die von Zwischenspülgängen unterbrochen werden. Bei den meisten Maschinen ist der Vorspülgang für stark verschmutztes Geschirr zuschaltbar, wird aber nur in Ausnahmefällen vom Verbraucher gewählt, so daß in den meisten Maschinen ein Hauptspülgang, ein Zwischenspülgang mit reinem Wasser und ein Klarspülgang durchgeführt werden. Die Temperatur des Hauptspülgangs variiert dabei je nach Maschinentyp und Programmstufenwahl zwischen 40 und 65°C. Im Klarspülgang werden aus einem Dosiertank in der Maschine Klarspülmittel zugegeben, die üblicherweise als Hauptsbestandteil nichtionische Tenside enthalten. Solche Klarspüler liegen in flüssiger Form vor und sind im Stand der Technik breit beschrieben. lhre Aufgabe besteht vornehmlich darin, Kalkflecken und Beläge auf dem Geschirr zu verhindern.

Die Mittel können als "normale" Reiniger formuliert werden, welche zusammen mit handelsüblichen Ergänzungsmitteln (Klarspüler, Regeneriersalz) eingesetzt werden. Mit besonderem Vorteil kann aber mit den erfindungsgemäßen Produkten auf die zusätzliche Dosierung von Klarspülmitteln verzichtet werden. Diese sogenannten "2in1"-Produkte führen zu einer Vereinfachung der Handhabung und nehmen dem Verbraucher die Last der zusätzlichen Dosierung zweier unterschiedlicher Produkte (Reiniger und Klarspüler) ab.

Selbst beim Einsatz von "2in1"-Produkten sind zum Betrieb einer Haushaltsgeschirrspülmaschine in Zeitabständen zwei Dosiervorgänge erforderlich, da nach einer bestimmten Anzahl von Spülvorgängen das Regeneriersalz im Wasserenthärtungssystem der Maschine nachgefüllt werden muß. Diese Wasserenthärtungssysteme bestehen aus lonenaustauscherpolymeren, welche das der Maschine zulaufende Hartwasser enthärten und im Anschluß an das Spülprogramm durch eine Spülung mit Salzwasser regeneriert werden.

Es lassen sich aber auch erfindungsgemäße Produkte, welche als sogenannte "3in1"-Produkte die herkömmlichen Reiniger, Klarspüler und eine Salzersatzfunktion in sich vereinen, bereitstellen.

Im Rahmen der vorliegenden Erfindung können dem erfindungsgemäßen Mittel auch ungesättigte Carbonsäuren der Formel TVI als Monomer zugesetzt werden,

R¹(R²)C=C(R³)COOH (TVI),

in der R¹ bis R³ unabhängig voneinander für -H -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste wie vorstehend definiert oder für -COOH oder -COOR⁴ steht, wobei R⁴ ein gesättigter oder ungesättigter, geradkettigter oder verzweigter Kohlenwasserstoffrest mit 1 bis 12 Kohlenstoffatomen ist.

Unter den ungesättigten Carbonsäuren, die sich durch die Formel (TVI) beschreiben lassen, sind insbesondere Acrylsäure (R¹ = R² = R³ = H), Methacrylsäure (R¹ = R² = H; R³ = CH₃) und/oder Maleinsäure (R¹ = COOH; R² = R³ = H) bevorzugt.

Bei den Sulfonsäuregruppen-haltigen Monomeren sind solche der Formel (TVII) bevorzugt,

R⁵(R⁶)C=C(R⁷)-X-SO₃H (TVII),

in der R⁵ bis R⁷ unabhängig voneinander für -H -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste wie vorstehend definiert oder für -COOH oder -COOR⁴ steht, wobei R⁴ ein gesättigter oder ungesättigter, geradkettigter oder verzweigter Kohlenwasserstoffrest mit 1 bis 12 Kohlenstoffatomen ist, und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂- und -C(O)-NH-CH(CH₂CH₃)-.

Unter diesen Monomeren bevorzugt sind solche der Formeln (TVIIa), (TVIIb) und/oder (TVIIc),

H₂C=CH-X-SO₃H (TVIIa),

H₂C=C(CH₃)-X-SO₃H (TVIIb),

HO₃S-X-(R⁶)C=C(R⁷)-X-SO₃H (TVIIc),

in denen R⁶ und R⁷ unabhängig voneinander ausgewählt sind aus -H, -CH₃,CH₂CH₃, -CH₂CH₂CH₃, -CH(CH₃)₂ und X für eine optional vorhandene Spacergruppe steht, die ausgewählt ist aus -(CH₂)ₙ- mit n = 0 bis 4, -COO-(CH₂)ₖ-mit k = 1 bis 6, -C(O)-NH-C(CH₃)₂- und -C(O)-NH-CH(CH₂CH₃)-.

Besonders bevorzugte Sulfonsäuregruppen-haltige Monomere sind dabei 1-Acrylamido-1-propansulfonsäure (X = -C(O)NH-CH(CH₂CH₃) in Formel (TVIIa), 2-Acrylamido-2-propansulfonsäure (X = -C(O)NH-C(CH₃)₂ in Formel (TVIIa), 2-Acrylamido-2-methyl-1-propansulfonsäure (X = -C(O)NH-CH(CH₃)CH₂- in Formel (TVIIa), 2-Methacrylamido-2-methyl-1-propansulfonsäure (X = -C(O)NH-CH(CH₃)CH₂- in Formel (TVIIb), 3-Methacrylamido-2-hydroxy-propansulfonsäure (X = -C(O)NH-CH₂CH(OH)CH₂- in Formel VIIb), Allylsulfonsäure (X = CH₂ in Formel (TVIIa), Methallylsulfonsäure (X = CH₂ in Formel (TVIIb), Allyloxybenzolsulfonsäure (X = -CH₂-O-C₆H₄- in Formel (TXVIIa), Methallyloxybenzolsulfonsäure (X = -CH₂-O-C₆H₄- in Formel Vllb), 2-Hydroxy-3-(2-propenyloxy)propansulfonsäure, 2-Methyl-2-propenl-sulfonsäure (X = CH₂ in Formel (TVIIb), Styrolsulfonsäure (X = C₆H₄ in Formel (TVIIa)), Vinylsulfonsäure (X nicht vorhanden in Formel (TVIIa)), 3-Sulfopropylacrylat (X = -C(O)NH-CH₂CH₂CH₂- in Formel (TVIIa)), 3-Sulfopropylmethacrylat (X = -C(O)NH-CH₂CH₂CH₂- in Formel (TVIIb)), Sulfomethacrylamid (X = -C(O)NH- in Formel (TVIIb)), Sulfomethylmethacrylamid (X = -C(O)NH-CH₂- in Formel (TVIIb)) sowie wasserlösliche Salze der genannten Säuren.

Als weitere ionische oder nichtionogene Monomere kommen insbesondere ethylenisch ungesättigte Verbindungen in Betracht. Vorzugsweise beträgt der Gehalt der erfindungsgemäß eingesetzten Polymere an Monomeren der Grupp iii) weniger als 20 Gew.-%, bezogen auf das Polymer. Besonders bevorzugt zu verwendende Polymere bestehen lediglich aus Monomeren der Gruppen i) und ii).

Zusammenfassend sind Copolymere aus
i) ungesättigten Carbonsäuren der Formel (TVI).

   R¹(R²)C=C(R³)COOH (TVI),

   in der R¹ bis R³ unabhängig voneinander für -H -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste wie vorstehend definiert oder für -COOH oder -COOR⁴ steht, wobei R⁴ ein gesättigter oder ungesättigter, geradkettigter oder verzweigter Kohlenwasserstoffrest mit 1 bis 12 Kohlenstoffatomen ist,
ii) Sulfonsäuregruppen-haltigen Monomeren der Formel (TVII)

   R⁵(R⁶)C=C(R⁷)-X-SO₃H (TVII),

   in der R⁵ bis R⁷ unabhängig voneinander für -H -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste wie vorstehend definiert oder für -COOH oder -COOR⁴ steht, wobei R⁴ ein gesättigter oder ungesättigter, geradkettigter oder verzweigter Kohlenwasserstoffrest mit 1 bis 12 Kohlenstoffatomen ist, und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂- und -C(O)-NH-CH(CH₂CH₃)-
iii) gegebenenfalls weiteren ionischen oder nichtionogenen Monomeren besonders bevorzugt.

Besonders bevorzugte Copolymere bestehen aus
i) einer oder mehrerer ungesättigter Carbonsäuren aus der Gruppe Acrylsäure, Methacrylsäure und/oder Maleinsäure
ii) einem oder mehreren Sulfonsäuregruppen-haltigen Monomeren der Formeln (TVIIa), (TVIIb) und/oder (TVIIc):

   H₂C=CH-X-SO₃H (TVIIa),

   H₂C=C(CH₃)-X-SO₃H (TVIIb),

   HO₃S-X-(R⁶)C=C(R⁷)-X-SO₃H (TVIIc),

   in der R⁶ und R⁷ unabhängig voneinander ausgewählt sind aus -H, -CH₃, -CH₂CH₃, -CH₂CH₂CH₃, -CH(CH₃)₂ und X für eine optional vorhandene Spacergruppe steht, die ausgewählt ist aus -(CH₂)ₙ- mit n = 0 bis 4, -COO-(CH₂)ₖ mit k = 1 bis 6, -C(O)-NH-C(CH₃)₂- und -C(O)-NH-CH(CH₂CH₃)-
iii) gegebenenfalls weiteren ionischen oder nichtionogenen Monomeren.

Die erfindungsgemäß in den Mitteln anthaltenen Copolymere können die Monomere aus den Gruppen i) und ii) sowie gegebenenfalls iii) in variierenden Mengen enthalten, wobei sämtliche Vertreter aus der Gruppe i) mit sämtlichen Vertretern aus der Gruppe ii) und sämtlichen Vertretern aus der Gruppe iii) kombiniert werden können. Besonders bevorzugte Polymere weisen bestimmte Struktureinheiten auf, die nachfolgend beschrieben werden.

So sind beispielsweise erfindungsgemäße Mittel bevorzugt, die dadurch gekennzeichnet sind, daß sie ein oder mehrere Copolymere enthalten, die Struktureinheiten der Formel (TVIII)

-[CH₂-CHCOOH]ₘ-[CH₂-CHC(O)-Y-SO₃H]ₚ- (TVIII),

enthalten, in der m und p jeweils für eine ganze natürliche Zahl zwischen 1 und 2000 sowie Y für eine Spacergruppe steht, die ausgewählt ist aus substituierten oder unsubstituierten aliphatischen, aromatischen oder araliphatischen Kohlenwasserstoffresten mit 1 bis 24 Kohlenstoffatomen, wobei Spacergruppen, in denen Y für -O-(CH₂)ₙ- mit n = 0 bis 4, für -O-(C₆H₄)-, für -NH-C(CH₃)₂- oder -NH-CH(CH₂CH₃)- steht, bevorzugt sind.

Diese Polymere werden durch Copolymerisation von Acrylsäure mit einem Sulfonsäuregruppen-haltigen Acrylsäurederivat hergestellt. Copolymerisiert man das Sulfonsäuregruppen-haltige Acrylsäurederivat mit Methacrylsäure, gelangt man zu einem anderen Polymer, dessen Einsatz in den erfindungsgemäßen Mitteln ebenfalls bevorzugt und dadurch gekennzeichnet ist, daß die Mittel ein oder mehrere Copolymere enthalten, die Struktureinheiten der Formel (TIX)

-[CH₂-C(CH₃)COOH]ₘ-[CH₂-CHC(O)-Y-SO₃H]ₚ- (TIX),

enthalten, in der m und p jeweils für eine ganze natürliche Zahl zwischen 1 und 2000 sowie Y für eine Spacergruppe steht, die ausgewählt ist aus substituierten oder unsubstituierten aliphatischen, aromatischen oder araliphatischen Kohlenwasserstoffresten mit 1 bis 24 Kohlenstoffatomen, wobei Spacergruppen, in denen Y für -O-(CH₂)ₙ- mit n = 0 bis 4, für -O-(C₆H₄)-, für -NH-C(CH₃)₂- oder -NH-CH(CH₂CH₃)- steht, bevorzugt sind.

Völlig analog lassen sich Acrylsäure und/oder Methacrylsäure auch mit Sulfonsäuregruppen-haltigen Methacrylsäurederivaten copolymerisieren, wodurch die Struktureinheiten im Molekül verändert werden. So sind erfindungsgemäße Mittel, die ein oder mehrere Copolymere enthalten, welche Struktureinheiten der Formel (TX)

-[CH₂-CHCOOH]ₘ-[CH₂-C(CH₃)C(O)-Y-SO₃H]ₚ- (TX),

enthalten, in der m und p jeweils für eine ganze natürliche Zahl zwischen 1 und 2000 sowie Y für eine Spacergruppe steht, die ausgewählt ist aus substituierten oder unsubstituierten aliphatischen, aromatischen oder araliphatischen Kohlenwasserstoffresten mit 1 bis 24 Kohlenstoffatomen, wobei Spacergruppen, in denen Y für -O-(CH₂)ₙ- mit n = 0 bis 4, für -O-(C₆H₄)-, für -NH-C(CH₃)₂- oder -NH-CH(CH₂CH₃)- steht, bevorzugt sind, ebenfalls eine bevorzugte Ausführungsform der vorliegenden Erfindung, genau wie auch Mittel bevorzugt sind, die dadurch gekennzeichnet sind, daß sie ein oder mehrere Copolymere enthalten, die Struktureinheiten der Formel (TXI)

-[CH₂-C(CH₃)COOH]ₘ-[CH₂-C(CH₃)C(O)-Y-SO₃H]ₚ- (TXI),

enthalten, in der m und p jeweils für eine ganze natürliche Zahl zwischen 1 und 2000 sowie Y für eine Spacergruppe steht, die ausgewählt ist aus substituierten oder unsubstituierten aliphatischen, aromatischen oder araliphatischen Kohlenwasserstoffresten mit 1 bis 24 Kohlenstoffatomen, wobei Spacergruppen, in denen Y für -O-(CH₂)ₙ- mit n = 0 bis 4, für -O-(C₆H₄)-, für -NH-C(CH₃)₂- oder -NH-CH(CH₂CH₃)- steht, bevorzugt sind.

Anstelle von Acrylsäure und/oder Methacrylsäure bzw. in Ergänzung hierzu kann auch Maleinsäure als besonders bevorzugtes Monomer aus der Gruppe i) eingesetzt werden. Man gelangt auf diese Weise zu erfindungsgemäß bevorzugten Mitteln, die dadurch gekennzeichnet sind, daß sie ein oder mehrere Copolymere enthalten, die Struktureinheiten der Formel (TXI)

-[HOOCCH-CHCOOH]ₘ-[CH₂-CHC(O)-Y-SO₃H]ₚ- (TXI),

enthalten, in der m und p jeweils für eine ganze natürliche Zahl zwischen 1 und 2000 sowie Y für eine Spacergruppe steht, die ausgewählt ist aus substituierten oder unsubstituierten aliphatischen, aromatischen oder araliphatischen Kohlenwasserstoffresten mit 1 bis 24 Kohlenstoffatomen, wobei Spacergruppen, in denen Y für -O-(CH₂)ₙ- mit n = 0 bis 4, für -O-(C₆H₄)-, für -NH-C(CH₃)₂- oder -NH-CH(CH₂CH₃)- steht, bevorzugt sind und zu Mitteln, welche dadurch gekennzeichnet sind, daß sie ein oder mehrere Copolymere enthalten, die Struktureinheiten der Formel (XII)

-[HOOCCH-CHCOOH]ₘ-[CH₂-C(CH₃)C(O)O-Y-SO₃H]ₚ- (TXII),

enthalten, in der m und p jeweils für eine ganze natürliche Zahl zwischen 1 und 2000 sowie Y für eine Spacergruppe steht, die ausgewählt ist aus substituierten oder unsubstituierten aliphatischen, aromatischen oder araliphatischen Kohlenwasserstoffresten mit 1 bis 24 Kohlenstoffatomen, wobei Spacergruppen, in denen Y für -O-(CH₂)ₙ- mit n = 0 bis 4, für -O-(C₆H₄)-, für -NH-C(CH₃)₂- oder -NH-CH(CH₂CH₃)- steht, bevorzugt sind.

Zusammenfassend sind maschinelle Geschirrspülmittel bevorzugt, die als Inhaltsstoff b) ein oder mehrere Copolymere enthält, die Struktureinheiten der Formeln (TVII) und/oder (TVIII) und/oder (TIX) und/oder (TX) und/oder (TXI) und/oder (TXII)

-[CH₂-CHCOOH]ₘ-[CH₂-CHC(O)-Y-SO₃H]ₚ- (TVII),

-[CH₂-C(CH₃)COOH]ₘ-[CH₂-CHC(O)-Y-SO₃H]ₚ- (TVIII),

-[CH₂-CHCOOH]ₘ-[CH₂-C(CH₃)C(O)-Y-SO₃H]ₚ- (TIX),

-[CH₂-C(CH₃)COOH]ₘ-[CH₂-C(CH₃)C(O)-Y-SO₃H]ₚ- (TX),

-[HOOCCH-CHCOOH]ₘ-[CH₂-CHC(O)-Y-SO₃H]ₚ- (TXI),

-[HOOCCH-CHCOOH]ₘ-[CH₂-C(CH₃)C(O)O-Y-SO₃H]ₚ- (TXII),

enthalten, in denen m und p jeweils für eine ganze natürliche Zahl zwischen 1 und 2000 sowie Y für eine Spacergruppe steht, die ausgewählt ist aus substituierten oder unsubstituierten aliphatischen, aromatischen oder araliphatischen Kohlenwasserstoffresten mit 1 bis 24 Kohlenstoffatomen, wobei Spacergruppen, in denen Y für -O-(CH₂)ₙ- mit n = 0 bis 4, für -O-(C₆H₄)-, für -NH-C(CH₃)₂- oder -NH-CH(CH₂CH₃)- steht, bevorzugt sind.

In den Polymeren können die Sulfonsäuregruppen ganz oder teilweise in neutralisierter Form vorliegen, d.h. daß das acide Wasserstoffatom der Sulfonsäuregruppe in einigen oder allen Sulfonsäuregruppen gegen Metallionen, vorzugsweise Alkalimetallionen und insbesondere gegen Natriumionen, ausgetauscht sein kann. Entsprechende Mittel, die dadurch gekennzeichnet sind, daß die Sulfonsäuregruppen im Copolymer teil- oder vollneutralisiert vorliegen, sind erfindungsgemäß bevorzugt.

Die Monomerenverteilung der in den erfindungsgemäßen Mitteln eingesetzten Copolymeren beträgt bei Copolymeren, die nur Monomere aus den Gruppen i) und ii) enthalten, vorzugsweise jeweils 5 bis 95 Gew.-% i) bzw. ii), besonders bevorzugt 50 bis 90 Gew.-% Monomer aus der Gruppe i) und 10 bis 50 Gew.-% Monomer aus der Gruppe ii), jeweils bezogen auf das Polymer.

Bei Terpolymeren sind solche besonders bevorzugt, die 20 bis 85 Gew.-% Monomer aus der Gruppe i), 10 bis 60 Gew.-% Monomer aus der Gruppe ii) sowie 5 bis 30 Gew.-% Monomer aus der Gruppe iii) enthalten.

Die Molmasse der in den erfindungsgemäßen Mitteln eingesetzten Polymere kann variiert werden, um die Eigenschaften der Polymere dem gewünschten Verwendungszweck anzupassen. Bevorzugte maschinelle Geschirrspülmittel sind dadurch gekennzeichnet, daß die Copolymere Molmassen von 2000 bis 200.000 gmol⁻¹, vorzugsweise von 4000 bis 25.000 gmol⁻¹ und insbesondere von 5000 bis 15.000 gmol⁻¹ aufweisen.

Der Gehalt an einem oder mehreren Copolymeren in den Mitteln kann je nach Anwendungszweck und gewünschter Produktleistung varieren, wobei bevorzugte erfindungsgemäße maschinelle Geschirrspülmittel dadurch gekennzeichnet sind, daß sie das bzw. die Copolymer(e) in Mengen von 0,25 bis 50 Gew.-%, vorzugsweise von 0,5 bis 35 Gew.-%, besonders bevorzugt von 0,75 bis 20 Gew.-% und insbesondere von 1 bis 15 Gew.-% enthalten.

Wie bereits weiter oben erwähnt, werden in den Mitteln besonders bevorzugt sowohl Polyacrylate als auch die vorstehend beschriebenen Copolymere aus ungesättigten Carbonsäuren, Sulfonsäuregruppen-haltigen Monomeren sowie gegebenenfalls weiteren ionischen oder nichtionogenen Monomeren eingesetzt. Die Polyacrylate wurden dabei weiter oben ausführlich beschrieben. Besonders bevorzugt sind Kombinationen aus den vorstehend beschriebenen Sulfonsäuregruppen-haltigen Copolymeren mit Polyacrylaten niedriger Molmasse, beispielsweise im Bereich zwischen 1000 und 4000 Dalton. Solche Polyacrylate sind kommerziell unter dem Handelsnamen Sokalan^{®} PA15 bzw. Sokalan^{®} PA25 (BASF) erhältlich.

Die Mittel können auch als Weichspüler oder Waschzusatzmittel konfektioniert werden. Je nach gewünschtem Verwendungszweck können weitere Inhaltsstoffe eingesetzt werden. Weichspülerzusammensetzungen für die Spülbadavivage sind im Stand der Technik breit beschrieben. Üblicherweise enthalten diese Zusammensetzungen als Aktivsubstanz eine kationische quartäre Ammoniumverbindung, die in Wasser dispergiert wird. Je nach Gehalt der fertigen Weichmacherzusammensetzung an Aktivsubstanz spricht man von verdünnten, anwendungsfertigen Produkten (Aktivsubstanzgehalte unter 7 Gew.-%) oder sogenannten Konzentraten (Aktivsubstanzgehalt über 7 Gew.-%). Wegen des geringeren Volumens und den damit gleichzeitig verringerten Verpackungs- und Transportkosten besitzen die Textilweichmacherkonzentrate Vorteile aus ökologischer Sicht und haben sich im Markt mehr und mehr durchgesetzt. Aufgrund der Einarbeitung von kationischen Verbindungen, die nur eine geringe Wasserlöslichkeit aufweisen, liegen übliche Weichspülerzusammensetzungen in Form von Dispersionen vor, besitzen ein milchig-trübes Aussehen und sind nicht durchscheinend. Aus Gründen der Produktästhetik kann es aber auch gewünscht sein, dem Verbraucher durchscheinende, klare Weichspüler zur Verfügung zu stellen, die sich optisch von den bekannten Produkten abheben.

Als textilweichmachende Aktivsubstanz enthalten Weichspüler vorzugsweise kationische Tenside, die bereits weiter oben ausführlich beschrieben wurden (Formeln TXII, TXIII und TXIV). Besonders bevorzugt enthalten diese erfindungsgemäße Mittel sogenannte Esterquats. Während es eine Vielzahl möglicher Verbindungen aus dieser Substanzklasse gibt, werden erfindungsgemäß mit besonderem Vorzug Esterquats eingesetzt, die sich durch Umsetzung von Trialkanolaminen mit einer Mischung aus Fettsäuren und Dicarbonsäuren, gegebenenfalls nachfolgende Alkoxylierung des Reaktionsproduktes und Quaternierung in an sich bekannter Weise herstellen lassen, wie es in der DE 195 39 846 beschrieben ist.

Die auf diese Weise hergestellten Esterquats eignen sich in hervorragender Weise zur Herstellung erfindungsgemäßer Portionen, die als Weichspüler eingesetzt werden können. Da je nach Wahl des Trialkanolamins, der Fettsäuren und der Dicarbonsäuren sowie des Quaternierungsmittels eine Vielzahl geeigneter Produkte hergestellt und in den erfindungsgemäßen Mitteln eingesetzt werden kann, ist eine Beschreibung der erfindungsgemäß vorzugsweise einzusetzenden Esterquats über ihren Herstellungsweg präziser als die Angabe einer allgemeinen Formel.

Die genannten Komponenten, die miteinander zu den vorzugsweise einzusetzenden Esterquats reagieren, können in variierenden Mengenverhältnissen zueinander eingesetzt werden. Im Rahmen der vorliegenden Erfindung sind Weichspüler bevorzugt, in denen ein Umsetzungsprodukt von Trialkanolaminen mit einer Mischung aus Fettsäuren und Dicarbonsäuren im molaren Verhältnis 1:10 bis 10:1, vorzugsweise 1:5 bis 5:1, das gegebenenfalls alkoxyliert und anschließend in an sich bekannter Weise quaterniert wurde, in Mengen von 2 bis 60, vorzugsweise 3 bis 35 und insbesondere 5 bis 30 Gew.-% enthalten ist. Besonders bevorzugt ist dabei die Verwendung von Triethanolamin, so daß weitere bevorzugte Weichspüler der vorliegenden Erfindung ein Umsetzungsprodukt von Triethanolamin mit einer Mischung aus Fettsäuren und Dicarbonsäuren im molaren Verhältnis 1:10 bis 10:1, vorzugsweise 1:5 bis 5:1, das gegebenenfalls alkoxyliert und anschließend in an sich bekannter Weise quaterniert wurde, in Mengen von 2 bis 60, vorzugsweise 3 bis 35 und insbesondere 5 bis 30 Gew.-% enthalten.

Als Fettsäuren können im Reaktionsgemisch zur Herstellung der Esterquats sämtliche aus pflanzlichen oder tierischen Ölen und Fetten gewonnenen Säuren verwendet werden. Dabei kann im Rekationsgemisch als Fettsäure durchaus auch eine bei Raumtemperatur nicht-feste, d.h. pastöse bis flüssige, Fettsäure eingesetzt werden.

Die Fettsäuren können unabhängig von ihrem Aggregatzustand gesättigt oder einbis mehrfach ungesättigt sein. Selbstverständlich können nicht nur "reine" Fettsäuren eingesetzt werden, sondern auch die bei der Spaltung aus Fetten und Ölen gewonnenen technischen Fettsäuregemische, wobei diese Gemische aus ökonomischer Sicht wiederum deutlich bevorzugt sind.

So lassen sich in den Reaktionsmischungen zur Herstellung der Esterquats für die klaren wäßrigen Weichspüler beispielsweise einzelne Spezies oder Gemische folgender Säuren einsetzen: Caprylsäure, Pelargonsäure, Caprinsäure, Laurinsäure, Myristinsäure, Palmitinsäure, Stearinsäure, Octadecan-12-ol-säure, Arachinsäure, Behensäure, Lignocerinsäure, Cerotinsäure, Melissinsäure, 10-Undecensäure, Petroselinsäure, Petroselaidinsäure, Ölsäure, Elaidinsäure, Ricinolsäure, Linolaidinsäure, α- und β-Eläosterainsäure, Gadoleinsäure, Erucasäure, Brassidinsäure. Selbstverständlich sind auch die Fettsäuren mit ungerader Anzahl von C-Atomen einsetzbar, beispielsweise Undecansäure, Tridecansäure, Pentadecansäure, Heptadecansäure, Nonadecansäure, Heneicosansäure, Tricosansäure, Pentacosansäure, Heptacosansäure.

Die Verwendung von Fettsäuren der Formel XIII im Reaktionsgemisch zur Herstellung der Esterquats bevorzugt, so daß bevorzugte Weichspüler ein Umsetzungsprodukt von Trialkanolaminen mit einer Mischung aus Fettsäuren der Formel TXIII,

R¹-CO-OH (TXIII)

in der R1-CO- für einen aliphatischen, linearen oder verzweigten Acylrest mit 6 bis 22 Kohlenstoffatomen und 0 und/oder 1, 2 oder 3 Doppelbindungen steht und Dicarbonsäuren im molaren Verhältnis 1:10 bis 10:1, vorzugsweise 1:5 bis 5:1, das gegebenenfalls alkoxyliert und anschließend in an sich bekannter Weise quaterniert wurde, in Mengen von 2 bis 60, vorzugsweise 3 bis 35 und insbesondere 5 bis 30 Gew.-% in den Mitteln enthalten.

Als Dicarbonsäuren, die sich zur Herstellung der in den einzusetzenden Esterquats eignen, kommen vor allem gesättigte oder ein- bzw. mehrfach ungesättigte α,ωDicarbonsäuren in Betracht. Beispielhaft seien hier die gesättigten Spezies Oxalsäure, Malonsäure, Bernsteinsäure, Glutarsäure, Adipinsäure, Pimelinsäure, Korksäure, Azelainsäure, Sebacinsäure, Undecan- und Dodecansäure, Brassylsäure, Tetra- und Pentadecansäure, Thapisäure sowie Hepta-, Octa- und Nonadecansäure, Eicosan- und Heneicosansäure sowie Phellogensäure genannt. Vorzugsweise im Reaktionsgemisch eingesetzt werden dabei Dicarbonsäuren, die der allgemeinen Formel XIII folgen, so daß erfindungsgemäße Mittel bevorzugt sind, die ein Umsetzungsprodukt von Trialkanolaminen mit einer Mischung aus Fettsäuren und Dicarbonsäuren der Formel TXIV,

HO-OC-[X]-CO-OH (TXIV)

in der X für eine gegebenenfalls hydroxysubstituierte Alkylengruppe mit 1 bis 10 Kohlenstoffatomen steht, im molaren Verhältnis 1:10 bis 10:1, vorzugsweise 1:5 bis 5:1, das gegebenenfalls alkoxyliert und anschließend in an sich bekannter Weise quaterniert wurde, in Mengen von 2 bis 60, vorzugsweise 3 bis 35 und insbesondere 5 bis 30 Gew.-% in den Mitteln enthalten.

Unter der Vielzahl der herstellbaren und einsetzbaren Esterquats haben sich wiederum solche besonders bewährt, in denen das Alkanolamin Treithanolamin und die Dicarbonsäure Adipinsäure ist. Somit sind im Rahmen der vorliegenden Erfindung Mittel besonders bevorzugt, die ein Umsetzungsprodukt von Triethanolamin mit einer Mischung aus Fettsäuren und Adipinsäure im molaren Verhältnis 1:5 bis 5:1, vorzugsweise 1:3 bis 3:1, das anschließend in an sich bekannter Weise quaterniert wurde, in Mengen von 2 bis 60, vorzugsweise 3 bis 35 und insbesondere 5 bis 30 Gew.-% in den Mitteln enthalten

Die Mittel können - und_abhängig davon, ob sie als Textilwaschmittel, Waschhilfsmittel oder Weichspüler formuliert werden - auch mit weiteren Zusatznutzen ausgestattet werden. Hier sind beispielsweise farbübertragungsinhibierende Zusammensetzungen, Mittel mit "Anti-Grau-Formel", Mittel mit Bügelerleichterung, Mittel mit besonderer Duftfreisetzung, Mittel mit verbesserter Schmutzablösung bzw. Verhinderung von Wiederanschmutzung, antibakterielle Mittel, UV-Schutzmittel, farbauffrischende Mittel usw. formulierbar. Einige Beispiele werden nachstehend erläutert:

Da textile Flächengebilde, insbesondere aus Reyon, Zellwolle, Baumwolle und deren Mischungen, zum Knittern eigen können, weil die Einzelfasern gegen Durchbiegen, Knicken. Pressen und Quetschen quer zur Faserrichtung empfindlich sind, können die erfindungsgemäßen Mittel synthetische Knitterschutzmittel enthalten. Hierzu zählen beispielsweise synthetische Produkte auf der Basis von Fettsäuren, Fettsäureestern. Fettsäureamiden, -alkylolestern, -alkylolamiden oder Fettalkoholen, die meist mit Ethylenoxid umgesetzt sind, oder Produkte auf der Basis von Lecithin oder modifizierter Phosphorsäureester.

Zur Bekämpfung von Mikroorganismen können die erfindungsgemäßen Mittel antimikrobielle Wirkstoffe enthalten. Hierbei unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppen sind beispielsweise Benzalkoniumchloride, Alkylarlylsulfonate, Halogenphenole und Phenolmercuriacetat, wobei bei den erfindungemäßen Mitteln auch gänzlich auf diese Verbindungen verzichtet werden kann.

Um unerwünschte, durch Sauerstoffeinwirkung und andere oxidative Prozesse verursachte Veränderungen an den Mitteln und/oder den behandelten Textilien zu verhindern, können die Mittel Antioxidantien enthalten. Zu dieser Verbindungsklasse gehören beispielsweise substituierte Phenole, Hydrochinone, Brenzcatechnine und aromatische Amine sowie organische Sulfide, Polysulfide, Dithiocarbamate, Phosphite und Phosphonate.

Ein erhöhter Tragekomfort kann aus der zusätzlichen Verwendung von Antistatika resultieren, die den erfindungsgemäßen Mitteln zusätzlich beigefügt werden. Antistatika vergrößern die Oberflächenleitfähigkeit und ermöglichen damit ein verbessertes Abfließen gebildeter Ladungen. Äußere Antistatika sind in der Regel Substanzen mit wenigstens einem hydrophilen Molekülliganden und geben auf den Oberflächen einen mehr oder minder hygroskopischen Film. Diese zumeist grenzflächenaktiven Antistatika lassen sich in stickstoffhaltige (Amine, Amide, quartäre Ammoniumverbindungen), phosphorhaltige (Phosphorsäureester) und schwefelhaltige (Alkylsulfonate, Alkylsulfate) Antistatika unterteilen. Lauryl- (bzw.

Stearyl-) dimethylbenzylammoniumchloride eignen sich als Antistatika für Textilien bzw. als Zusatz zu Waschmitteln, wobei zusätzlich ein Avivageeffekt erzielt wird.

Zur Verbesserung des Wasserabsorptionsvermögens, der Wiederbenetzbarkeit der behandelten Textilien und zur Erleichterung des Bügelns der behandelten Textilien können in den Mitteln beispielsweise Silikonderivate eingesetzt werden. Diese verbessern zusätzlich das Ausspülverhalten der erfindungsgemäßen Mittel durch ihre schauminhibierenden Eigenschaften. Bevorzugte Silikonderivate sind beispielsweise Polydialkyl- oder Alkylarylsiloxane, bei denen die Alkylgruppen ein bis fünf C-Atome aufweisen und ganz oder teilweise fluoriert sind. Bevorzugte Silikone sind Polydimethylsiloxane, die gegebenenfalls derivatisiert sein können und dann aminofunktionell oder quaterniert sind bzw. Si-OH-, Si-H- und/oder Si-Cl-Bindungen aufweisen. Die Viskositäten der bevorzugten Silikone liegen bei 25°C im Bereich zwischen 100 und 100.000 Centistokes, wobei die Silikone in Mengen zwischen 0,2 und 5 Gew.%, bezogen auf das gesamte Mittel eingesetzt werden können.

Schließlich können die Mittel auch UV-Absorber enthalten, die auf die behandelten Textilien aufziehen und die Lichtbeständigkeit der Fasern verbessern. Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, in 3-Stellung Phenylsubstituierte Acrylate (Zimtsäurederivate), gegebenenfalls mit Cyanogruppen in 2-Stellung, Salicylate, organische Ni-Komplexe sowie Naturstoffe wie Umbelliferon und die körpereigene Urocansäure geeignet.

Weitere denkbare und in speziellen Ausführungsformen bevorzugte Additive sind Tenside, die insbesondere die Löslichkeit der wasserlöslichen Wandung des flexiblen, vorzugsweise elastischen, Hohlkörpers oder der Kompartimentierungs-Einrichtung beeinflussen können, aber auch deren Benetzbarkeit und die Schaumbildung beim Auflösen steuern können, sowie Schauminhibitoren, aber auch Bitterstoffe, die ein versehentliches Verschlucken solcher Hohlkörper oder Teile solcher Hohlkörper durch Kinder verhindern können.

Duftstoffe werden den Waschmittel-, Reinigungsmittel- und/oder Pflegemitteln zugesetzt, um den ästhetischen Gesamteindruck der Produkte zu verbessern und dem Verbraucher neben der technischen Leistung (Weichspülergebnis) ein sensorisch typisches und unverwechselbares Produkt zur Verfügung zu stellen. Als Parfümöle oder Duftstoffe können einzelne Riechstoff-Verbindungen verwendet werden, beispielsweise die synthetischen Produkte vom Typ der Ester, Ether, Aldehyde, Ketone, Alkohole und Kohlenwasserstoffe. Riechstoff-Verbindungen vom Typ der Ester sind beispielsweise Benzylacetat, Phenoxyethylisobutyrat, p-t-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether. Zu den Aldehyden zählen z. B. lineare Alkanale mit 8 bis 18 C-Atomen, Citral, Citronellal, Citronellyloxyacetaldehyd, Cyclamenaldehyd, Hydroxycitronellal, Lileal und Bourgeonal.

Zu den Ketonen zählen die lonone, α-Isomethylionon, und Methylcedrylketon. Zu den Alkoholen zählen Anethol, Citronellol, Eugenol, Geraniol, Linalool, Phenylethylalkohol und Terpineol. Zu den Kohlenwasserstoffen zählen hauptsächlich Terpene wie Limonen und Pinen. Bevorzugt werden Mischungen verschie-dener Riechstoffe verwendet, die so aufeinander abgestimmt sind, dass sie gemeinsam eine ansprechende Duftnote erzeugen. Solche Parfümöle können auch natürliche Riechstoff-Gemische enthalten, wie sie aus pflanzlichen Quellen zugänglich sind. Beispiele sind Pine-, Citrus-, Jasmin-, Patchouli-, Rosen- oder Ylang-Ylang-Öl. Ebenfalls geeignet sind Muskatöl, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl.

Üblicherweise liegt der Gehalt an Duftstoffen im Bereich bis zu 2 Gew.-% der gesamten Waschmittel-, Reinigungsmittel- oder Pflegemittel-Portion.

Die Duftstoffe können direkt in die waschaktive(n), reinigungsaktive(n) oder pflegeaktive(n) Zubereitung(en) eingearbeitet werden; es kann aber auch vorteilhaft sein, die Duftstoffe auf Träger aufzubringen, die die Haftung des Parfüms auf der Wäsche verstärken und durch eine langsamere Duftfreisetzung für lang-anhaltenden Duft von Textilien sorgen. Als solche Trägermaterialien haben sich beispielsweise Cyclodextrine bewährt. Dabei können die Cyclodextrin-Parfüm-Komplexe zusätzlich noch mit weiteren Hilfsstoffen beschichtet werden.

Die trägerfixiertern Bleichkatalysatoren werden zum Bleichen von Farben, insbesondere von Haarfarben und/oder Farbanschmutzungen auf harten und/oder weichen Oberflächen, vorzugsweise zum Bleichen von farbigen Anschmutzungen beim Waschen von Textilien, verwendet.

Die trägerfixiertern Bleichkatalysatoren lassen sich in flüssigen, festen und/oder gelförmigen Mitteln, insbesondere Reinigungsmitteln, verwenden, vorzugsweise zum Bleichen von harten Oberflächen, wie Geschirr und/oder zum Bleichen von farbigen Anschmutzungen auf oder in Oberflächen.

Der trägerfixierter, erfindungsgemäße verwendete Bleichkatalysator, insbesondere die Mittel enthaltend den trägerfixierten Bleichkatalysator, können in eine Dosierkammer, vorzugsweise Klarspülkammer oder Waschmaschinenspülkammer, eingebracht werden. Die trägerfixierten Bleichkatalysator(en) enthaltenden Mittel können in flüssiger, fester, und/oder gelförmiger Form vorliegen, die dabei auch portioniert, d.h. schon für die übliche Anwendungsmenge separat vorportioniert, vorliegen können.

Besonders bevorzugt kann es sein, die Form der trägerfixierte Bleichkatalysatoren so zu gestalten, dass sie einfarch wiederverwendbar sind.

Als Träger sind dann insbesondere Formkörper. Gewebe und/oder Partikel auf denen die Bleichkatalysatoren fixiert sind geeignet. Bevorzugt können die Partikel auch in Formkörpern oder Geweben eingesetzt werden.

Geeignet sind beispielsweise Tücher, auf denen die Bleichkatalysatoren fixiert sind, oder geeignete Vorrichtungen, die auf Trägern insbesondere auf Partikeln, fixierte Bleichkatalysatoren enthaltend. Solche Vorrichtungen, z.B. Säckchen die auf Partikeln fixierte Bleichkatalysatoren z.B. als Granulat enthalten, können zur Wasch- bzw Spülflotte hinzugegeben, nach dem Wasch- bzw. Spülvorgang leicht entfernt und so im nächster Vorgang wieder zugesetzt werden. Voraussetzung ist es dass die Vorrichtung für die entsprechenden Verwendungen in geeigneter Form vorliegen und den Kontakt mit Flüssigkeiten insbesondere Wasser (z.B. die Spül-oder Waschflotte) und andere Lösungsmittel, ihre Wirkungsmöglichkeit entfalten, d.h. die Aktivierung von Persauerstoffverbindungen zu ermöglichen.

Die Mittel können als Wasch-, Reinigungs-, Pflege- und/oder Haarbehandlungsmittel (insbesondere zur Färbung, bevorzugt Haarbleiche), Baustoffe, Kosmetika (beispielsweise zur Bleichung von Flecken und/oder Verfärbungen, insbesondere auf Zähnen oder der Haut), Klebstoffe, antibakterielle Mittel und/oder Desinfektionsmittel verwendet werden.

Die Mittel können je nach Anwendungszweck anionische Tenside, kationische Tenside, amphotere Tenside, Buildersubstanzen, Bleichmittel, Bleichaktivatoren, Bleichstabilisatoren, weitere Bleichkatalysatoren, Enzyme, Polymere, Cobuilder, Alkalisierungsmittel, Acidifizierungsmittel, Antiredepositionsmittel, Silberschutzmittel, Färbemittel, optische Aufheller, UV-Schutzsubstanzen, Weichspüler, Duftstoffe, schmutzabweisende Stoffe, Anti-Knitter-Stoffe, antibakterielle Stoffe, Farbschutzstoffe, Verfärbungsinhibitoren, Vitamine, Schichtsilikate, -geruchskomplexierende Substanzen, Klarspüler, Schauminhibitoren, Schäumungsmittel, Konservierungsmittel und/oder Hilfsmittel aufweisen. Insbesondere die bereits weiter oben beschriebenen Inhaltsstoffe sind hierfür einsetzbar.

### Beispiele:

Verfahren zur Messung der Stabilität der erfindungsgemäß verwendbaren trägerfixierten Bleichkatalysatoren.

Es wurden 500 mg trägerfixierter Bleichkatalysator mit einem Gehalt an Übergangsmetall von > 200 mg/l in 1 L Wasser oder 1 L Waschflotte (Waschmittel-Waschflotte Megaperls, Firma Henkel übliche empfohlene Anwenderdosierung) mit einer Wassertemperatur von 40 °C für 1 Stunde gegeben, nach Entfernen der trägerfixierten Bleichkatalysatoren, wies der Restwassergehalt Übergangsmetallrückstände von < 0,02 mg/l, vorzugsweise von < 0,01 mg/l und besonders bevorzugt von < 0,001 mg/l auf. (Nachweisgrenze < 10 mikrogramm/L)

Die Beispiele 1 bis 2 geben Verfahren zur Herstellung von erfindungsgemäß verwendbaren Liganden an. Die Beispiele 3 bis 4 geben Verfahren zur Herstellung der erfindungsgemäß verwendeten an Träger über Ligand(en) gebundene Bleichkatalysator(en) an.

### Beispiel 1

### Darstellung von ((6-Methyl-2-pyridyl)methyl)bis(2-pyridylmethyl)amin (me₁tpa)

### Verfahren zur Herstellung von Bis(2-pyridylmethyl)amin

10.80 g (0.1 mol) 2-Methylpyridylamin wurden in 50 ml Methanol gelöst und mit 10.82 g (0.101 mol) 2-Pyridylcarboxaldehyd versetzt und zwei Stunden bei Raumtemperatur gerührt. Danach wurden bei 0 °C im Eisbad innerhalb von 15 Minuten 1.9 g (0.050 mol) Natriumborhydrid hinzugegeben und die Lösung nach Beendigung der Gasentwicklung über Nacht bei Raumtemperatur gerührt. Die Lösung wurde mit halbkonzentrierter HCl versetzt, um noch nicht umgesetztes Hydrid zu vernichten und dann fünfmal mit je 20 ml Chloroform extrahiert. Die vereinigten organischen Phasen wurden verworfen, die wässrige Phase mit verdünnter NaOH auf einen pH-Wert von 8 gebracht und fünfmal mit je 20 ml Chloroform extrahiert. Die vereinigten organischen Phasen wurden über MgSO₄ getrocknet, das Lösungsmittel am Rotationsverdampfer abgetrennt und der Rückstand bei 2.5 Torr /165 °C fraktioniert destilliert.

### Verfahren zur Herstellung von ((6-Methyl-2-pyridyl)methyl)bis(2-pyridylmethyl)amin (me₁tpa)

In einem 250 ml Dreihalskolben wurden 4.98 g (0.025 mol) Bis(2-pyridylmethyl)amin in 100 ml Methanol gelöst und mit 3.33 g (0.0275 mol) 6-Methyl-2-pyridylaldehyd sowie 4.2 g (0.07 mol) Essigsäure versetzt. Die auf 0 °C abgekühlte Lösung wurde mit 1.38 g (0.022 mol) Natriumcyanoborhydrid, in 50 ml Methanol suspendiert, versetzt. Der entstehende Cyanwasserstoff wurde mit alkalischer KMnO₄-Lösung oxidiert. Nach drei Tagen Rühren bei Raumtemperatur wurde durch Zugabe von konz. HCl nicht umgesetztes Hydrid vernichtet. Das Methanol wurde am Rotationsverdampfer abgetrennt, der Rückstand mit Wasser aufgenommen und mit NaOH alkalisch gemacht, wobei sich ein braunes Öl abscheidet, welches fünfmal mit je 20 ml Chloroform extrahiert wurde. Die vereinigten organischen Phasen wurden über MgSO₄ getrocknet und das Lösungsmittel am Rotationsverdampfer abgetrennt. Das Produkt ließ man in Ligroin (Sdp.: 80-110 °C) auskristallisieren.

### Beispiel 2

### Darstellung von ((6-Chlor-methyl-2-pyridyl)methyl)bis(2-pyridylmethyl)amin (Cl-me₁tpa)

In einem 250 ml Dreihalskolben wurde 1 Äquivalent Bis(2-pyridylmethyl)amin in 100 ml THF gelöst und mit 1 Äquivalent 2,6-bis(chloromethyl)-Pyridin sowie 4 Äquivalenten N,N-Diiisopropylethylamid versetzt. Die Lösung wurde bei Raumtemperatur 7 Tage gerührt. Die Lösung wurde filtiert und das Filtrat säulenchromatographisch gereinigt.

### Beispiel 3

### Fixierung auf chlormethyliertem Polystyrol (Merrifield-Harz (chlormethyliertes Polystyrol)):

### Fixierung dipodaler Liganden (Bis(2-pyridylmethyl)amin):

In einem 250 ml Dreihalskolben wurden 4 g (0.02 mol) Bis(2-pyridylmethyl)amin (bpa) in 100 ml Acetonitril gelöst und in dieser Lösung 10 g (ca. 0.01 mol Cl) Merrifield-Harz (chlormethyliertes Polystyrol) suspendiert. Diese Suspension wurde mit 100 mg (0.7 mol) Natriumiodid versetzt und 48 Stunden unter Rückfluß erhitzt. Das Merrifield-Harz (chlormethyliertes Polystyrol) wurde abfiltriert und mit 25 ml Acetonitril, 25 ml einer 10%igen wässrigen 1:1 Mischung aus Methanol und Kaliumcarbonat, einer wäßrigen 1:1 Mischung aus Methanol und Wasser sowie 25 ml Methanol und Ethanol gewaschen. Der erhaltene Feststoff wurde im Vakuum getrocknet.

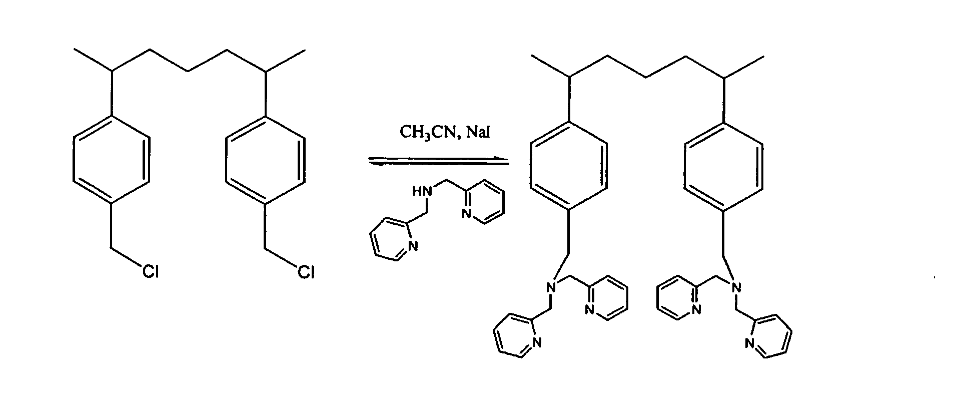

### Beispiel 4

### Fixierung tripodaler Liganden

In einem 250 ml Dreihalskolben wurden unter Stickstoff 1 Äquivalent ((6-Chlormethyl-2-pyridyl)methyl)bis(2-pyridylmethyl)amin (Cl-me₁tpa) in 100 ml Tetrahydrofuran gelöst und zu einer Suspension von jeweils 1 Äquivalent n-Butyllithium (in Heptan gelöst) und Merrifield-Harz (chlormethyliertes Polystyrol) gegeben und bei Raumtemperatur über Nacht gerührt. Das Merrifield-Harz (chlormethyliertes Polystyrol) wurde abfiltriert und mit 25 ml Acetonitril, 25 ml einer 10%igen wäßrigen 1:1 Mischung aus Methanol und Kaliumcarbonat, einer wäßrigen 1:1 Mischung aus Methanol und Wasser sowie 25 ml Methanol und Ethanol gewaschen. Der erhaltene Feststoff wurde im Vakuum getrocknet.

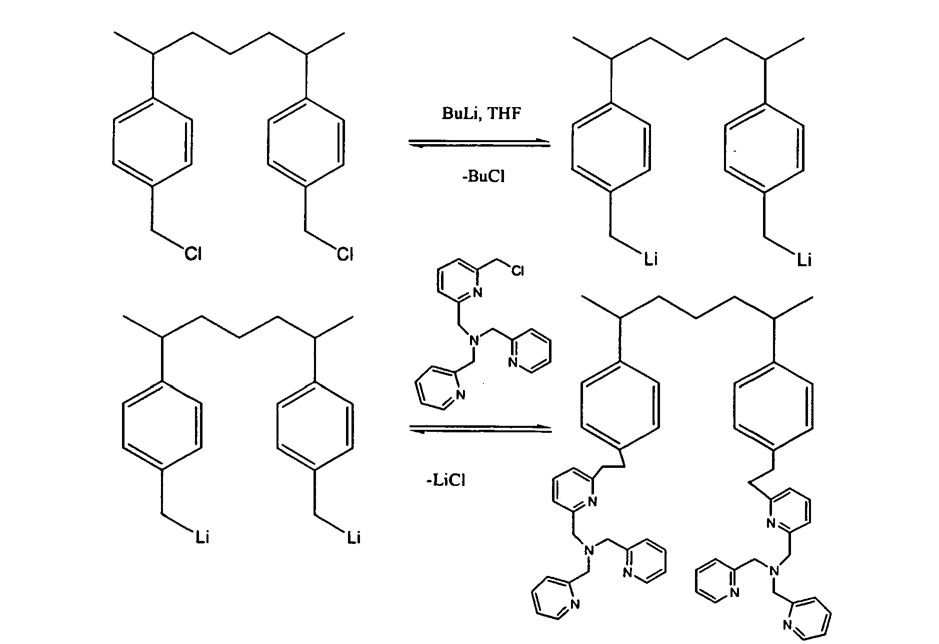

### Beispiel 5

### Fixierung tripodaler Liganden

In einem 250 ml Dreihalskolben wurden unter Stickstoff 1 Äquivalent des Natrium-Alkoholats von ((6-Hydroxy-methyl-2-pyridyl)methyl)bis(2-pyridylmethyl)amin (HO-me₁tpa) in 100 ml Tetrahydrofuran gelöst und zu einer Suspension von 1 Äquivalent Merrifield-Harz (chlormethyliertes Polystyrol, Fa. Fluka) gegeben und bei Raumtemperatur über Nacht gerührt. Das Merrifield-Harz (chlormethyliertes Polystyrol) wurde abfiltriert und mit 25 ml Acetonitril, 25 ml einer 10%igen wäßrigen 1:1 Mischung aus Methanol und Kaliumcarbonat, einer wäßrigen 1:1 Mischung aus Methanol und Wasser sowie 25 ml Methanol und Ethanol gewaschen. Der erhaltene Feststoff wurde im Vakuum getrocknet.

### Beispiel 6

### Fixierung macrozyklischer Liganden

In einem 50 ml Kolben werden 1 g (0.008 mol) Triazacyclononan (Fa. Fluka) und 1 g (0.008 mol) *N*,*N*-Dimethylformamid-dimethylacetal (Fa. Fluka) in Tetrahydrofuran gelöst und bei 85°C unter Rückfluß 3 h gerührt. Zu dieser Lösung wird 1g Merrifield-Harz (chlormethyliertes Polystyrol) gegeben und die Suspension 1 h bei Raumtemperatur gerührt. Das Merrifield-Harz (chlormethyliertes Polystyrol) wird abfiltriert und mit 25 ml Acetonitril, 25 ml einer 10%igen wäßrigen 1:1 Mischung aus Methanol und Kaliumcarbonat, einer wäßrigen 1:1 Mischung aus Methanol und Wasser sowie 25 ml Methanol und Ethanol gewaschen. Der erhaltene Feststoff wird im Vakuum getrocknet.
Anschließend wird das Harz mit 25 Äquivalenten NaOH in Ethanol versetzt und 24 h unter Rückfluß gerührt. Abschließend wird das Merrifield-Harz (chlormethyliertes Polystyrol) abfiltriert und mit 25 ml Acetonitril, 25 ml einer 10%igen wäßrigen 1:1 Mischung aus Methanol und Kaliumcarbonat, einer wäßrigen 1:1 Mischung aus Methanol und Wasser sowie 25 ml Methanol und Ethanol gewaschen. Der erhaltene Feststoff wird im Vakuum getrocknet.

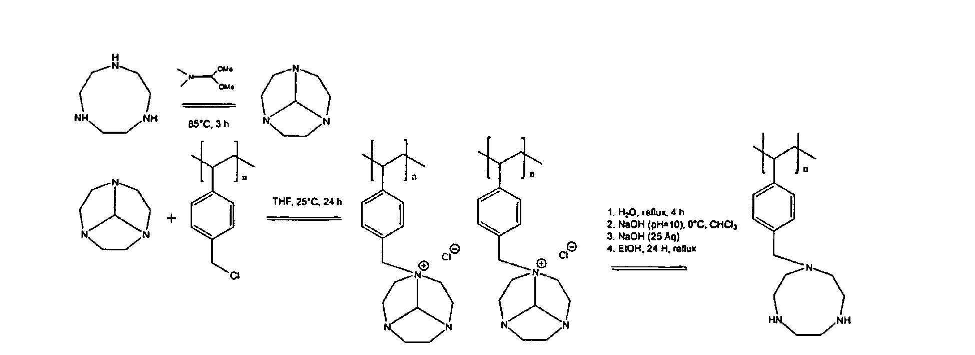

### Abb. 1: Fixierung des Katalysators fix-TACN

### Beispiel 7

### Beladung der fixierten Liganden mit Übergangsmetallen

Auf Merrifield-Harz (chlormethyliertes Polystyrol) fixierte Liganden werden in 5 ml Acetonitril suspendiert und mit einer äquimolaren Lösung von Mn(ClO₄)₂·6H₂O oder Fe(ClO₄)₃ oder Mn(OAc)₃ (Fa. Fluka) in 5 ml Wasser gegeben und 10 Minuten gerührt. Das beladene Merrifield-Harz (chlormethyliertes Polystyrol) wird anschließend im Vakuum getrocknet.

### Beispiel 8

### Untersuchung auf Bleichaktivität (Morin-Test)

Zum Test verschiedener fixierter Komplexe bezüglich ihres Bleichverhaltens wird der zeitliche Verlauf der Extinktion einer Morin-Lösung bei 400 nm beobachtet. Für diese spektroskopischen Untersuchungen wurde in einer Durchflußküvette eine wäßrige Morin-Lösung [5 mg Morin /l, Fa. Fluka] vorgelegt und der im Reaktionsgefäß befindlichen Morinlösung bei einem konstanten pH-Wert von 9,5 (mit Hilfe eines pH-Stat-Gerätes oder Puffer) 980mg/L Perborat-Monohydrat (Fluka), entspricht dem Gehalt von 4,9 g/l in einer Standard-Waschmittelformulierung, hinzugegeben. Die zu testenden Katalysatoren waren unlöslich. Zwei Minuten nach Start des Gerätes werden die "Kugeln" mit den zu testenden Substanzen zugesetzt, so daß im Reaktionsgefäß das komplexierte Übergangsmetall dann mit einer Konzentration von 0.25 mg/l vorliegt. Durch einen Filter konnte der Übergang der Produkte in die Durchflußküvette verhindert werden.

### Ergebnisse mit dipodalen Ligandensystemen:

### Fixierter Ligand hergestellt nach Beispiel 3 und Beladung nach Beispiel 7.

**Tabelle 1: maximale Entfärbung**

| Beladung mit Ligand | Übergangsmetall | max. Entfärbung |
|---|---|---|
| Merrifield 1.5 mmol/ bpa | Fe³⁺ | 5 % |
| Merrifield 2.5 mmol/ bpa | Mn²⁺ | 30 % |
| Merrifield 2.5 mmol/ bpa | Mn^{3/4+} | 40% |
| Merrifield 1 mmol/ bpa | Mn²⁺ | 30 % |
| Merrifield 5.5 mmol/ bpa | Mn²⁺ | 38 % |
| Merrifield 2.5 mmol/ bpa | Mn^{3/4+} | 25 % |
| Merrifield 5.5 mmol/ bpa | Mn^{3/4+} | 17 % |
| Perborat | | 14 % |

### Ergebnisse mit makrozyklischen Ligandensystemen:

### Fixierter Ligand hergestellt nach Beispiel 6 und Beladung nach Beispiel 7.

**Tabelle 2:**

| | Entfärbung durch Mn-tacn in [%] | |
|---|---|---|
| Zeit [min] | (fixiert) | (gelöst) |
| 0 | 0 | 0 |
| 5 | 60 | 60 |
| 10 | 83 | 88 |
| 15 | 89 | 94 |
| 20 | 90 | 84 |
| 25 | 91 | 84 |
| 30 | 92 | 99 |

Fixierte bzw. heterogene Katalysatorsysteme zeigen - auch im Vergleich mit homogenen Katalysatoren oder Perborat - z. T. sehr hohe Aktivitäten bei der katalytischen Entfärbung bzw. katalytischen Bleiche von Morin (Tabelle 2).

### Beispiel 9

### Mehrfachnutzung der fixierten Katalysatoren

Zum Test verschiedener fixierter Komplexe bezüglich der mehrfachen Nutzung wird der zeitliche Verlauf der Extinktion einer Morin-Lösung bei 400 nm über 3 mal 30 Minuten beobachtet. Eingesetzt wurde ein makrozyklisches Ligandensystem mit Mn^{3+/4+} beladen, weitere Details wie in Beispiel 8 beschrieben. Nach dem jeweiligen Test wird der Katalysator abfiltriert, getrocknet und anschließend nochmals eingesetzt

**Tabelle 3: Mehrfachnutzung**

| Zeit [min] | Entfärbung | Entfärbung | Entfärbung | Perborat | |
|---|---|---|---|---|---|
| | 1. Test | 2. Test | 3. Test | | |
| 0 | 0 | 0 | 0 | 0 | |
| 5 | 78 | 46 | 44 | 3 | |
| 10 | 90 | 71 | 58 | 5 | |
| 15 | 90 | 82 | 71 | 7 | |
| 20 | 94 | 88 | 75 | 8 | |
| 25 | 94 | 91 | 78 | 10 | |
| 30 | 95 | 92 | 85 | 10 | |

Beim dritten Test konnte mit dem gleichen Material trotz leichter Verluste bei der Rückgewinnung eine Entfärbung von 85 % erzielt werden (Tabelle 3). Eine mehrfache Nutzung ist damit selbst ohne Wiederbeladung mit Übergangsmetall denkbar.

### Beispiel 10

### Waschversuche / Nachweis von Übergangsmetallen im Waschwasser

Für die Bleichkatalysatoren nach Beispiel 3 und 7 wurde der an die Waschlauge abgegebene Mangangehalt gemessen. In eine Waschlauge mit 980mg/L Perborat-Monohydrat, entspricht 4,9g/L Waschmittelformulierung bzw. handelsübliche Waschmittel ohne Übergangsmetallbleichkatalysatoren (Persil color und Persil megaperls, Henkel KGaA) mit der vorgegebenen Dosierung wurden 500 mg/L polymerfixierte Katalysatoren zugesetzt. Der Mangangehalt (mg Mn/L) wurde per Atom-Absorptionsspektroskopie aus Proben nach 90 Minuten Waschzyklus bei 60°C ohne Wäsche in einem Laborlaunderometer (Dreifachbestimmung, in Tabelle 4 sind die Maximalwerte angegeben) bestimmt.

**Tabelle 4: Konzentration von Übergangmetallen in Waschwasser**

| Lösung mit Persil megaperls + Katalysatoren, | Zeit | |
|---|---|---|
| mg Mn/l | t=0 | t=90 min |
| | < 0.05 | < 0.05 |
| | 0.06 | 0.12 |
| | 0.07 | 0.12 |

Im Waschwasser war auch nach 90 min praktisch kein Mangan nachweisbar. Eine Textilschädigung aufgrund der Anhaftung des Übergangsmetalls auf den Textilien kann daher ausgeschlossen werden.

## Patentansprüche

1. Verwendung von trägerfixierten Bleichkatalysatoren zur Katalyse von Peroxidverbindungen zum Bleichen von Farben, insbesondere von Haarfarben und/oder Farbanschmutzungen auf und/oder in harten und/oder weichen Oberflächen, **dadurch gekennzeichnet, dass** der oder die trägerfixierten Bleichkatalysatoren über wenigstens einen organischen Liganden des Bleichkatalysators kovalent an einen Träger gebunden ist und wobei der oder die Bleichkatalysatoren einen Komplex mit wenigstens einem Übergangsmetall ausbilden.

2. Verwendung nach Anspruch 1, **dadurch gekennzeichnet, dass** wenigstens ein an einen Träger kovalent gebundener Ligand ein Übergangsmetall freier Liganden ist, der mit einem Übergangsmetall chelatisiert, das von einer anderen Quelle, vorzugsweise aus der Bleichmittelzusammensetzung und/oder zugesetztem Wasser, abstammt, und so den Übergangsmetall aufweisenden Komplex gemäß Anspruch 1 ausbildet.

3. Verwendung nach Anspruch 1 oder 2, wobei das Übergangsmetall ausgewählt ist aus der Gruppe, umfassend Mangan, Eisen, Cobalt, Ruthenium, Molybdän, Titan, Vanadium und/oder Kupfer.

4. Verwendung nach einem der vorherigen Ansprüche, **dadurch gekennzeichnet, dass** der organische Ligand ein mono- oder multipodaler Ligand, vorzugsweise dipodaler, tripodaler, tetrapodaler, pentapodaler oder hexapodaler Ligand ist.

5. Verwendung nach einem der vorherigen Ansprüche, **dadurch gekennzeichnet, dass** der organische Ligand über eine Einfach-, Doppel- oder Dreifach-Bindung an den Träger kovalent gebunden ist.

6. Verwendung nach einem der vorherigen Ansprüche, **dadurch gekennzeichnet, dass** der organische Ligand über wenigstens ein Stickstoffatom des Liganden mittels einer kovalenten Bindung an den Träger gebunden ist.

7. Verwendung nach einem der vorherigen Ansprüche, **dadurch gekennzeichnet, dass** der kovalent an einen Träger bindbare organische Ligand vorzugsweise ausgewählt ist aus der Gruppe umfassend:
Bis(2-pyridylmethyl)amin, (2-Pyridylmethyl)(2-(2-pyridyl)-ethyl)amin, (2-Pyridylmethyl)(3-(N,N-dimethylamino)-propyl)amin, (2-Pyridylmethyl)(2-(N,N-dimethylamino)-ethyl)amin, (2-Pyridylmethyl)(2-hydroxyethyl)amin, (2-Pyridylmethyl)(3-hydroxypropyl)amin, (2-Pyridylmethyl)(2-N-morpholinoethyl)amin, (2-Pyridylmethyl)(2-N-piperidinoethyl)amin, (2-Pyridylmethyl)(2-N-pyrrolidinoethyl)amin, (2-Pyridylmethyl)(2-N-piperazinoethyl)amin, (2-Hydroxybenzyl)(2-pyridylmethyl)amin, (2-Hydroxybenzyl)(2-(2-pyridyl)-ethyl)amin, (2-Hydroxybenzyl)(3-(N,N-dimethylamino)-propyl)amin, (2-Hydroxybenzyl) 2-(N,N-dimethylamino)-ethyl)amin, (2-Hydroxybenzyl)(2-hydroxyethyl)amin, (2-Hydroxybenzyl)(3-hydroxypropyl)amin, (2-Hydroxybenzyl)(2-N-morpholinoethyl)amin, (2-Hydroxybenzyl)(2-N-piperidinoethyl)amin, (2-Hydroxybenzyl)(2-N-pyrrolidinoethyl)amin, (2-Hydroxybenzyl)(2-N-piperazinoethyl)amin, ((6-Methyl-2-pyridyl)methyl)bis(2-pyridylmethyl)amin, Bis((6-Methyl-2-pyridyl)methyl)(2-pyridylmethyl)amin, Tris((6-methyl-2-pyridyl)methyl)amin, [(Benzimidazol-2-yl)methyl][(6-Methyl-2-pyridyl)methyl)(2-pyridyl)methyl]amin,
Bis[(Benzimidazol-2-yl)methyl][(6-Methyl-2-pyridyl)methyl)]amin, [(5,6-dimethylbenzimidazol-2-yl)methyl][(6-Methyl-2-pyridyl)methyl)(2-pyridyl)methyl]amin, Bis[(5,6-dimethylbenzimidazol-2-yl)methyl][(6-Methyl-2-pyridyl)methyl)]amin, [(2-pyridyl)methyl(6-Methyl-2-pyridyl)(2-chinolyl)methyl]amin, Bis[(2-chinolyl)(6-Methyl-2-pyridyl) methyl]amin, [(2-pyridyl)methyl](2-N-morpholinoethyl)][(6-methyl-2-pyridyl)-methyl]amin,[(2-pyridyl)methyl](2-N-piperidinoethyl)][(6-methyl-2-pyridyl)-methyl]amin, *pmap [2-*(2-pyridyl)ethyl][(2-pyridyl)methyl])][(6-methyl-2-pyridyl)-methyl]amin, *pmea* [(2-pyridyl)methyl][2-(2-pyridyl)ethyl])][(6-methyl-2-pyridyl)-methyl]amin, N,N,N',N'-Tetrakis[2-benzimidazolylmethyl]-1,3-diamino-2-propanol, N,N,N',N'-Tetrakis[2-(5,6-dimethyl)-benzimidazolylmethyl]-1,3-diamino-2-propanol, N,N,N',N'-Tetrakis[2-(2-hydroxyethyl)-benzimidazolylmethyl]-1,3-diamino-2-propanol und/oder N,N,N',N'-Tetrakis[2-(1-methyl)-imidazolylmethyl]-1,3-diamino-2-propanol.

8. Verwendung nach einem der vorherigen Ansprüche, **dadurch gekennzeichnet, dass** der Träger ein Polymer aufweist, das wenigstens einen zur Ausbildung einer kovalenten Bindung geeigneten Substituenten und/oder funktionelle Gruppe enthält, vorzugsweise ausgewählt aus der Gruppe umfassend -H, -OH, -NH₂, -NH-R, -Halogen, -SH, -Si-OR, -C=C, -C≡C, -OR, -NCO, -COOH, -COOR, -CHO, -CN, -NH-C=O, -O=C-O-C=O und/oder Epoxid.

9. Verwendung nach einem der vorherigen Ansprüche, **dadurch gekennzeichnet, dass** das zur Ausbildung einer kovalenten Bindung an wenigstens einen Liganden geeignete Polymer ausgewählt ist aus der Gruppe umfassend Polyvinylchlorid, Polybutadien, Polychlorbutadien, Polyvinilydenchlorid, Polyacrylnitril, Polydichlormethyloxaisobutan, Polyurethan, Polystyrole, Polymethacrylate, Polyvinylalkohole, Polyethylenimine, Cellulose, Chitosan, Polysiloxane, Polyamide, Polyamine, Polyformaldehyde, Polyethylen, Polypropylen, Polytetrafluorethylen, Polyisobutylen, Polydimethylpheylenoxid, Chlormethyliertes Polystyrol und/oder Polyisocyanate, wobei Chlormethyliertes Polystyrol am meisten bevorzugt ist.

10. Verwendung nach einem der vorherigen Ansprüche, **dadurch gekennzeichnet, dass** der Träger ein Formkörper, vorzugsweise Pulver, Partikel, Agglomerat und/oder Faser ist und vorzugsweise ein Tuch ist.

11. Verwendung nach Anspruch 10 **dadurch gekennzeichnet, dass** die Partikel vorzugsweise einen Partikeldurchmesser von 20 µm und 1 mm und vorzugsweise zwischen 200 µm und 0,6 mm aufweisen.

12. Verwendung nach einem der vorherigen Ansprüche, **dadurch gekennzeichnet, dass** das Übergangsmetall im Bleichkatalysator-Komplex Eisen und/oder Mangan ist.

13. Verwendung nach einem der vorherigen Ansprüche, **dadurch gekennzeichnet, dass** das Übergangsmetall in der Oxidationsstufe +2, +3 oder +4 vorliegt.

14. Verwendung nach einem der vorherigen Ansprüche, **dadurch gekennzeichnet, dass** der trägerfixierte Bleichkatalysator Persauerstoffverbindungen ausgewählt aus der Gruppe umfassend organische Persäuren, Wasserstoffperoxid, Perborat und/oder Percarbonat aktiviert.

15. Verwendung nach einem der vorherigen Ansprüche, **dadurch gekennzeichnet, dass** der trägerfixierte Bleichkatalysator bei einem pH-Wert von zwischen 7-14, vorzugsweise 8-10 und bevorzugt ≥ 9,5 regenerierbar ist.

16. Verwendung nach einem der vorherigen Ansprüche, **dadurch gekennzeichnet, dass** das Restwasser von in 1 L Wasser oder von in 1 L Waschflotte mit einer Wassertemperatur von 40 °C für 1 Stunde behandelten trägerfixierten Bleichkatalysatoren mit einem Gehalt an Übergangsmetall von > 200 mg/l, nach Entfernen der trägerfixierten Bleichkatalysatoren einen Restwassergehalt an Übergangsmetallrückständen von ≤ 0,02 mg/l, vorzugsweise von ≤ 0,01 mg/l und besonders bevorzugt von ≤ 0,001 mg/l, aufweist.

17. Verwendung nach einem der vorherigen Ansprüche, **dadurch gekennzeichnet, dass** der an einen Träger kovalent bindende Ligand ausgewählt ist aus der Gruppe umfassend Pyridin-2-yl enthaltende Liganden, 2-Aminoethyl enthaltende Liganden, N-Methyl-N,N',N'-tris(3-methyl-pyridin-2-ylmethyl)-ethylen-1,2-diamin und/oder N-Ethyl-N,N',N'-tris(3-methyl-pyridin-2-ylmethyl)-ethylen-1,2-diamin.

18. Verwendung nach einem der vorstehenden Ansprüche, **dadurch gekennzeichnet, dass** die trägerfixierten Bleichkatalysatoren zum Bleichen von farbigen Anschmutzungen beim Waschen von Textilien eingesetzt werden.

19. Verwendung der trägerfixierten Bleichkatalysatoren nach einem der vorherigen Ansprüche in flüssigen, festen und/oder gelförmigen Mitteln, insbesondere Reinigungsmitteln, für harte Oberflächen, insbesondere für Geschirr, zum Bleichen von farbigen Anschmutzungen.

20. Verwendung der trägerfixierten Bleichkatalysatoren nach einem der vorherigen Ansprüche, wobei flüssige, feste, und/oder gelförmige trägerfixiertere Bleichkatalysatoren in eine Dosierkammer, vorzugsweise Klarspülkammer oder Waschmaschinenspülkammer, eingebracht werden.

21. Verwendung eines Mittels mit trägerfixierten Bleichkatalysatoren nach einem der vorherigen Ansprüche, **dadurch gekennzeichnet, dass** es 0,0025 Gew.-% bis 0,25 Gew.-%, insbesondere 0,01 Gew.-% bis 0,1 Gew.-% Bleichkatalysator(en) enthält.

22. Verwendung eines Mittels mit trägerfixierten Bleichkatalysatoren nach einem der vorherigen Ansprüche, **dadurch gekennzeichnet, dass** es ein Wasch-, Reinigungs-, Pflege-, Haarbehandlungs-, Baustoff, Kosmetika, Klebstoff, Antibakterielles Mittel und/oder Desinfektionsmittel ist.

23. Verwendung eines Mittels mit trägerfixierten Bleichkatalysatoren nach einem der vorherigen Ansprüche, **dadurch gekennzeichnet, dass** es anionische Tenside, kationische Tenside, amphotere Tenside, Buildersubstanzen, Bleichmittel, Bleichaktivatoren, Bleichstabilisatoren, Bleichkatalysatoren, Enzyme, Polymere, Cobuilder, Alkalisierungsmittel, Acidifizierungsmittel, Antiredepositionsmittel, Silberschutzmittel, Färbemittel, optische Aufheller, UV-Schutzsubstanzen, Weichspüler, Duftstoffe, schmutzabweisende Stoffe, Anti-Knitter-Stoffe, antibakterielle Stoffe, Farbschutzstoffe, Verfärbungsinhibitoren, Vitamine, Schichtsilikate, geruchskomplexierende Substanzen, Klarspüler, Schauminhibitoren, Schäumungsmittel, Konservierungsmittel und/oder Hilfsmittel aufweist.

## Claims

1. Use of support-fixed bleaching catalysts for the catalysis of peroxide compounds for bleaching colors, especially hair colors and/or color stains on and/or in hard and/or soft surfaces, **characterized in that** the support-fixed bleaching catalysts or catalysts is bonded covalently to a support via at least one organic ligand of the bleaching catalyst, the bleaching catalyst or catalysts forming a complex with at least one transition metal.

2. Use according to Claim 1, **characterized in that** at least one ligand bonded covalently to a support is a transition-metal-free ligand which is chelating with a transition metal which originates from another source, preferably from the bleach composition and/or added water, and so forms the transition metal complex according to claim 1.

3. Use according to Claim 1 or 2, **characterized in that** the transition metal is selected from the group consisting of manganese, iron, cobalt, ruthenium, molybdenum, titanium, vanadium and/or copper.

4. Use according to one of the preceding claims, **characterized in that** the organic ligand is a monopodale or multipodale ligand, preferably a dipodal, tripodal, tetrapodale, pentapodale or hexapodale ligand.

5. Use according to one of the preceding claims, **characterized in that** the organic ligand is bonded covalently to the support via a single, double or triple bond.

6. Use according to one of the preceding claims, **characterized in that** the organic ligand is bonded to the support by means of a covalent bond via at least one nitrogen atom of the ligand.

7. Use according to one of the preceding claims, **characterized in that** the organic ligand which can be bonded covalently to a support is preferably selected from the group consisting of:
bis(2-pyridylmethyl)amine, (2-pyridylmethyl)(2-(2-pyridyl)ethyl)amine, (2-pyridylmethyl)(3-(N,N-dimethylamino)propyl)amine, (2-pyridylmethyl)(2-(N,N-dimethylamino)ethyl)amine, (2-pyridylmethyl)-(2-hydroxyethyl) amine, (2-pyridylmethyl) (3-hydroxypropyl) amine, (2-pyridylmethyl) (2-N-morpholinoethyl)amine, (2-pyridylmethyl)(2-N-piperidinoethyl)amine, (2-pyridylmethyl)(2-N-pyrrolidinoethyl)amine, (2-pyridylmethyl)(2-N-piperazinoethyl)amine, (2-hydroxybenzyl)(2-pyridylmethyl)-amine, (2-hydroxybenzyl)(2-(2-pyridyl)ethyl)amine, (2-hydroxybenzyl)(3-(N,N-dimethylamino)propyl)-amine, (2-hydroxybenzyl)2-(N,N-dimethylaminoethyl)amine, (2-hydroxybenzyl)(2-hydroxyethyl)amine, (2-hydroxybenzyl)(3-hydroxypropyl)amine, (2-hydroxybenzyl)(2-N-morpholinoethyl)amine, (2-hydroxybenzyl)(2-N-piperidinoethyl)amine, (2-hydroxybenzyl)(2-N-pyrrolidinoethyl)amine, (2-hydroxybenzyl)(2-N-piperazinoethyl)amine, ((6-methyl-2-pyridyl)methyl)bis(2-pyridylmethyl)-amine, bis((6-methyl-2-pyridyl)methyl)(2-pyridylmethyl)amine, tris((6-methyl-2-pyridyl)methyl)-amine, [(benzimidazol-2-yl)methyl][(6-methyl-2-pyridyl)methyl)(2-pyridyl)methyl]amine, bis[(benzimidazol-2-yl)methyl][(6-methyl-2-pyridyl)-methyl)]amine, [(5,6-dimethylbenzimidazol-2-yl)methyl][(6-methyl-2-pyridyl)methyl)(2-pyridyl) methyl] amine, bis[(5,6-dimethylbenzimidazol-2-yl)methyl][(6-methyl-2-pyridyl)-methyl)]amine, [(2-pyridyl)methyl(6-methyl-2-pyridyl) (2-quinolyl)methyl] amine, bis[(2-quinolyl)(6-methyl-2-pyridyl)methyl]amine, [(2-pyridyl)methyl](2-N-morpholinoethyl)][(6-methyl-2-pyridyl)methyl]amine, [(2-pyridyl)methyl](2-N-piperidinoethyl)][(6-methyl-2-pyridyl)methyl]-amine, *pmap* [2- (2-pyridyl) ethyl] [ (2-pyridyl) - methyl])][(6-methyl-2-pyridyl)methyl]amine, *pmea* [(2-pyridyl)methyl][2-(2-pyridyl)ethyl])] [(6-methyl-2-pyridyl) methyl] , N,N,N',N'-tetrakis-[2-benzimidazolylmethyl]-1,3-diamino-2-propanol, N,N,N',N'-tetrakis[2-(5,6-dimethyl)benzimidazolylmethyl]-1,3-diamino-2-propanol, N,N,N',N'-tetrakis[2-(2-hydroxyethyl)benzimidazolylmethyl]-1,3-diamino-2-propanol and/or N,N,N',N'-tetrakis-[2-(1-methyl)imidazolylmethyl]-1,3-diamino-2-propanol.

8. Use according to one of the preceding claims, **characterized in that** the support comprises a polymer containing at least one functional group and/or substituents suitable for forming a covalent bond, preferably selected from the group consisting of -H, -OH, -NH₂, -NH-R, -halogen, -SH, -Si-OR, -C=C, -C≡C, -OR, -NCO, -COOH, -COOR, -CHO, -CN, -NH-C=O, -O=C-O-C=O and/or epoxide.

9. Use according to one of the preceding claims, **characterized in that** the polymer suitable for forming a covalent bond to at least one ligand is selected from the group consisting of polyvinyl chloride, polybutadiene, polychlorobutadiene, polyvinylidene chloride, polyacrylonitrile, polydichloromethyloxaisobutane, polyurethane, polystyrenes, polymethacrylates, polyvinyl alcohols, polyethylenimines, cellulose, chitosan, polysiloxanes, polyamides, polyamines, polyformaldehydes, polyethylene, polypropylene, polytetrafluoroethylene, polyisobutylene, polydimethylphenylene oxide, chloromethylated polystyrene and/or polyisocyanates, chloromethylated polystyrene being the most preferred.

10. Use according to one of the preceding claims, **characterized in that** the support is a shaped article, preferably powder, particle, agglomerate and/or fiber and is preferably a cloth.

11. Use according to one of the preceding claims, **characterized in that** the particle preferably has a particle diameter of 20 µm and 1 mm and preferably between 200 µm and 0.6 mm.

12. Use according to one of the preceding claims, **characterized in that** the transition metal in the bleaching catalyst complex is iron and/or manganese.

13. Use according to one of the preceding claims, **characterized in that** the transition metal is in oxidation state +2, +3 or +4.

14. Use according to one of the preceding claims, **characterized in that** the support-fixed bleaching catalyst activates peroxygen compounds selected from the group consisting of organic peracids, hydrogen peroxide, perborate and/or percarbonate.

15. Use according to one of the preceding claims, **characterized in that** the support-fixed bleaching catalyst can be regenerated at a pH of between 7-14, preferably 8-10 and more preferably ≥ 9.5.

16. Use according to one of the preceding claims, **characterized in that** the residual water from support-fixed bleaching catalysts having a transition metal content of > 200 mg/l and treated in 1 L of water or in 1 L of wash liquor with a water temperature of 40°C for 1 hour, following removal of the support-fixed bleaching catalysts, has a residual content of transition metal residues of ≤ 0.02 mg/l, preferably of ≤ 0.01 mg/l and more preferably of ≤ 0.001 mg/l.

17. Use according to one of the preceding claims, **characterized in that** the ligand bonding covalently to a support is selected from the group consisting of ligands containing pyridin-2-yl, ligands containing 2-aminoethyl, N-methyl-N,N',N'-tris(3-methylpyridin-2-ylmethyl)ethylene-1,2-diamine and/or N-ethyl-N,N',N'-tris(3-methylpyridin-2-ylmethyl)ethylene-1,2-diamine.

18. Use according to one of the preceding claims, **characterized in that** the support-fixed bleaching catalysts are used for bleaching colored stains in connection with the laundering of textiles.

19. Use of the support-fixed bleaching catalysts according to one of the preceding claims in liquid, solid and/or gel-form compositions, especially cleaning products, for hard surfaces, in particular for tableware, for bleaching colored stains.

20. Use of the support-fixed bleaching catalysts according to one of the preceding claims, wherein liquid, solid and/or gel-form more support-fixed bleaching catalysts are introduced into a metering compartment, preferably rinse aid compartment or washing machine rinse draw.

21. Use of a composition comprising support-fixed bleaching catalysts according to one of the preceding claims, **characterized in that** it contains from 0.0025% by weight to 0.25% by weight, in particular from 0.01% by weight to 0.1% by weight of bleaching catalyst(s).

22. Use of a composition comprising support-fixed bleaching catalysts according to one of the preceding claims, **characterized in that** it is a washing, cleaning, care, hair treatment, building material, cosmetic, adhesive, antibacterial product and/or disinfectant.

23. Use of a composition comprising support-fixed bleaching catalysts according to one of the preceding claims, **characterized in that** it comprises anionic surfactants, cationic surfactants, amphoteric surfactants, builder substances, bleaches, bleach activators, bleach stabilizers, bleaching catalysts, enzymes, polymers, cobuilders, alkalizing agents, acidifying agents, antiredeposition agents, silver protectants, colorants, optical brighteners, UV stabilizers, fabric softeners, fragrances, soil repellents, anticrease substances, antibacterial substances, color protectants, discoloration inhibitors, vitamins, phyllosilicates, odor-complexing substances, rinse aids, foam inhibitors, foaming agents, preservatives and/or auxiliaries.

## Revendications

1. Utilisation de catalyseurs de blanchiment fixés à un support pour la catalyse de composés peroxyde afin de blanchir des couleurs, en particulier des couleurs capillaires et/ou des taches colorées sur et/ou dans des surfaces dures et/ou molles, **caractérisée en ce que** le ou les catalyseurs de blanchiment fixés à un support sont liés de manière covalente à un support par l'intermédiaire d'au moins un ligand organique du catalyseur de blanchiment, et le ou les catalyseurs de blanchiment formant un complexe avec au moins un métal de transition.

2. Utilisation selon la revendication 1, **caractérisée en ce qu'**au moins un ligand lié de manière covalente à un support est un ligand exempt de métal de transition, qui est chélaté avec un métal de transition, qui provient d'une autre source, de préférence de la composition d'agent de blanchiment et/ou de l'eau ajoutée, et forme ainsi le complexe présentant un métal de transition selon la revendication 1.

3. Utilisation selon la revendication 1 ou 2, dans laquelle le métal de transition est choisi dans le groupe comprenant le manganèse, le fer, le cobalt, le ruthénium, le molybdène, le titane, le vanadium, et/ou le cuivre.

4. Utilisation selon l'une quelconque des revendications précédentes, **caractérisée en ce que** le ligand organique est un ligand monopode ou multipode, de préférence bipode, tripode, tétrapode, pentapode ou hexapode.

5. Utilisation , selon l'une quelconque des revendications précédentes, **caractérisée en ce que** le ligand organique est lié de façon covalente au support par l'intermédiaire d'une simple, double ou triple liaison.

6. Utilisation selon l'une quelconque des revendications précédentes, **caractérisée en ce que** le ligand organique est lié au support par l'intermédiaire d'au moins un atome d'azote du ligand, au moyen d'une liaison covalente.

7. Utilisation selon l'une quelconque des revendications précédentes, **caractérisée en ce que**, le ligand organique pouvant se lier de manière covalente à un support, est choisi de préférence dans le groupe comprenant :
la bis(2-pyridylméthyl)amine, la (2-pyridylméthyl)(2-(2-pyridyl)-éthyl)amine, la (2-pyridylméthyl)(3-(N,N-diméthylamino)-propyl)amine, la (2-pyridylméthyl)(2-(N,N-diméthylamino)-éthyl)amine, la (2-pyridylméthyl)(2-hydroxyéthyl)amine, la (2-pyridylméthyl)(3-hydroxypropyl)amine, la (2-pyridylméthyl)(2-N-morpholino-éthyl)amine, la (2-pyridylméthyl)(2-N-pipéridinoéthyl)amine, la (2-pyridylméthyl)(2-N-pyrrolidinoéthyl)amine, la (2-pyridylméthyl)(2-N-pipérazino-éthyl)amine, la (2-hydroxybenzyl)(2-pyridylméthyl)amine, la (2-hydroxybenzyl)(2-(2-pyridyl)-éthyl)amine, la (2-hydroxybenzyl)(3-(N,N-diméthylamino)-propyl)amine, la (2-hydroxybenzyl)-2-(N,N-diméthylamino)-éthyl)amine, la (2-hydroxybenzyl)(2-hydroxyéthyl)amine, la (2-hydroxybenzyl)(3-hydroxypropyl)amine, la (2-hydroxybenzyl)(2-N-morpholinoéthyl)amine, la (2-hydroxybenzyl)(2-N-pipéridinoéthyl)amine, la (2-hydroxybenzyl)(2-N-pyrrolidino-éthyl)amine, la (2-hydroxybenzyl)(2-N-pipérazinoéthyl)amine, la ((6-méthyl-2-pyridyl)méthyl)bis(2-pyridylméthyl)amine, la bis((6-méthyl-2-pyridyl)-méthyl)(2-pyridylméthyl)amine, la tris((6-méthyl-2-pyridyl)méthyl)amine, la [(benzimidazol-2-yl)méthyl][(6-méthyl-2-pyridyl)méthyl)(2-pyridyl)méthyl]amine, la bis[(benzimidazol-2-yl)méthyl][(6-méthyl-2-pyridyl)méthyl)]amine, la [(5,6-diméthylbenzimidazol-2-yl)méthyl][(6-méthyl-2-pyridyl)méthyl)(2-pyridyl)-méthyl]-amine, la bis[(5,6-diméthylbenzimidazol-2-yl)méthyl][(6-méthyl-2-pyridyl)-méthyl)]amine, la [(2-pyridyl)méthyl(6-méthyl-2-pyridyl)(2-quinoléyl)méthyl]-amine, la bis[(2-quinoléyl)(6-méthyl-2-pyridyl)méthyl]amine, la [(2-pyridyl)-méthyl](2-N-morpholinoéthyl)][(6-méthyl-2-pyridyl)-méthyl]amine, la [(2-pyridyl)-méthyl](2-N-pipéridinoéthyl)][(6-méthyl-2-pyridyl)-méthyl]amine, la *pmap* [2(2-pyridyl)éthyl][(2-pyridyl)méthyl])][(6-méthyl-2-pyridyl)-méthyl]amine, la *pmea* [(2-pyridyl)méthyl][2-(2-pyridyl)éthyl])][(6-méthyl-2-pyridyl)-méthyl]amine, le N,N,N',N'-tétrakis[2-benzimidazolylméthyl]-1,3-diamino-2-propanol, le N,N,N',N'-tétrakis[2-(5,6-diméthyl)-benzimidazolylméthyl]-1,3-diamino-2-propanol, le N,N,N',N'-tétrakis[2-(2-hydroxyéthyl)-benzimidazolyl-méthyl]-1,3-diamino-2propanol et/ou le N,N,N',N'-tétrakis[2-(1-méthyl)-imidazolylméthyl]-1,3-diamino-2-propanol.

8. Utilisation selon l'une quelconque des revendications précédentes, **caractérisée en ce que** le support présente un polymère qui contient au moins un substituant et/ou groupe fonctionnel convenant à la formation d'une liaison covalente qui est choisi dans le groupe comprenant -H-, -OH, -NH₂, -NH-R, -halogéno, -SH, -Si-OR, -C=C, -C≡C, -OR, -NCO, -COOH, - COOR, -CHO, -CN, -NH-C=O, -O=C-O-C=O, et/ou un groupe époxyde.

9. Utilisation selon l'une quelconque des revendications précédentes, **caractérisée en ce que** le polymère convenant à la formation d'une liaison covalente à au moins un ligand, est choisi dans le groupe comprenant le poly(chlorure de vinyle), le polybutadiène, le polychlorobutadiène, le poly(chlorure de vinylidène), le polyacrilonitrile, le polydichlorométhyloxaisobutane, le polyuréthane, les polystyrènes, les polyméthacrylates, les poly(alcool vinylique)s les polyéthylèneimines, la cellulose, le chitosan, les polysiloxanes, les polyamides, les polyamines, le polyformaldéhyde, le polyéthylène, le polypropylène, le polytétrafluoroéthylène, le polyisobutylène, le poly(oxyde de diméthylphénylène), le polystyrène chlorométhylé, et/ou les polyisocyanates, le polystyrène chlorométhylé, étant le plus préféré.

10. Utilisation selon l'une quelconque des revendications précédentes, **caractérisée en ce que** le support est un corps moulé, de préférence une poudre, des particules, un aggloméré, et/ou des fibres et, de préférence, un tissu.

11. Utilisation selon l'une quelconque des revendications précédentes, **caractérisée en ce que** les particules présentent de préférence un diamètre de particule entre 20 µm et 1 mm, et de préférence compris entre 200 µm et 0,6 mm.

12. Utilisation selon l'une quelconque des revendications précédentes, **caractérisée en ce que** le métal de transition dans le complexe de catalyseur de blanchiment, est le fer et/ou le manganèse.

13. Utilisation selon l'une quelconque des revendications précédentes, **caractérisée en ce que** le métal de transition est présent dans l'état d'oxydation +2, +3 ou +4.

14. Utilisation selon l'une quelconque des revendications précédentes, **caractérisée en ce que** le catalyseur de blanchiment fixé à un support active les composés peroxy choisis dans le groupe comprenant les peracides organiques, le peroxyde d'hydrogène, un perborate et/ou un percarbonate.

15. Utilisation selon l'une quelconque des revendications précédentes, **caractérisé en ce que** le catalyseur de blanchiment fixé à un support peut être régénéré à une valeur de pH comprise entre 7 et 14, de préférence entre 8 et 10, et préférentiellement ≥ à 9,5.

16. Utilisation selon l'une quelconque des revendications précédentes, **caractérisée en ce que** l'eau résiduelle de catalyseurs de blanchiments fixés à un support traités dans 1 l d'eau ou dans 1 l de lessive avec une température de l'eau de 40°C pendant 1 h, ayant une teneur en métal de transition > 200 mg/l après élimination des catalyseurs de blanchiment fixés à un support, présente une teneur d'eau résiduelle en résidus de métaux de transition ≤ 0,02 mg/l, de préférence ≤ 0,01 mg/l, et de manière particulièrement préférée ≤ 0,001 mg/l.

17. Utilisation selon l'une quelconque des revendications précédentes, **caractérisée en ce que** le ligand se liant de manière covalente à un support, est choisi dans le groupe comprenant les ligands contenant un groupe pyridin-2-yle, les ligands contenant un groupe 2-aminoéthyle, la N-méthyl-N,N',N'-tris(3-méthyl-pyridin-2-yl-méthyl)-éthylène-1,2-diamine, et /ou la N-éthyl-N,N',N'-tris(3-méthyl-pyridin-2-yl-méthyl)-éthylène-1,2-diamine.

18. Utilisation selon l'une quelconque des revendications précédentes, **caractérisée en ce que** les catalyseurs de blanchiment fixés à un support sont mis en oeuvre pour blanchir des salissures colorées lors du lavage de textiles.

19. Utilisation des catalyseurs de blanchiment fixés à un support, selon l'une quelconque des revendications précédentes, dans des agents liquides, solides, et/ou sous forme de gels, en particulier des agents de nettoyage, pour les surfaces dures, en particulier pour la vaisselle, pour blanchir des salissures colorées.

20. Utilisation des catalyseurs de blanchiment fixés à un support, selon l'une quelconque des revendications précédentes, dans laquelle des catalyseurs de blanchiment fixés à un support, liquides, solides, et/ou sous forme de gels, sont introduits dans un compartiment de dosage, de préférence un compartiment de rinçage ou un compartiment de lavage de machine à laver.

21. Utilisation d'un agent avec des catalyseurs de blanchiment fixés à un support, selon l'une quelconque des revendications précédentes, **caractérisé en ce qu'**il contient 0,0025% en poids à 0,25% en poids, en particulier 0,01 % en poids à 0,1% en poids de catalyseur(s) de blanchiment.

22. Utilisation d'un agent avec des catalyseurs de blanchiment fixés à un support, selon l'une quelconque des revendications précédentes, **caractérisé en ce qu'**il constitue un agent de lavage, un agent de nettoyage, un agent de soins, un agent de traitement capillaire, un matériau de construction, un produit cosmétique, une colle, un agent anti-bactérien, et/ou un agent désinfectant.

23. Utilisation d'un agent avec des catalyseurs de blanchiment fixés à un support, selon l'une quelconque des revendications précédentes, **caractérisé en ce qu'**il présente des tensio-actifs anioniques, des tensio-actifs cationiques, des tensio-actifs amphotères, des substances builders, des agents de blanchiment, des activateurs de blanchiment, des stabilisants de blanchiment, des catalyseurs de blanchiment, des enzymes, des polymères, des co-builders, des agents d'alcalinisation, des agents d'acidification, des agents anti-redépôt, des agents protégeant l'argenterie, des colorants, des azurants optiques, des substances protégeant des UV, des adoucissants, des substances odoriférantes, des substances repoussant la saleté, des substances anti-froisse, des substances anti-bactériennes, des substances protectrices des couleurs, des inhibiteurs de coloration, des vitamines, des silicates stratifiés, des substances formant des complexes odorants, des agents mouillants, des agents anti-mousse, des agents moussants, des agents conservateurs, et/ou des adjuvants.
